# EUROPEAN PATENT APPLICATION

(11) **EP 4 596 566 A1**
(43) Date of publication of application: **06.08.2025**
(21) Application number: 23872541.0
(22) Date of filing: 28.09.2023
(51) Int. Cl.: C07K 1/04, C07K 1/06

(54) **COMPOUND PRODUCTION METHOD**

(30) Priority: 28.09.2022 JP 2022154483
(71) Applicant: The University of Tokyo, Bunkyo-ku, Tokyo 113-8654 (JP)
(72) Inventor: SUGA, Hiroaki, Tokyo 113-8654 (JP); GOTO, Yuki, Tokyo 113-8654 (JP); VINOGRADOV, Alexander, Tokyo 113-8654 (JP); ZHANG, Yue, Tokyo 113-8654 (JP); CHANG, Jun Shi, Tokyo 113-8654 (JP)
(74) Representative: D Young & Co LLP
(86) International application number: PCT/JP2023/035472
(87) International publication number: WO 2024/071320

(57) **Abstract**

The present invention provides a method which makes it possible to simply produce a peptide including an aromatic 6-membered ring in the backbone. The present invention relates to a production method for a compound represented by Formula (I), the method including reacting an amino acid or amino acid derivative, or a peptide, which is bonded to a solid-phase carrier and has a free amino group, or a solid-phase carrier having a free amino group with a compound represented by Formula (1) to obtain a compound represented by Formula (2), in which a compound obtained by deprotecting PG of the compound represented by Formula (2) is optionally subjected to one or more amino acid condensation reactions and then is cleaved from the solid-phase carrier to perform a cyclization reaction.

## Description

### Technical Field

The present invention relates to a compound production method, in particular, a solid-phase synthesis method for a compound including an aromatic 6-membered ring.

### Background Art

A thiopeptide is a group of complex peptidic natural products having a nitrogen-containing 6-membered heterocyclic ring (usually pyridine, Py). The thiopeptide also includes other non-proteinogenic structures, in particular, cysteine/threonine/serine-derived azoles (thiazole, Thz; methyloxazole, MeOxz; and oxazole, Oxz), and dehydroamino acids (dhAA; for example, dehydroalanine, Dha; and dehydrobutyline, Dhb). The thiopeptide has attracted widespread attention as a candidate for a peptide pharmaceutical, and has therefore served as a major synthetic target and an engineering target in the past 20 years (Non-Patent Literatures 1 to 3).

Numerous approaches to a chemical synthesis of thiopeptides have been developed over the years, and even complex compounds having two cyclic structures (for example, thiostrepton, siomycin, and nosiheptide) have been synthesized (Non-Patent Literatures 4 to 14). However, in all of the synthesis methods described in Non-Patent Literatures 4 to 14, characteristic cyclic peptide moieties of thiopeptides/pyrithides are synthesized by a liquid phase synthesis method. So far, solid-phase peptide synthesis (SPPS) has been used only in the preparation of a tail region of valinomycin and in some enzymatic approaches (Non-Patent Literatures 15 to 17).

By the way, as a part of thiopeptide bioengineering, the biosynthesis of lactazole A, which is a potential thiopeptide of Streptomyces lactacystinaeus, has been reconstructed in vitro, and an mRNA display platform utilizing lactazole biosynthesis for de novo discovery of a designed pseudo-synthetic thiopeptide having a biological activity has been established (Patent Literature 1 and Non-Patent Literatures 18 to 21).

### Citation List

### Patent Literature

Patent Literature 1: Pamphlet of International Publication No. 2020/067550

### Non-Patent Literatures

Non-Patent Literature 1: Just-Baringo, X.; Albericio, F.; Alvarez, M. Thiopeptide antibiotics: retrospective and recent advances. Mar. Drugs. 2014, 12, 317-351.
Non-Patent Literature 2: Nicolaou, K. How thiostrepton was made in the laboratory. Angew. Chem. Int. Ed. 2012, 51, 12414-12436.
Non-Patent Literature 3: Vinogradov, A. A.; Suga, H. Introduction to thiopeptides: biological activity, biosynthesis, and strategies for functional reprogramming. Cell Chem. Biol. 2020, 27, 1032-1051.
Non-Patent Literature 4: Nicolaou, K.; Safina, B. S.; Zak, M.; Estrada, A. A.; Lee, S. H. Total synthesis of thiostrepton, Part 1: construction of the dehydropiperidine/thiazoline containing macrocycle. Angew. Chem. Int. Ed. 2004, 43, 5087-5092.
Non-Patent Literature 5: Nicolaou, K.; Zak, M.; Safina, B. S.; Lee, S. H.; Estrada, A. A. Total synthesis of thiostrepton, part 2: construction of the quinaldic acid macrocycle and final stages of the synthesis. Angew. Chem. Int. Ed. 2004, 43, 5092-5097.
Non-Patent Literature 6: Nicolaou, K.; Zak, M.; Safina, B. S.; Estrada, A. A.; Lee, S. H.; Nevalainen, M. Total synthesis of thiostrepton. Assembly of key building blocks and completion of the synthesis. J. Am. Chem. Soc. 2005, 127, 11176-11183.
Non-Patent Literature 7: Nicolaou, K.; Safina, B. S.; Zak, M.; Lee, S. H.; Nevalainen, M.; Bella, M.; Estrada, A. A.; Funke, C.; Zecri, F. J.; Bulat, S. Total synthesis of thiostrepton. Retrosynthetic analysis and construction of key building blocks. J. Am. Chem. Soc. 2005, 127, 11159-11175.
Non-Patent Literature 8: Mori, T.; Higashibayashi, S.; Goto, T.; Kohno, M.; Satouchi, Y.; Shinko, K.; Suzuki, K.; Suzuki, S.; Tohmiya, H.; Hashimoto, K. Total synthesis of siomycin A: completion of the total synthesis. Chem.: Asian J. 2008, 3, 1013-1025.
Non-Patent Literature 9: Wojtas, K. P.; Riedrich, M.; Lu, J. Y.; Winter, P.; Winkler, T.; Walter, S.; Arndt, H. D. Total synthesis of nosiheptide. Angew. Chem. Int. Ed. 2016, 55, 9772-9776.
Non-Patent Literature 10: Lu, J. Y.; Riedrich, M.; Mikyna, M.; Arndt, H. D. Aza Wittig Supported Synthesis of the A Ring of Nosiheptide. Angew. Chem. Int. Ed. 2009, 48, 8137-8140.
Non-Patent Literature 11: Moody, C.; Bagley, M. The first synthesis of promothiocin A. Chem. Commun. 1998, 2049-2050.
Non-Patent Literature 12: Akasapu, S.; Hinds, A. B.; Powell, W. C.; Walczak, M. A. Total synthesis of micrococcin P1 and thiocillin I enabled by Mo (VI) catalyst. Chem. Sci. 2019, 10, 1971-1975.
Non-Patent Literature 13: Hwang, H.-J.; Son, Y.-J.; Kim, D.; Lee, J.; Shin, Y.-J.; Kwon, Y.; Ciufolini, M. A. Diversity-oriented routes to thiopeptide antibiotics: total synthesis and biological evaluation of micrococcin P2. Org. Biomol. Chem. 2022, 1893-1899.
Non-Patent Literature 14: Christy, M. P.; Johnson, T.; McNerlin, C. D.; Woodard, J.; Nelson, A. T.; Lim, B.; Hamilton, T. L.; Freiberg, K. M.; Siegel, D. Total synthesis of micrococcin P1 through scalable thiazole forming reactions of cysteine derivatives and nitriles. Org. Lett. 2020, 22, 2365-2370.
Non-Patent Literature 15: Just Baringo, X.; Bruno, P.; Ottesen, L. K.; Canedo, L. M.; Albericio, F.; Alvarez, M. Total synthesis and stereochemical assignment of baringolin. Angew. Chem. Int. Ed. 2013, 52, 7818-7821.
Non-Patent Literature 16: Wever, W. J.; Bogart, J. W.; Baccile, J. A.; Chan, A. N.; Schroeder, F. C.; Bowers, A. A. Chemoenzymatic synthesis of thiazolyl peptide natural products featuring an enzyme-catalyzed formal [4+2] cycloaddition. J. Am. Chem. Soc. 2015, 137, 3494-3497.
Non-Patent Literature 17: Wever, W. J.; Bogart, J. W.; Bowers, A. A. Identification of pyridine synthase recognition sequences allows a modular solid-phase route to thiopeptide variants. J. Am. Chem. Soc. 2016, 138, 13461-13464.
Non-Patent Literature 18: Vinogradov, A. A.; Shimomura, M.; Goto, Y.; Ozaki, T.; Asamizu, S.; Sugai, Y.; Suga, H.; Onaka, H. Minimal lactazole scaffold for in vitro thiopeptide bioengineering. Nat. Commun. 2020, 11, 1-13.
Non-Patent Literature 19: Vinogradov, A. A.; Shimomura, M.; Kano, N.; Goto, Y.; Onaka, H.; Suga, H. Promiscuous enzymes cooperate at the substrate level en route to lactazole A. J. Am. Chem. Soc. 2020, 142, 13886-13897.
Non-Patent Literature 20: Vinogradov, A. A.; Nagai, E.; Chang, J. S.; Narumi, K.; Onaka, H.; Goto, Y.; Suga, H. Accurate Broadcasting of Substrate Fitness for Lactazole Biosynthetic Pathway from Reactivity-Profiling mRNA Display. J. Am. Chem. Soc. 2020, 142, 20329-20334.
Non-Patent Literature 21: Vinogradov, A. A.; Nagano, M.; Goto, Y.; Suga, H. Site-Specific Nonenzymatic Peptide S/O-Glutamylation Reveals the Extent of Substrate Promiscuity in Glutamate Elimination Domains. J. Am. Chem. Soc. 2021, 143, 13358-13369.

### Summary of Invention

### Technical Problem

As described above, a liquid phase synthesis method has hitherto been mainly used as a synthesis method for a compound including an aromatic 6-membered ring, such as a thiopeptide. However, in the liquid phase synthesis, since it is necessary to prepare a unit constituting a peptide sequence each and every time individually depending on a specific synthetic target peptide, it is difficult to apply a synthesis route used for the synthesis of a specific compound to other derivatives (that is, it is extremely difficult to synthesize a wide variety of derivatives). In addition, in order to remove impurities that are by-products in the liquid phase synthesis, a complicated purification operation is required, which requires a lot of time, effort, and cost.

Therefore, there is a demand for a production method for a compound having an aromatic 6-membered ring, such as a thiopeptide, using solid-phase synthesis, which enables parallel synthesis of a wide variety of derivatives and allows a purification operation to be simply carried out.

An object to be accomplished by the present invention is to provide a method for simply producing a peptide including an aromatic 6-membered ring in the backbone.

### Solution to Problem

As a result of intensive studies to accomplish the object, the present inventors have found that it is possible to simply produce a peptide including an aromatic 6-membered ring in the backbone by carrying out solid-phase synthesis using a predetermined compound, leading to completion of the present invention.

That is, the present invention is as follows.
[1] A production method for a compound represented by Formula (I), 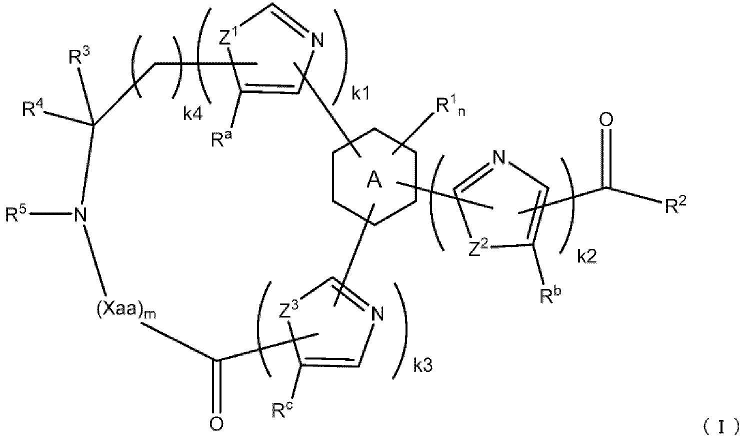 (in Formula (I),
   ring A is an aromatic 6-membered ring,
   Z¹, Z², and Z³ are each independently an oxygen atom or a sulfur atom,
   R^{a}, R^{b}, and R^{c} are each independently a hydrogen atom or a hydrocarbon group,
   k1, k2, and k3 are each independently an integer of 0 or more and 2 or less,
   k4 is an integer of 0 or more and 2 or less,
   n is an integer of 0 or more and 2 or less,
   R¹ and R² are each independently a monovalent group,
   R³ is a hydrogen atom, a side chain of an amino acid, or a side chain of an amino acid derivative, forms a ring together with R⁴ or R⁵, or forms a double bond together with R⁴,
   R⁴ is a hydrogen atom or a hydrocarbon group, or forms a ring or a double bond together with R³,
   R⁵ is a hydrogen atom or a hydrocarbon group, or forms a ring together with R³,
   (Xaa)ₘ is a peptide including m pieces of amino acids and/or amino acid derivatives, and
   m is an integer of 2 or more),
   the method including:
      reacting an amino acid or amino acid derivative, or a peptide, which is bonded to a solid-phase carrier and has a free amino group, or a solid-phase carrier having a free amino group with a compound represented by the following formula: 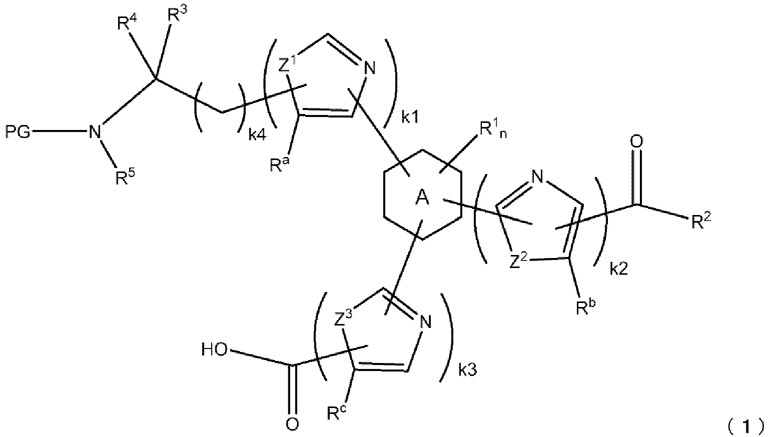 in Formula (1),
      ring A, Z¹, Z², Z³, R^{a}, R^{b}, R^{c}, k1, k2, k3, k4, n, R¹, R², R³, R⁴, and R⁵ are each defined in the same manner as in Formula (I), and
      PG is a protective group)
      to obtain a compound represented by the following formula: 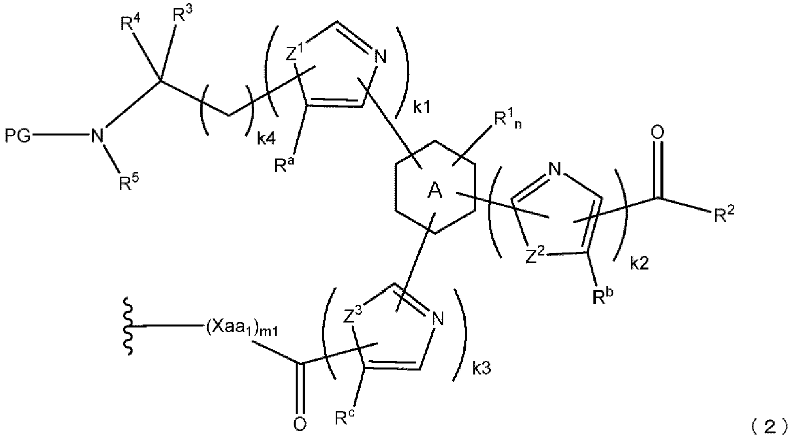 (in Formula (2),
         ring A, Z¹, Z², Z³, R^{a}, R^{b}, R^{c}, k1, k2, k3, k4, n, R¹, R², R³, R⁴, and R⁵ are each defined in the same manner as in Formula (1),
         (Xaa₁)ₘ₁ is a peptide residue including m1 pieces of amino acids and/or amino acid derivatives bonded to the solid-phase carrier in a case where m1 is 2 or more, an amino acid residue or an amino acid derivative residue in a case where m1 is 1, or a single bond in a case where m1 is 0,
         m1 is an integer of 0 or more, and
         a wavy line indicates that (Xaa₁)ₘ₁ in a case where m1 is 1 or more or a carbonyl in a case where m1 is 0 is bonded to the solid-phase carrier),
         in which a compound obtained by deprotecting PG of the compound represented by Formula (2) is optionally subjected to one or more amino acid condensation reactions and then is cleaved from the solid-phase carrier to perform a cyclization reaction.
[2] The method according to [1],
   in which the compound represented by Formula (1) is represented by Formula (1-A), 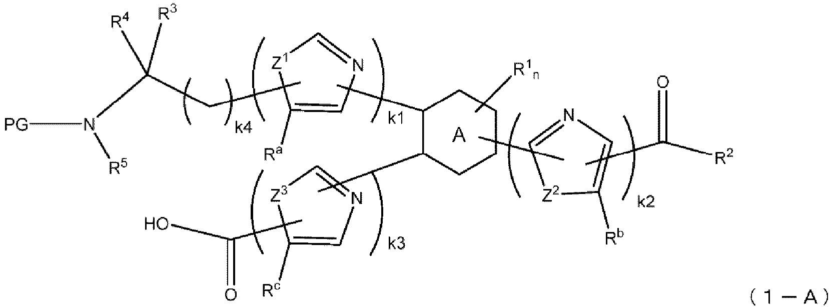 (in Formula (1-A),
   ring A, Z¹, Z², Z³, R^{a}, R^{b}, R^{c}, k1, k2, k3, k4, n, R¹, R², R³, R⁴, R⁵, and PG are each defined in the same manner as in Formula (1)).
[3] The method according to [1],
   in which the compound represented by Formula (1) is represented by Formula (1-B), 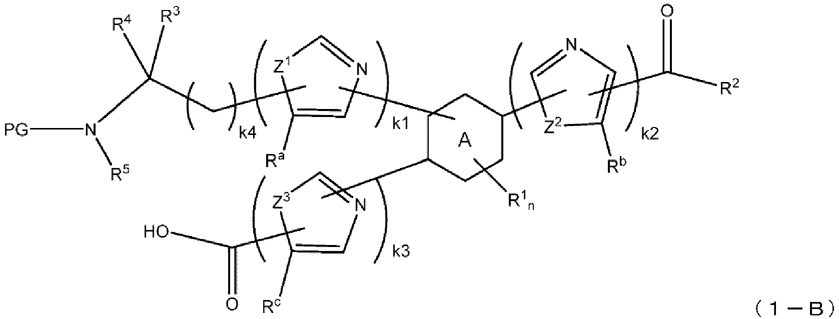 (in Formula (1-B),
   ring A, Z¹, Z², Z³, R^{a}, R^{b}, R^{c}, k1, k2, k3, k4, n, R¹, R², R³, R⁴, R⁵, and PG are each defined in the same manner as in Formula (1)).
[4] The method according to [1],
   in which the compound represented by Formula (1) is represented by Formula (1-C), 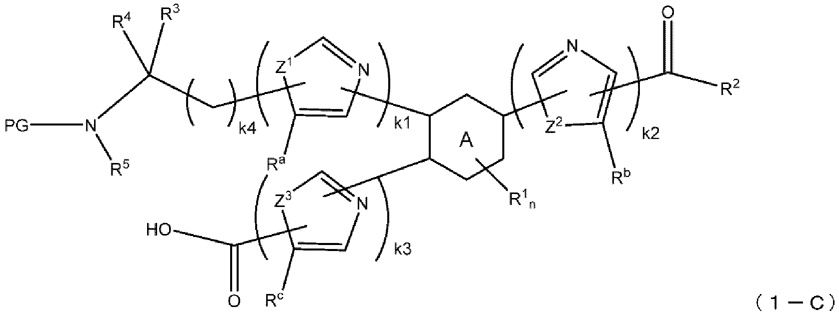 (in Formula (1-C),
   ring A, Z¹, Z², Z³, R^{a}, R^{b}, R^{c}, k1, k2, k3, k4, n, R¹, R², R³, R⁴, R⁵, and PG are each defined in the same manner as in Formula (1)).
[5] The method according to any one of [1] to [4],
   in which a side chain functional group of an amino acid and/or amino acid derivative constituting (Xaa)ₘ of the compound obtained by the cyclization reaction is protected by a protective group, and
   the method further includes deprotecting the protective group of the side chain functional group.
[6] The method according to any one of [1] to [5],
   in which the compound obtained by the cyclization reaction has a monovalent group represented by the following formula:

   -CR⁷₂-Se-R⁸ (3)

   (in Formula (3), Se represents selenium, R⁷'s each independently represent a hydrogen atom or a hydrocarbon group, and R⁸ represents a hydrocarbon group which may have a substituent), and
   the method further includes oxidizing the group represented by Formula (3) after the cyclization reaction to form a double bond.
[7] The method according to any one of [1] to [6], the method further including:
   eliminating R² after the cyclization reaction, and reacting the carboxy group generated by the elimination with one or more of compounds.

### Advantageous Effect of Invention

According to the present invention, it is possible to provide a method which makes it possible to simply produce a peptide including an aromatic 6-membered ring in the backbone.

### Brief Description of Drawings

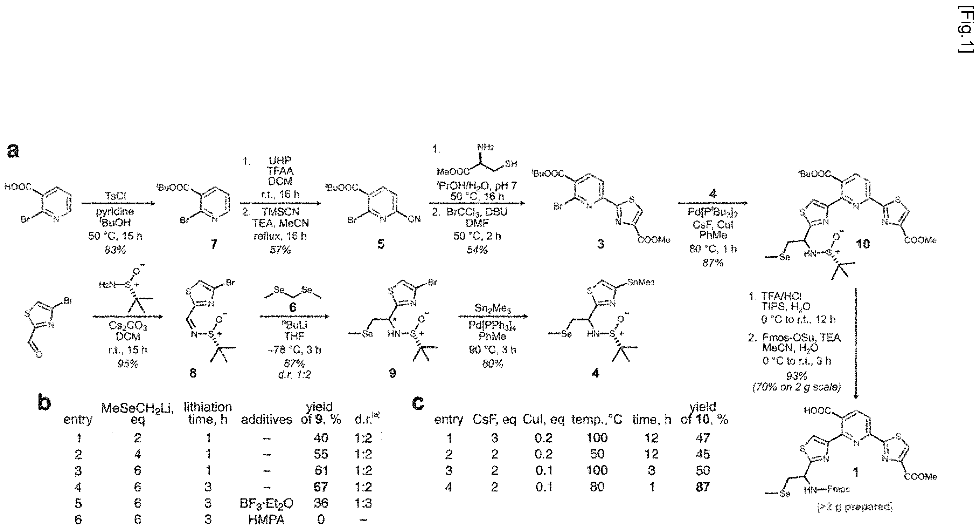

[Figure 1] Figure 1(a) illustrates an overall synthesis scheme, Figure 1(b) illustrates optimization of reaction conditions for addition of selenomethyl lithium to imine 8 ([a] indicates an isomer ratio of (R,R) to (R,S)), and Figure 1(c) illustrates optimization of reaction conditions for formation of a bond between 2-bromopyridine 3 and 4-stannylthiazole 4, in a synthesis method for a compound 1 (compound represented by Formula (1)). Abbreviations: TsCl: 4-toluenesulfonyl chloride; UHP: urea/hydrogen peroxide; TFAA: trifluoroacetic anhydride; TEA: triethylamine; TMSCN: trimethylsilyl cyanide; DBU: diazabicycloundecene; TFA: trifluoroacetic acid; TIPS: triisopropylsilane; Fmoc-OSu: N-(9-fluorenylmethoxycarbonyloxy)succinimide; DCM: dichloromethane; DMF: N,N-dimethylformamide; THF: tetrahydrofuran; HMPA: hexamethylphosphoramide.

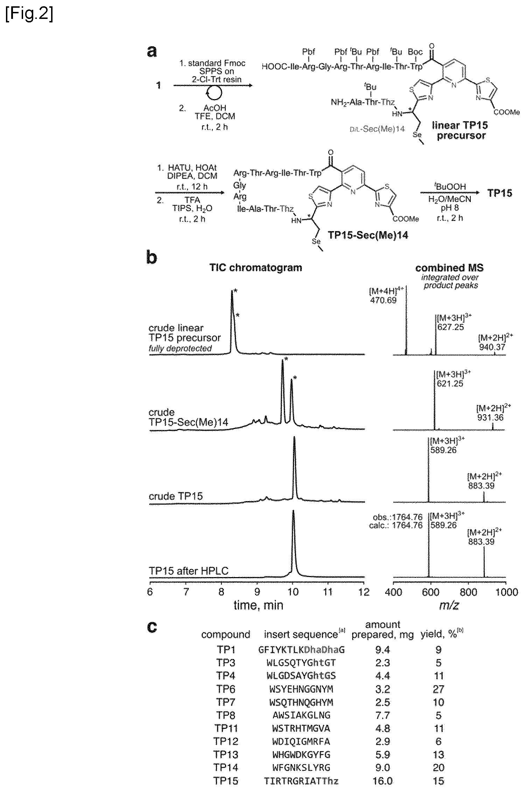

[Figure 2] Figure 2(a) illustrates an overall synthesis scheme for TP15, Figure 2(b) illustrates the analysis of a reaction product shown in Figure 2(a) by LC/MS, and Figure 2(c) illustrates the synthesis results of eleven synthesized compounds, in peptide synthesis. Abbreviations in Figure 2(a): AcOH: acetic acid, TFE:
   tetrafluoroethylene, HATU:
   (1-[bis(dimethylamino)methylene]-1H-1,2,3-triazolo[4,5-b]pyridinium 3-oxide, HOAt: 1-hydroxy-7-azabenzotriazole, DIPEA: N,N-diisopropylethylamine. In Figure 2(b), a total ion chromatogram integrated over the LC peaks and the MS results are displayed. The peaks labeled with an asterisk (*) correspond to product isomers derived from undefined stereochemistry at Sec14. After oxidative elimination from Sec14 to Dha14, both isomers are converted into a single product. In Figure 2(c), [a] has Dha: dehydroalanine; htG: γ^{S,L} homoGln (a product of Cys alkylation with iodoacetamide); Thz: thiazole, and [b] indicates the yield with respect to the resin loading at the start.
[Figure 3] Figure 3 illustrates 11 types of peptides synthesized in Example 2.
[Figure 4] Figure 4 illustrates the functionalization of a lactazole-like thiopeptide in a tail region. A synthesis scheme for C-terminal modification using the synthesis of ^{ct}TP4 as an example, and LC/MS analysis of each reaction product are shown. The results of integrating the total ion chromatogram and the MS results over the LC peaks are shown. †: A by-product generated in a process for hydrolysis of a methyl ester. Abbreviations: DCE: 1,2-dichloroethane; EDCI:
   1-(3-dimethylaminopropyl)-3-ethylcarbodiimide hydrochloride.
[Figure 5] Figure 5 illustrates 6 types of peptides synthesized in Example 3.

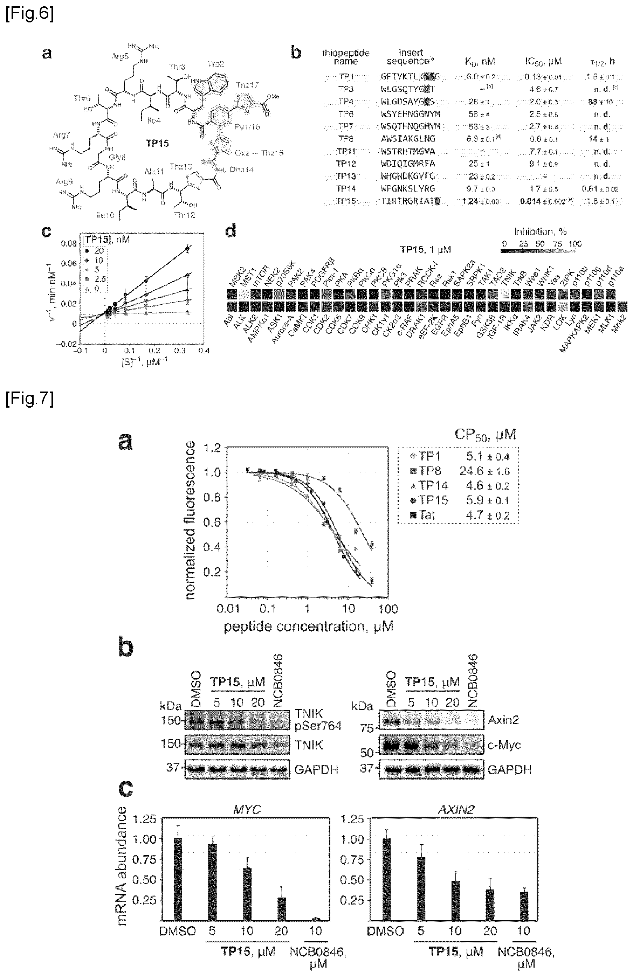

[Figure 6] Figure 6 illustrates biochemical characteristics of the synthesized compound. Figure 6(a) illustrates a structural formula of TP15 as a lactazole-like thiopeptide. Figure 6(b) illustrates a binding affinity (K_{D} value) to TNIK and an inhibition (IC₅₀) by a thiopeptide. The half-life (τ1/2) in the human serum was also shown. These values were obtained by non-linear regression of the experimental data. [a]: S emphasized by the shadow represents a Dha residue, C of TP3 and TP4 represents γ^{S,L}hGln (a product of IAA alkylation of Cys), and C of TP5 represents Thz. [b]: It was indicated that the binding/inhibition was not measured. [c]: n.d. indicates that the value was not determined. [d]: For TP8, the binding was measured in the presence of 1 mM ATP. [e]: For TP15, Kᵢ was measured as 3 nM. Figure 6(c) illustrates Lineweaver-Burk plots of TP15 for the inhibition of TNIK. The thiopeptide acts as a substrate competitive inhibitor of an enzyme. Figure 6(d) illustrates the results of Color-coded kinase selectivity profiling of TP15 for a panel of human kinases of 67. The compound exhibits good selectivity for a target enzyme.
[Figure 7] Figure 7 illustrates that TP15 inhibits TNIK in an HCT116 cancer cell line. Figure 7(a) illustrates that the CAPA assays of TP1, 8, 14, and 15 were carried out in HEK293H cells. Three out of the four thiopeptides exhibited cytoplasmic translocation at concentrations comparable to the well-known cell-penetrating peptide Tat. Figures 7(b) and 7(c) illustrate an inhibition of TNIK autophosphorylation and a down-regulation of Wnt target genes by TP15 in HCT116 cells. The sub-confluent HCT116 cells were treated with the indicated compounds for 24 hours and the cellular levels of TNIK, TNIK pSer764 (autophosphorylation product), c-Myc, and Axin2 were analyzed by an immunoblotting method (Figure 7(b)) and RT-qPCR (Figure 7(c)). NCB0846 is a small molecule TNIK inhibitor.
[Figure 8] Figure 8 illustrates 6 types of peptides synthesized in Example 5.
[Figure 9] Figure 9 illustrates 7 types of peptides synthesized in Example 5.
[Figure 10] Figure 10 illustrates 3 types of peptides synthesized in Example 5.

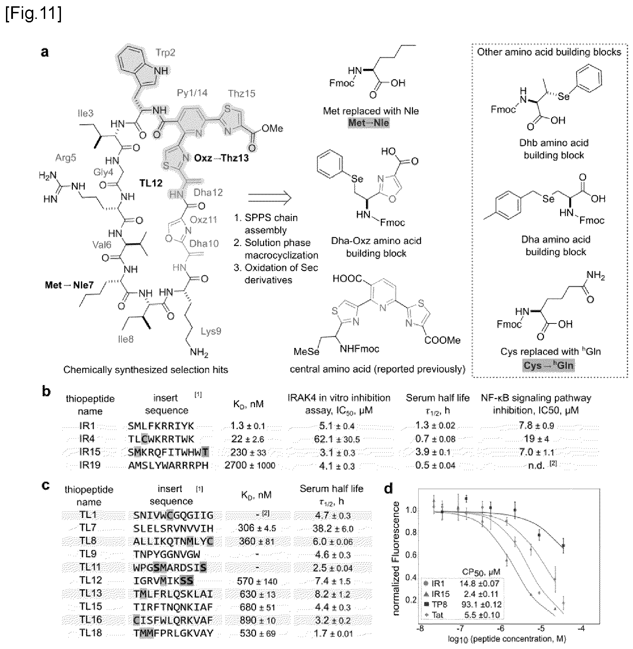

[Figure 11] Figure 11(a) illustrates TL12 which is a lactazole-like binder of TLR10, retrosynthesis thereof, and amino acid building blocks used. Figure 11(b) illustrates a binding affinity (K _{D} value) to IRAK4 and an inhibition (IC₅₀) by the synthesized thiopeptide. The half-life (τ_{1/2}) in the human serum and the inhibition (IC₅₀) of the NF-kB signaling pathway in THP-1 cells are also shown. These values were obtained by non-linear regression of the experimental data. [1]: The shaded C in IR4 is hGln (which is a product of IAA alkylation of Cys, but is replaced by homoglutamine); the shaded M in IR15 is Nle (in which Met is replaced by Nle); and the shaded T in IR15 represents a Dhb residue. [2]: n.d. indicates that the inhibition was not measured. Figure 11(c) illustrates the binding affinity (K_{D} value) to TLR10 and the half-life (τ _{1/2}) in the human serum. [1]: The shaded C in TL1, TL8, and TL16 is hGln (a product of IAA alkylation of Cys, but is replaced by homoglutamine); the shaded M in TL8, TL11, TL12, TL13, and TL18 is Nle (in which Met is replaced by Nle); the shaded S in 8S of TL11 and TL12 represents a Dha residue; and the shaded S in 9S of TL12 represents an oxazole. [2]: "-" indicates that the determination was not made. Figure 11(d) illustrates the results of a CAPA assay of IR1, IR15, and TP8. The assay was carried out in HEK293T cells. IR15 is shown to be taken up into the cytoplasm of HEK293T cells much more efficiently than Tat which is a known cell-penetrating peptide.
[Figure 12] Figure 12 illustrates an example of a peptide synthesized in Example 6.
[Figure 13] Figure 13 illustrates an example of the peptide synthesized in Example 6.
[Figure 14] Figure 14 illustrates an example of the peptide synthesized in Example 6.
[Figure 15] Figure 15 illustrates the synthesis results of a compound synthesized in Example 6. The yield is with respect to the resin loading at the start. The compound names are shown according to the notation described in Figures 12 to 14.

### Description of Embodiments

Hereinafter, embodiments (hereinafter, referred to as "the present embodiments") for carrying out the present invention will be described in detail. However, the present invention is not limited thereto, and various modifications are possible without departing from the gist of the present invention.

### [Compound Production Method]

The production method of the present embodiment is a production method for a compound represented by Formula (I), 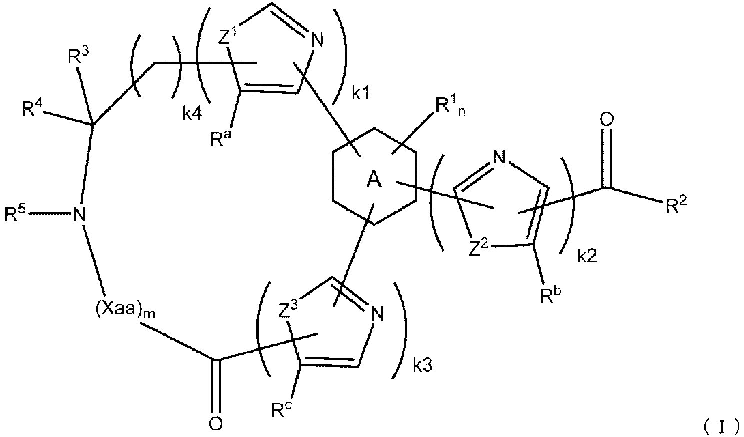 the method including:
reacting an amino acid or amino acid derivative, or a peptide, which is bonded to a solid-phase carrier and has a free amino group, or a solid-phase carrier having a free amino group with a compound represented by the following formula: 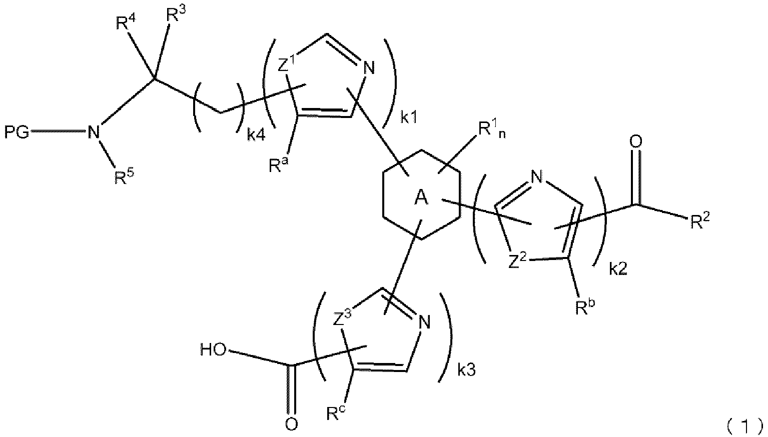 to obtain a compound represented by the following formula: 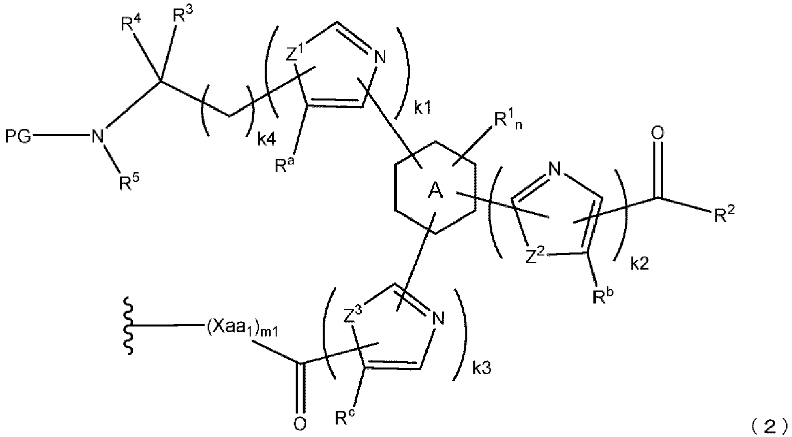 in which a compound obtained by deprotecting PG of the compound represented by Formula (2) is cleaved from the solid-phase carrier to perform a cyclization reaction, after one or more amino acid condensation reactions are optionally performed.

Here, in Formula (I),
ring A is an aromatic 6-membered ring,
Z¹, Z², and Z³ are each independently an oxygen atom or a sulfur atom,
R^{a}, R^{b}, and R^{c} are each independently a hydrogen atom or a hydrocarbon group,
k1, k2, and k3 are each independently an integer of 0 or more and 2 or less,
k4 is an integer of 0 or more and 2 or less,
n is an integer of 0 or more and 2 or less,
R¹ and R² are each independently a monovalent group,
R³ is a hydrogen atom, a side chain of an amino acid, or a side chain of an amino acid derivative, forms a ring together with R⁴ or R⁵, or forms a double bond together with R⁴,
R⁴ is a hydrogen atom or a hydrocarbon group, or forms a ring or a double bond together with R³,
R⁵ is a hydrogen atom or a hydrocarbon group, or forms a ring together with R³,
(Xaa)ₘ is a peptide including m pieces of amino acids and/or amino acid derivatives, and
m is an integer of 2 or more.

In addition, in Formula (1),
ring A, Z¹, Z², Z³, R^{a}, R^{b}, R^{c}, k1, k2, k3, k4, n, R¹, R², R³, R⁴, and R⁵ are each defined in the same manner as in Formula (I), and
PG is a protective group.

In addition, in Formula (2),
ring A, Z¹, Z², Z³, R^{a}, R^{b}, R^{c}, k1, k2, k3, k4, n, R¹, R², R³, R⁴, and R⁵ are each defined in the same manner as those in Formula (1),
(Xaa₁)ₘ₁ is a peptide residue including m1 pieces of amino acids and/or amino acid derivatives bonded to the solid-phase carrier in a case where m1 is 2 or more, an amino acid residue or an amino acid derivative residue in a case where m1 is 1, or a single bond in a case where m1 is 0,
m1 is an integer of 0 or more, and
a wavy line indicates that (Xaa₁)ₘ₁ in a case where m1 is 1 or more or a carbonyl in a case where m1 is 0 is bonded to the solid-phase carrier.

The production method of the present embodiment may further include deprotecting PG of the compound represented by Formula (2) before cleaving the compound obtained by deprotecting PG of the compound represented by Formula (2) from the solid-phase carrier; and bonding one or more amino acids or amino acid derivatives to the deprotected compound by an amino acid condensation reaction.

The production method of the present embodiment may further include deprotecting a protective group of a side chain functional group of the amino acid and/or amino acid derivative constituting (Xaa)ₘ of the compound obtained by the cyclization reaction.

The production method of the present embodiment may further include oxidizing a monovalent group represented by the following formula:

-CR⁷₂-Se-R⁸ (3)

(in Formula (3), Se represents selenium, R⁷'s each independently represent a hydrogen atom or a hydrocarbon group, and R⁸ represents a hydrocarbon group which may have a substituent) contained in the compound obtained by the cyclization reaction to form a double bond.

**The** production method of the present embodiment may further include eliminating a moiety corresponding to R² in Formula (I) of the compound obtained by the cyclization reaction, and reacting the carboxy group generated by the elimination with one or more of compounds.

As described above, the production method of the present embodiment enables the compound represented by Formula (1) to be easily incorporated into a peptide chain using a solid-phase carrier, and a peptide including the aromatic 6-membered ring in the backbone can be simply produced. **In** addition, after the peptide including an aromatic 6-membered ring in the backbone is produced, a reaction such as deprotection of a protective group of a side chain functional group of an amino acid and/or amino acid derivative, formation of a double bond, and modification of R² (that is, a tail portion of the cyclic peptide) can be further performed, and various peptide derivatives can be obtained.

Each reaction carried out in the production method of the present embodiment may be performed step by step or may be performed in a series in the system. In addition, the production method of the present embodiment may include a step or treatment that is usually performed in a solid-phase synthesis method, such as separation and washing of a solid-phase carrier, between the respective reactions.

### (Compound of Formula (I))

In the production method of the present embodiment, the compound represented by Formula (I) is produced. The compound represented by Formula (I) is a peptide including an aromatic 6-membered ring in the backbone, and examples thereof include thiopeptides and pyritides, which are natural products having a large potential as peptide pharmaceuticals, and artificial derivatives thereof.

In Formula (I), the ring A is an aromatic 6-membered ring, preferably a benzene ring or a nitrogen-containing aromatic 6-membered ring, more preferably the benzene ring, a pyridine ring, a pyridazine ring, a pyrimidine ring, a pyrazine ring, or a triazine ring, and still more preferably the benzene ring or the pyridine ring.

In Formula (I), Z¹, Z², and Z³ are each independently an oxygen atom or a sulfur atom. Z¹, Z², and Z³ may be different from or the same as each other. In addition, in a case where a plurality of Z¹'s, Z²'s, or Z³'s are present, the plurality of Z¹'s, Z²'s, or Z³'s may be different from or the same as each other.

In Formula (I), R^{a}, R^{b}, and R^{c} are each independently a hydrogen atom or a hydrocarbon group, preferably the hydrogen atom or a hydrocarbon group having 1 to 10 carbon atoms (including both ends; the same applies hereinafter in the present specification), and more preferably the hydrogen atom, an alkyl group having 1 to 3 carbon atoms, or an aryl group having 6 to 8 carbon atoms. R^{a}, R^{b}, and R^{c} may be different from or the same as each other.

R^{a}, R^{b}, and R^{c} may be each independently the hydrogen atom, a methyl group, an ethyl group, a propyl group, an isopropyl group, a phenyl group, or a tosyl group, may be each independently the hydrogen atom, the methyl group, the isopropyl group, or the phenyl group, and may be each independently the hydrogen atom or the methyl group.

In Formula (I), k1, k2, and k3 are each independently an integer of 0 or more and 2 or less, and may be an integer of 0 or more and 1 or less. k1, k2, and k3 may be different from or the same as each other. The sum of k1, k2, and k3 is 0 or more and 6 or less, preferably 0 or more and 4 or less, and more preferably 0 or more and 3 or less. The sum of k1, k2, and k3 may be 0 or more, 1 or more, or 2 or more, and may be 6 or less, 5 or less, 4 or less, 3 or less, or 2 or less. Furthermore, in a case where k1, k2, or k3 is 0, the azole moiety enclosed in the corresponding parentheses in Formula (I) is a single bond.

In Formula (I), k1 may be 0 or 1, k2 may be 0 or 1, and k3 may be 0.

In Formula (I), k4 is an integer of 0 or more and 2 or less, preferably 0 or 1, and more preferably 0. In a case where k4 is 0, the hydrocarbon moiety enclosed in the corresponding parentheses in Formula (I) is a single bond.

In Formula (I), n is an integer of 0 or more and 2 or less, preferably 0 or 1, and more preferably 0. A case where n is 0 means that R¹ in Formula (I) is not bonded to the ring A.

In Formula (I), R¹ is a monovalent group. The monovalent group is not particularly limited, and examples thereof include a hydrocarbon group such as an alkyl group, a hydroxy group, an amino group, and a carboxy group. R¹ is preferably selected from the hydrocarbon group, the hydroxy group, and the amino group. Here, the number of carbon atoms in the hydrocarbon group is preferably 1 to 10, more preferably 1 to 5, and still more preferably 1 to 3. In addition, the hydrocarbon group is preferably the alkyl group.

In Formula (I), R² is a monovalent group. The monovalent group is not particularly limited, and examples thereof include a hydrogen atom, -OH, -OR^{2a}, -NH₂, -NHR^{2b}, -NR^{2b}₂, and other groups (protective groups) that can be eliminated by a deprotection treatment.

Here, R^{2a} is a hydrocarbon group, preferably a hydrocarbon group having 1 to 30 carbon atoms, and more preferably an alkyl group having 1 to 3 carbon atoms or an aryl group having 6 to 8 carbon atoms. Examples of R^{2a} include a methyl group, an allyl group, a benzyl group, a benzyl group substituted with a hydrocarbon group having 1 to 10 carbon atoms, a tert-butyl group, a trityl group, and a trityl group substituted with a hydrocarbon group having 1 to 10 carbon atoms.

In addition, R^{2b}'s are each independently a hydrocarbon group, or two R^{2b}'s form a ring together. In a case where R^{2b} does not form a ring, R^{2b} is preferably a hydrocarbon group having 1 to 10 carbon atoms, more preferably an alkyl group having 1 to 3 carbon atoms or an aryl group having 6 to 8 carbon atoms, and is, for example, a methyl group or a phenyl group. In a case where two R^{2b}'s form a ring together, the ring is not particularly limited, but may be, for example, an aliphatic ring which has 3 to 15 (preferably 3 to 10, and more preferably 4 to 8) atoms constituting the ring and may include two or more heteroatoms in the ring. The heteroatom that can be included in the ring is not particularly limited, but is, for example, a nitrogen atom or an oxygen atom. Examples of the ring thus formed include a pyrrolidine ring, an imidazolidine ring, a pyrazolidine ring, an oxazolidine ring, an isoxazolidine ring, a piperidine ring, a piperazine ring, a morpholine ring, and a hexahydro-1,3,5-triazine ring.

R² may be a group in which R^{2a} in -OR^{2a} is substituted with a substituent including a heteroatom, such as a group in which R^{2a} in -OR^{2a} is a 4-{N-[1-(4,4-dimethyl-2,6-dioxocyclohexylidene)-3-methylbutyl]-amino}benzyl group (Dmab group), a benzyl group having a substituent other than a hydrocarbon group, or a trityl group having a substituent other than a hydrocarbon group.

In addition, in Formula (I), R² may be a functional group for evaluating the cell permeability of a compound represented by Formula (I), such as a monovalent group including a chloroalkyl moiety, a functional group including a labeling substance, a functional group for improving the water solubility of the compound represented by Formula (I), such as a monovalent group including a polyethylene glycol (PEG) moiety, a functional group for improving the stability in blood of the compound represented by Formula (I), such as a monovalent group including a hydrocarbon chain (the number of carbon atoms may be, for example, 1 to 20, or 3 to 10) moiety, an amino acid and an amino acid derivative, and a peptide chain. Examples of such R² include -NHR^{2c}. Here, R^{2c} is a monovalent group, and may be, for example, a chloroalkyl group which may include a linker, a group including a labeling substance which may include a linker, a polyethylene glycol group which may include a linker, a hydrocarbon group which may include a linker, an amino acid and an amino acid derivative, and a peptide chain. Here, the linker in R^{2c} is any group that links a nitrogen atom of -NHR^{2c} to a chloroalkyl group, a group including a labeling substance, a polyethylene glycol group, or a hydrocarbon group. Examples of the linker include an alkylene group which may include an ether bond or an amide bond in the main chain. The alkylene group may have, for example, 1 or more and 20 or less carbon atoms.

The chloroalkyl group in R^{2c} may have 1 to 20 carbon atoms or 2 to 15 carbon atoms. In addition, the number of chlorine atoms may be 1 to 5, 1 to 3, or 1. The chloroalkyl group which may include a linker may be a group described as a chloroalkane tag in L. Peraro et al., J. Am. Chem. Soc. 2018, 140, 36, 11360-11369.

Examples of the labeling substance in R^{2c} include enzymes such as peroxidase, alkaline phosphatase, β-galactosidase, glucose oxidase, malate dehydrogenase, glucose-6-phosphate dehydrogenase, and invertase; fluorescent substances such as a rhodamine derivative, a fluorescein derivative, a coumarin derivative, a dipyrromethene boron derivative, a Cy dye derivative, a pyrene derivative, a phycobilin protein, phycoerythrin, and phycocyanin; quenchers such as a dabsyl group derivative, Tide Quencher^{™} series, and black hole quencher derivatives; affinity tags such as a biotin derivative, a Flag peptide, a Myc peptide, and an HA peptide; and luminescent substances such as isoluminol and lucigenin; and radioactive substances such as ³H, ¹⁴C, ³²P, ³⁵S, and ¹²⁵I.

The peptide in R^{2c} may consist of two or more amino acids or amino acid derivatives, and the number of amino acids or amino acid derivatives may be 2 to 50, 2 to 30, 2 to 20, or 2 to 10.

In Formula (I), since R² can be a desired monovalent group by a C-terminal modification reaction which will be described later, R² is not limited to the above-described group.

In the present specification, the term "amino acid" includes not only a natural amino acid but also an artificial amino acid mutant and the like. Examples of the amino acid include proteinogenic amino acids and non-proteinogenic amino acids (natural or unnatural non-proteinogenic amino acids, chemically synthesized compounds having characteristics known in the related art as characteristics of an amino acid, and the like).

In a case where the proteinogenic amino acids are represented by a three-letter notation generally known in the related art, the proteinogenic amino acids are Arg, His, Lys, Asp, Glu, Ser, Thr, Asn, Gln, Cys, Gly, Pro, Ala, Ile, Leu, Met, Phe, Trp, Tyr, and Val. In addition, in a case where the proteinogenic amino acids are represented by one-letter notation generally known in the related art, the proteinogenic amino acids are R, H, K, D, E, S, T, N, Q, C, G, P, A, I, L, M, F, W, Y, and V. The proteinogenic amino acids are usually L-amino acids, but may have a structure as a D-amino acid included in the non-proteinogenic amino acids.

Examples of the non-proteinogenic amino acids include natural or unnatural amino acids other than the proteinogenic amino acids.

Examples of the non-proteinogenic amino acids include amino acids having a main chain structure different from that of natural amino acids (α,α-disubstituted amino acids (α-methylalanine, cycloleucine, and the like), N-methylamino acids, N-alkylamino acids, D-amino acids, β-amino acids, γ-amino acids, δ-amino acids, long-chain amino acids, α-hydroxy acids, α-thio acids, cyclic amino acids (cyclic α-amino acids, cyclic β-amino acids, aromatic amino acids, and the like), and the like); amino acids having a side chain structure different from that of natural amino acids (selenocysteine, norleucine, spinacine, nitrophenylalanine, tetrahydroisoquinoline carboxylic acid, tetrahydroisoquinoline carboxylic acid having a substituent (for example, a hydroxy group, a C1-C3 alkyl group, a halogen group, and the like), such as hydroxytetrahydroisoquinoline carboxylic acid, hydroxytryptophan, pentafluorophenylalanine, methoxyphenylalanine, γ^{S,L} homoglutamine, amino acids having a structure consisting of a plurality of rings, such as bicycloamino acids, azide group-containing amino acids, alkyne group-containing amino acids, alkene group-containing amino acids, chloroacetamide group-containing amino acids, photoreactive group-containing amino acids, fluorescent amino acids, ε-alkylated lysines, biotin group-containing amino acids, citrulline, ester group-containing amino acids, amino acids having an additional methylene in the side chain ("homo" amino acids; for example, homophenylalanine, homoglutamine, homohistidine, and the like), amino acids in which a carboxylic acid functional group in the side chain is substituted with a sulfonic acid group (such as cysteic acid and the like), and the like); and combinations thereof. Examples of the combinations thereof include amino acids in which the structure of the main chain and the structure of the side chain are different from those of the natural amino acids, an N-methylated product in which the main chain amino group of the above-described amino acid is methylated, and D-amino acids of the above-described amino acids. Specific examples of the other unnatural amino acids include the amino acids described in Pamphlet of International Publication No. 2015/030014.

Hereinafter, non-proteinogenic amino acids are exemplified as the specific structure, but in a case where the structure is defined by a stereochemistry of an asymmetric carbon, the compound may have opposite stereochemistry. That is, in a case where the amino acid is described as a D-amino acid, it may be an L-amino acid, and in a case where the amino acid is described as an L-amino acid, it may be a D-amino acid, or in any case, it may be a mixture (including a racemate) of the D-amino acids and the L-amino acids in any ratio. In addition, the compound may be an N-methylated product in which the main chain amino group is methylated.

Examples of the α,α-disubstituted amino acid include the following ones.

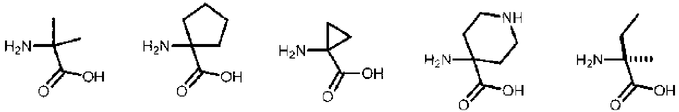

Examples of the N-methyl amino acid include the following ones, in addition to the N-methylated product in which the main chain amino group of the proteinogenic amino acid is methylated.

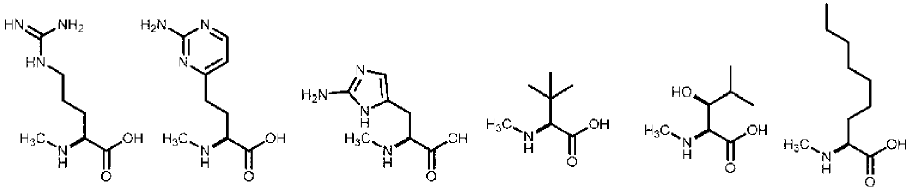

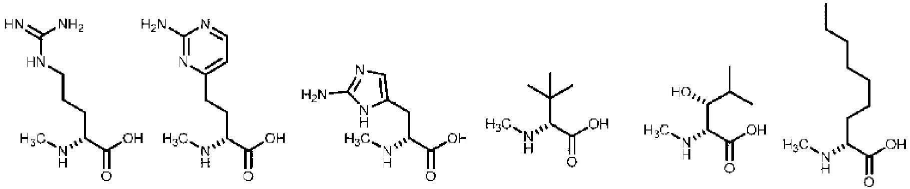

Examples of the D-amino acid include the following ones, in addition to the D-form of the proteinogenic amino acid.

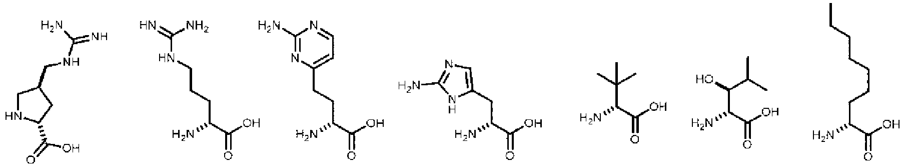

Examples of the β-amino acid (including an N-methylated β-amino acid) include the following ones, in addition to those having an additional methylene in the main chain of a proteinogenic amino acid. Furthermore, it is noted that the β-amino acid is shown as the amino acid having an additional methylene in the main chain, but the γ-amino acid and the δ-amino acid are amino acids having one or two additional methylene (amino acids having one, two, and three additional methylene in the main chain of a natural amino acid can each be understood as the β-amino acid, the γ-amino acid, and the δ-amino acid).

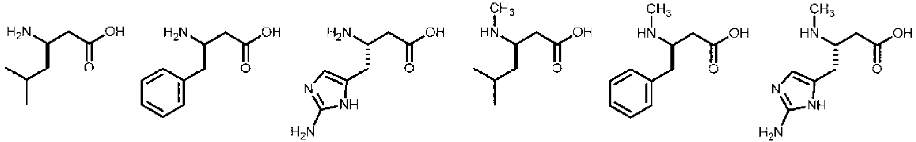

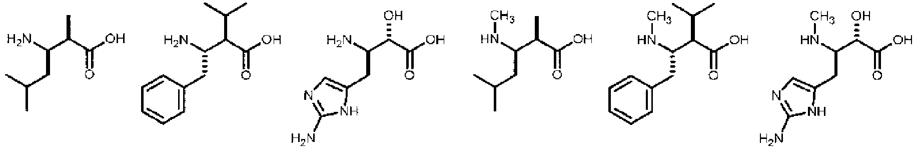

The long-chain amino acid may be a long-chain amino acid of a δ-amino acid or higher, and examples thereof include the following ones.

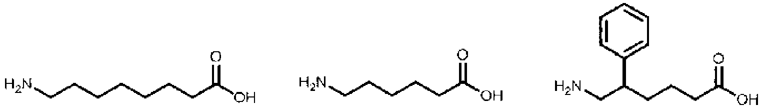

Examples of the α-hydroxy acid and the α-thio acid include the following ones.

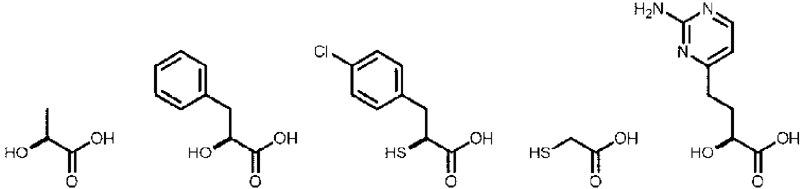

Examples of the cyclic amino acid (including an N-methylated cyclic amino acid) include the following ones.

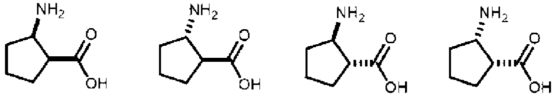

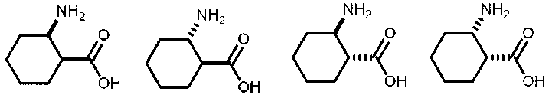

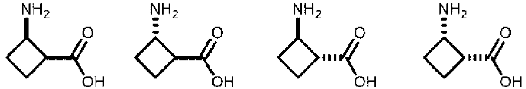

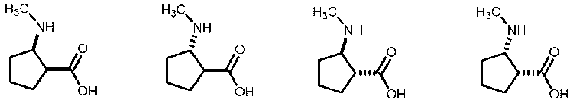

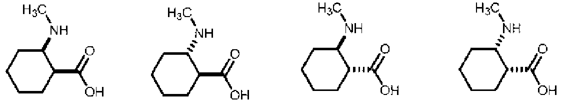

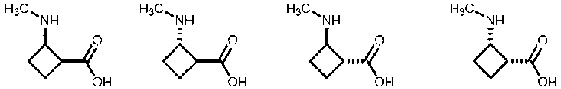

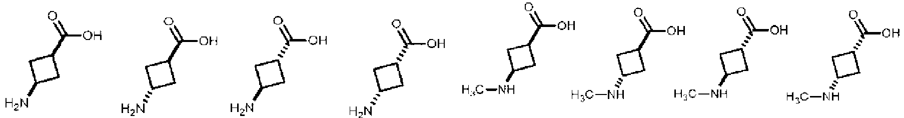

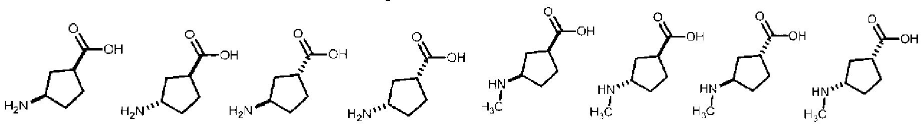

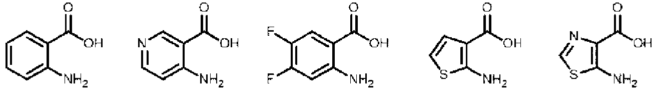

Examples of the amino acid having a structure consisting of a plurality of rings include the following ones.

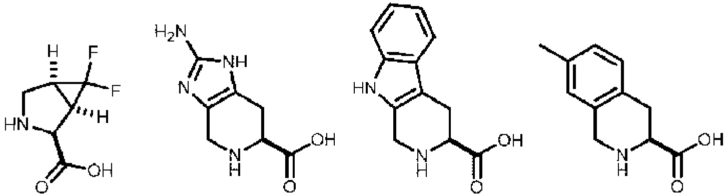

The azide group-containing amino acid, the alkyne group-containing amino acid, and the alkene group-containing amino acid are amino acids that can be used in a further ring-forming reaction after the introduction, and examples thereof include the following ones.

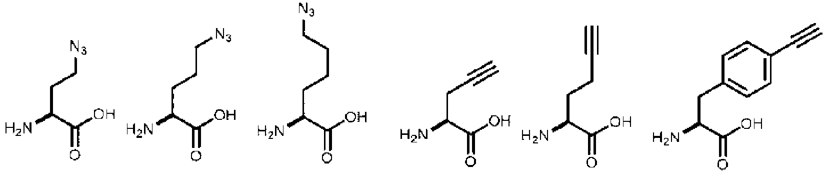

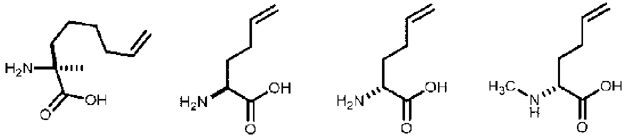

Examples of the amino acid having a structure of another side chain different from that of a natural amino acid include the following ones.

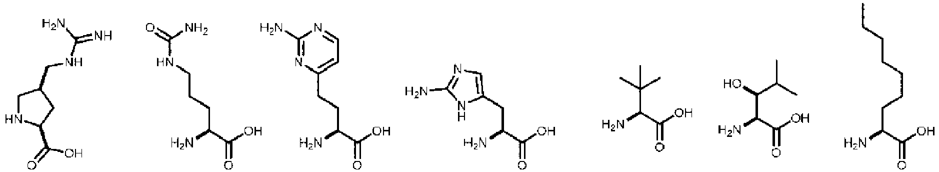

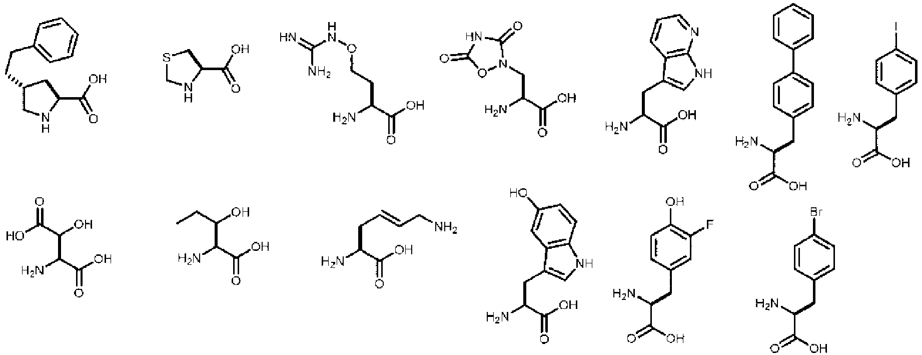

The non-proteinogenic amino acid is preferably selected from α,α-disubstituted amino acids, N-methyl amino acids, D-amino acids, β-amino acids, long-chain amino acids, α-hydroxy acids, α-thio acids, cyclic amino acids, selenocysteine, norleucine, spinacin, nitrophenylalanine, tetrahydroisoquinoline carboxylic acid, tetrahydroisoquinoline carboxylic acid having a substituent, such as hydroxytetrahydroisoquinoline carboxylic acid, hydroxytryptophan, pentafluorophenylalanine, methoxyphenylalanine, γ^{S,L} homoglutamine, amino acids having a structure consisting of a plurality of rings, azide group-containing amino acids, alkyne group-containing amino acids, alkene group-containing amino acids, chloroacetamide group-containing amino acids, ε-alkylated lysine, biotin group-containing amino acids, citrulline, ester group-containing amino acids, homoamino acids, cysteic acid, amino acids having the following structures, and D-forms and N-methylated forms in which the main chain amino group is methylated, of these amino acids.

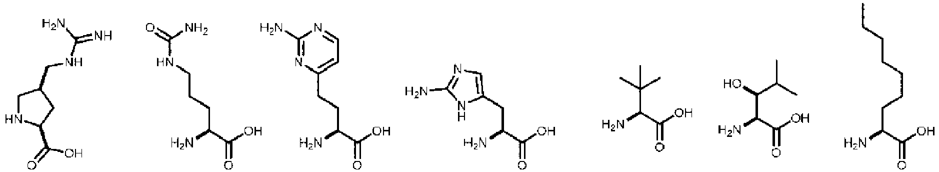

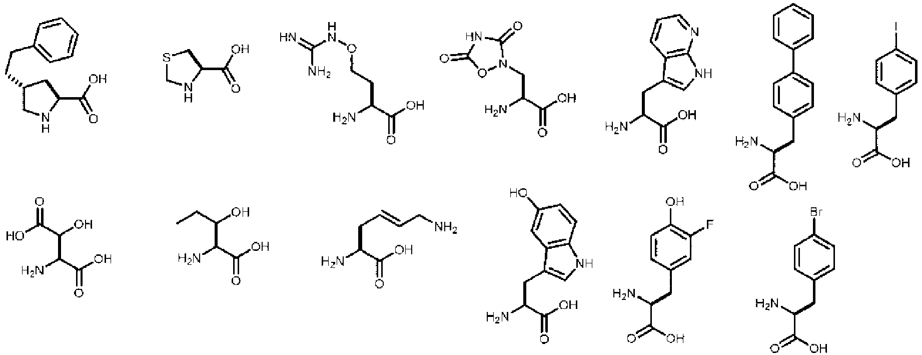

As the α,α-disubstituted amino acids, the N-methyl amino acids, the D-amino acids, the β-amino acids, the long-chain amino acids, the α-hydroxy acids, the α-thio acids, the cyclic amino acids, the amino acids having a structure consisting of a plurality of rings, the azide group-containing amino acids, the alkyne group-containing amino acids, and the alkene group-containing amino acids, the amino acids that are each exemplified above are preferable.

In addition, the amino acid derivative means a compound or partial structure derived from the amino acid defined above, and examples thereof include (i) an amino acid having a side chain functional group protected by a protective group, (ii) a thiazole ring or oxazole ring formed by a reaction of a side chain of an amino acid with an amide group between the side chain and an amino acid adjacent thereto in a peptide, (iii) a selenocysteine derivative having a group represented by the following formula:

-CR⁷₂-Se-R⁸ (3),

and (iv) an α,β-unsaturated amino acid.

Here, in Formula (3), Se represents selenium, R⁷'s each independently represent a hydrogen atom or a hydrocarbon group, and R⁸ represents a hydrocarbon group which may have a substituent. In Formula (3), R⁷'s are each independently preferably a hydrogen atom or a hydrocarbon group having 1 to 10 carbon atoms, and more preferably the hydrogen atom, an alkyl group having 1 to 3 carbon atoms, or an aryl group having 6 to 8 carbon atoms. In Formula (3), both of two R⁷'s may be hydrocarbon groups having 1 to 10 carbon atoms, or one may be a hydrogen atom and the other may be a hydrogen atom or a hydrocarbon group having 1 to 10 carbon atoms (more preferably the hydrogen atom, an alkyl group having 1 to 3 carbon atoms, or an aryl group having 6 to 8 carbon atoms). In addition, the hydrocarbon group of R⁸ is preferably a hydrocarbon group having 1 to 10 carbon atoms, and more preferably an alkyl group having 1 to 3 carbon atoms or an aryl group having 6 to 8 carbon atoms.

The substituent in R⁸ is not particularly limited, and examples thereof include a hydroxy group, a carboxy group, an amino group, a carbamoyl group, and the like.

Furthermore, the amino acid and the amino acid derivative may have the main chain amino group and/or the main chain carboxy group, which is protected by a protective group. Examples of such a protective group include an acetyl group, an allyl group (All), an allyloxycarbonyl group (Alloc), a benzyl group (Bzl), a benzyloxycarbonyl group (Z), a t-butyloxycarbonyl group (Boc), a benzyloxymethyl group (Bom), an o-bromobenzyloxycarbonyl group, a t-butyl group (tBu), a t-butyldimethylsilyl group, a 2-chlorobenzyl group, a 2-chlorobenzyloxycarbonyl group, a 2,6-dichlorobenzyl group, a cyclohexyl group, a cyclopentyl group, a 1-(4,4-dimethyl-2,6-dioxocyclohex-1-ylidene)ethyl group (Dde), an isopropyl group, a 4-methoxy-2,3-6-trimethylbenzylsulfonyl group (Mtr), a 2,3,5,7,8-pentamethylchroman-6-sulfonyl group (Pmc), a pivalyl group, a tetrahydropyran-2-yl group, a tosyl group (Tos), a 2,4,6-trimethoxybenzyl group, a trimethylsilyl group, a trityl group (Trt), and the like.

In the present specification, the thiazole ring or the oxazole ring formed by reaction of a side chain of an amino acid with an amide group between the side chain and an amino acid adjacent thereto in a peptide means a structure represented by the following formula. Here, in the following formula, B^{X} is an oxygen atom or a sulfur atom, and R^{X2} is a hydrogen atom or a hydrocarbon group. The hydrocarbon group of R^{X2} preferably has 1 to 10 carbon atoms, more preferably has 1 to 8 carbon atoms, still more preferably has 1 to 5 carbon atoms, and even still more preferably has 1 to 3 carbon atoms, and may have 1 carbon atom. In addition, the hydrocarbon group is preferably the alkyl group. R^{X2} may be the hydrogen atom, a methyl group, an ethyl group, a propyl group, an isopropyl group, a phenyl group, or a tosyl group, may be the hydrogen atom, the methyl group, the isopropyl group, or the phenyl group, and may be the hydrogen atom or the methyl group.

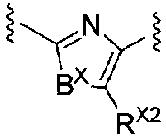

(ii) The thiazole ring or the oxazole ring may be present in the compound of the present embodiment in a form in which the thiazole rings and/or the oxazole rings are continuously bonded, for example. For example, in a case where the peptide includes (ii) the thiazole ring or the oxazole ring, the peptide may have the following structure.

Here, in the following formula, B^{X}'s are each independently an oxygen atom or a sulfur atom, R^{X2}'s are each independently a hydrogen atom or a hydrocarbon group, R^{X3} is a hydrogen atom or a hydrocarbon group, or forms a ring or a double bond together with R^{X1}, R^{X1} is a hydrogen atom, a side chain of an amino acid, or a side chain of an amino acid derivative, forms a ring together with R^{X3} or R^{X4}, or forms a double bond together with R^{X3}, R^{X4} is a hydrogen atom or a hydrocarbon group, or forms a ring together with R^{X1}, and j is an integer of 1 or more and 5 or less. The side chain of the amino acid derivative of R^{X1} may be a monovalent group derived from a side chain of an amino acid. Preferred aspects and the like of R^{X2} are each the same as those described above, and the hydrocarbon group of R^{X3} and R^{X4} preferably has 1 to 10 carbon atoms, more preferably has 1 to 5 carbon atoms, and still more preferably has 1 to 3 carbon atoms, and may have one carbon atom. In addition, the hydrocarbon group is preferably the alkyl group. The ring and the double bond formed by R^{X1} and R^{X3} or R^{X4} may be the same as the ring formed in a case where R³ forms a ring together with R⁴, the ring formed in a case where R³ forms a ring together with R⁵, and the group formed in a case where R³ forms a double bond together with R⁴, which will be described later. j is preferably 1 or more and 4 or less, more preferably 1 or more and 3 or less, and still more preferably 1 or more and 2 or less.

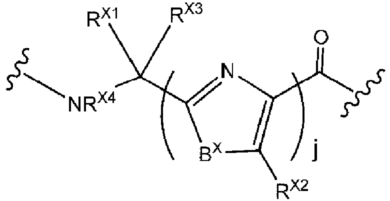

Examples of (iii) the selenocysteine derivative include a compound having the following structure. Here, in the following formula, definitions, preferred aspects thereof, and the like (including more preferable aspects, still more preferable aspects, even still more preferable aspects, and the like; the same applies hereinafter) of R⁷ and R⁸ are each the same as those in Formula (3).

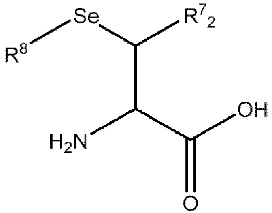

In addition, the compound having the above-described structure has the group represented by Formula (3) as a side chain of an α-amino acid, but a compound corresponding to a β-amino acid or a γ-amino acid of the compound (that is, a compound having the group represented by Formula (3) as a side chain of a β-amino acid or a γ-amino acid) is also included in (iii) the selenocysteine derivative.

Examples of (iv) the α,β-unsaturated amino acid include a compound having the following structure. Here, in the following formula, R⁷'s are each independently preferably a hydrogen atom or a hydrocarbon group having 1 to 10 carbon atoms, and more preferably the hydrogen atom, an alkyl group having 1 to 3 carbon atoms, or an aryl group having 6 to 8 carbon atoms. In the following formula, both of two R⁷'s may be hydrocarbon groups having 1 to 10 carbon atoms, or one may be a hydrogen atom and the other may be a hydrogen atom or a hydrocarbon group having 1 to 10 carbon atoms (more preferably the hydrogen atom, an alkyl group having 1 to 3 carbon atoms, or an aryl group having 6 to 8 carbon atoms).

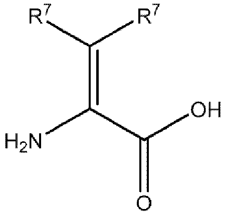

In a case where R² in Formula (I) includes an amino acid, an amino acid derivative, or a peptide chain, preferred examples of the amino acid or the amino acid derivative include, in addition to the proteinogenic amino acid and the above-described preferred non-proteinogenic amino acids, (i) an amino acid having a side chain functional group protected by a protective group, (ii) a thiazole ring or an oxazole ring formed by a reaction of a side chain of an amino acid with an amide group between the side chain and an amino acid adjacent thereto in a peptide, (iii) a selenocysteine derivative having a group represented by the following formula:

-CR⁷₂-Se-R⁸ (3),

(iv) an α,β-unsaturated amino acid, and the like.

In a case where R³ in Formula (I) is a side chain of an amino acid or an amino acid derivative, preferred examples of the amino acid or the amino acid derivative include, in addition to the proteinogenic amino acid and the above-described preferred non-proteinogenic amino acids, (i) an amino acid having a side chain functional group protected by a protective group, (iii) a selenocysteine derivative having a group represented by the following formula:

-CR⁷₂-Se-R⁸ (3),

and the like.

preferred examples of the amino acid or the amino acid derivative of Xaa in Formula (I) include, in addition to the proteinogenic amino acid and the above-described preferred non-proteinogenic amino acids, (i) an amino acid having a side chain functional group protected by a protective group, (ii) a thiazole ring or an oxazole ring formed by a reaction of a side chain of an amino acid with an amide group between the side chain and an amino acid adjacent thereto in a peptide, (iii) a selenocysteine derivative having a group represented by the following formula:

-CR⁷₂-Se-R⁸ (3),

(iv) an α,β-unsaturated amino acid, and the like.

Furthermore, with regard to the amino acid or amino acid derivative in a case of representing a group, in a case where the group is a monovalent group, the amino acid or amino acid derivative represents, for example, a monovalent group obtained by removing one hydrogen atom from the structure of the amino acid or amino acid derivative, and in a case where the group is a divalent group, the amino acid or amino acid derivative represents, for example, a divalent group obtained by removing two hydrogen atoms from the structure of the amino acid or amino acid derivative.

In addition, the amino acid or amino acid derivative in a case of representing a group may be an amino acid or amino acid derivative having one bonding site or may be an amino acid or amino acid derivative having two bonding sites. The same applies to the peptide chain, and the amino acid at the N-terminal of the peptide chain may have a bonding site and/or the amino acid at the C-terminal of the peptide chain may have a bonding site. In this case, a structure in which one hydrogen atom is subtracted from an amino group of an amino acid at an N-terminal and a carboxy group of an amino acid at a C-terminal may be a bonding site.

In Formula (I), R³ is a hydrogen atom, a side chain of an amino acid, or a side chain of an amino acid derivative, forms a ring together with R⁴ or R⁵, or forms a double bond together with R⁴. The side chain of the amino acid derivative may be a monovalent group derived from a side chain of an amino acid.

In Formula (I), in a case where R³ forms a ring together with R⁴, the ring thus formed is not particularly limited, but may be, for example, an aliphatic ring which may have a substituent, and the aliphatic ring may have 3 to 15 carbon atoms, 3 to 10 carbon atoms, or 4 to 8 carbon atoms. The substituent contained in the aliphatic ring may be, for example, a functional group contained in a side chain of an amino acid, and may be a hydrocarbon group (particularly, an alkyl group or an aryl group), a hydroxy group, an amino group, a carboxy group, a thiol group, an imidazolyl group, a guanidino group, a carbamoyl group, or an indolyl group.

In Formula (I), in a case where R³ forms a double bond with R⁴, the group thus formed is not particularly limited, but may be, for example, a group represented by =CR⁷₂. Here, R⁷'s are each independently a hydrogen atom or a hydrocarbon group, preferably the hydrogen atom or a hydrocarbon group having 1 to 10 carbon atoms, and more preferably the hydrogen atom, an alkyl group having 1 to 3 carbon atoms, or an aryl group having 6 to 8 carbon atoms.

In Formula (I), in a case where R³ forms a ring together with R⁵, the ring thus formed is not particularly limited, but may be, for example, a ring in which may not include a heteroatom other than a nitrogen atom to which R⁵ is bonded, such as a pyrrolidine ring, and the ring may have 3 to 10 carbon atoms, 3 to 6 carbon atoms, or 4 or 5 carbon atoms.

In Formula (I), R⁴ is a hydrogen atom or a hydrocarbon group, or forms a ring or a double bond together with R³. The hydrocarbon group of R⁴ preferably has 1 to 10 carbon atoms, more preferably has 1 to 5 carbon atoms, and still more preferably has 1 to 3 carbon atoms, and may have 1 carbon atom. In addition, the hydrocarbon group is preferably the alkyl group. In Formula (I), in a case where R⁴ does not form a ring together with R³, R⁴ may be the hydrogen atom or a methyl group.

In Formula (I), R⁵ is a hydrogen atom or a hydrocarbon group, or forms a ring together with R³. The hydrocarbon group of R⁵ preferably has 1 to 10 carbon atoms, more preferably has 1 to 5 carbon atoms, and still more preferably has 1 to 3 carbon atoms, and may have one carbon atom. In addition, the hydrocarbon group is preferably the alkyl group. R⁵ is preferably the hydrogen atom or forms a ring together with R³.

In Formula (I), (Xaa)ₘ is a peptide including m pieces of amino acids and/or amino acid derivatives, in which a C-terminal is bonded to NR⁵, and an N-terminal is bonded to a carbonyl bonded to the ring A in a case where k3 is 0, or bonded to a carbonyl bonded to the azole ring in a case where k3 is 1 or 2.

In Formula (I), m is an integer of 2 or more, may be, for example, 2 or more and 100 or less, may be 3 or more, 4 or more, 5 or more, 6 or more, 7 or more, 8 or more, 10 or more, 15 or more, or 20 or more, and may be 90 or less, 80 or less, 70 or less, 60 or less, 50 or less, 40 or less, 30 or less, or 25 or less within the above-described range. m may be in a range obtained by optionally combining the above-described upper limit value and lower limit value.

In Formula (I), in m pieces of amino acids and/or amino acid derivatives, m, m-1, m-2, m-3, m-4, m-5, m-6, m-7, m-8, m-9, m-10, or m-11 pieces of proteinogenic amino acids, or proteinogenic amino acids in number in a range in which these are the upper limit value or the lower limit value may be included. In addition, in m pieces of amino acids and/or amino acid derivatives, one, two, three, four, five, six, seven, eight, nine, ten, eleven, or a range where these are the upper limit value or the lower limit value of non-proteinogenic amino acids and/or amino acid derivatives may be included. The total number of non-proteinogenic amino acids and amino acid derivatives may be one, two, three, four, five, six, seven, eight, nine, ten, eleven, or a range where these are the upper limit value or the lower limit value.

The compound represented by Formula (I) is preferably represented by Formula (I-A) or (I-B), and more preferably represented by Formula (I-C). Furthermore, compounds represented by Formulae (I-A), (I-B), and (I-C) may be compounds produced by using the compounds represented by Formulae (1-A), (1-B), and (1-C) which will each be described later.

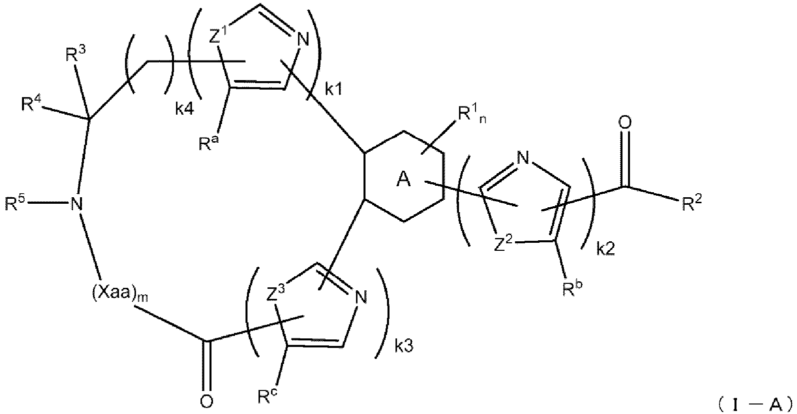

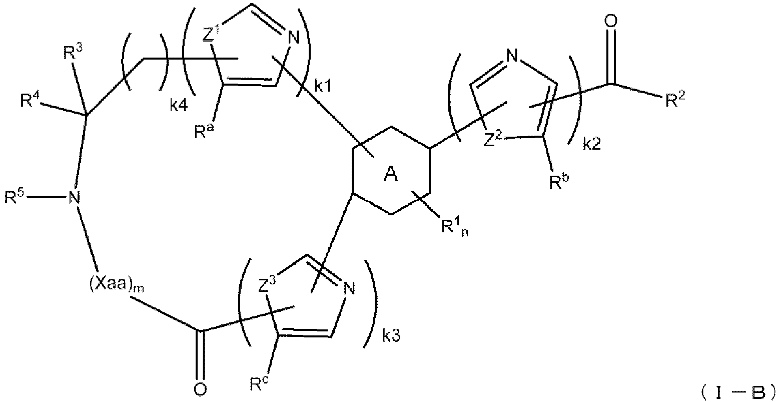

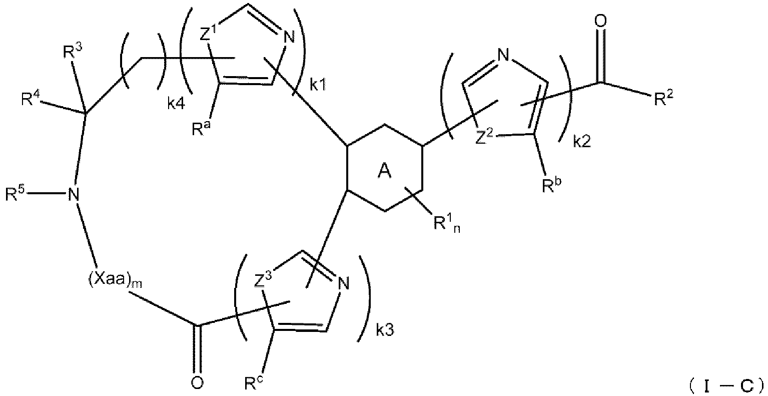

The compound represented by Formula (I) is preferably represented by Formula (I'). Furthermore, the compound represented by Formula (I') may be a compound produced by using a compound represented by Formula (1') which will be described later.

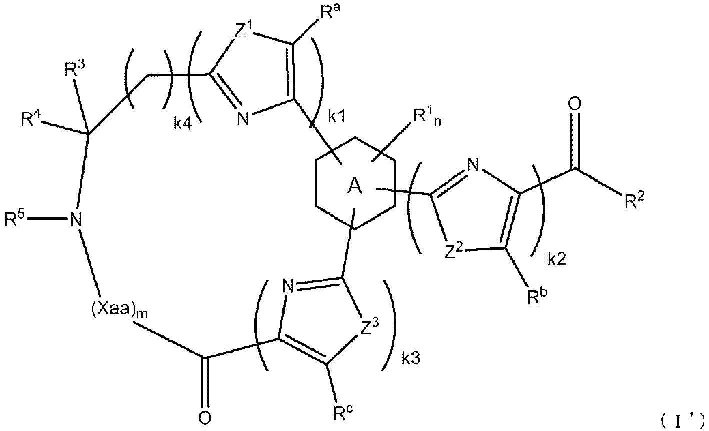

The compounds represented by Formulae (I-A), (I-B), and (I-C) are preferably represented by Formulae (I-A'), (I-B'), and (I-C'), respectively. The compound represented by Formula (I) is more preferably represented by Formula (I-C'). Furthermore, the compounds represented by Formulae (I-A'), (I-B'), and (I-C') may be compounds produced by using the compounds represented by Formulae (1-A'), (1-B'), and (1-C') which will be described later, respectively.

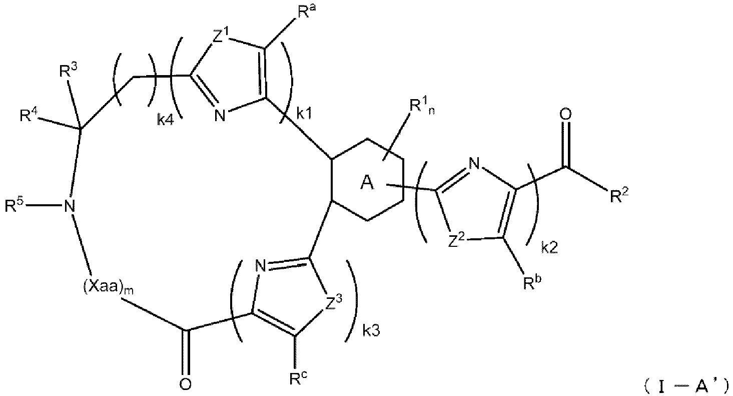

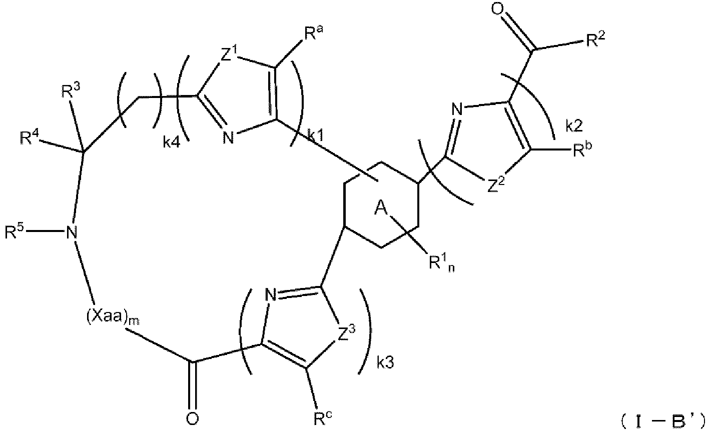

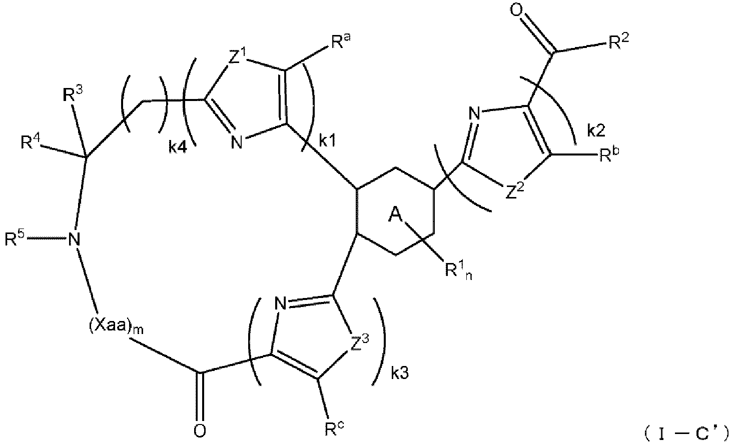

In Formulae (I'), (I-A), (I-B), (I-C), (I-A'), (I-B'), and (I-C'), definitions, preferred aspects, and the like of ring A, Z¹, Z², Z³, R^{a}, R^{b}, R^{c}, k1, k2, k3, k4, n, R¹, R², R³, R⁴, R⁵, (Xaa)ₘ, and m are each the same as those in Formula (I).

In Formulae (I-A), (I-B), (I-C), (I-A'), (I-B'), and (I-C'), in a case where the ring A is a pyridine ring,
in Formulae (I-A) and (I-A'), a nitrogen atom may be located at the following positions, 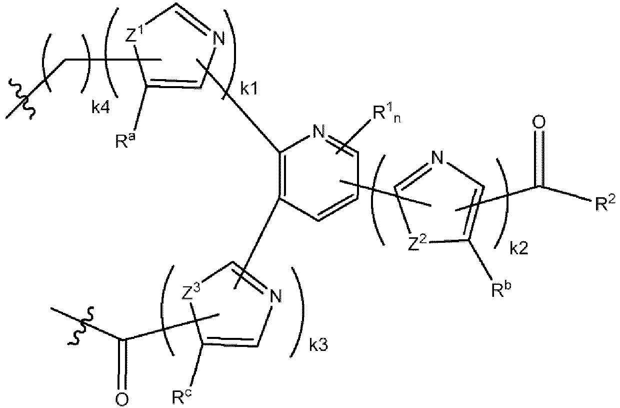 in Formulae (I-B) and (I-B'), a nitrogen atom may be located at the following position, and 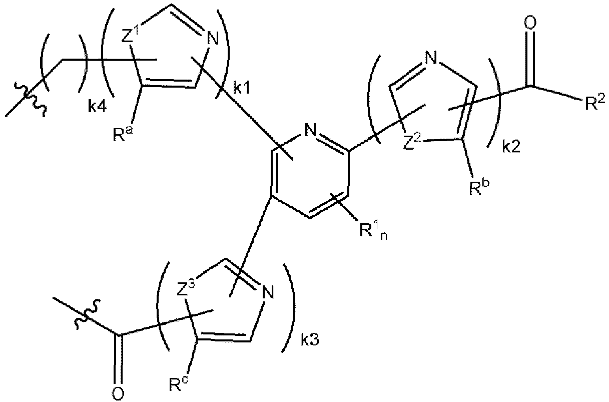 in Formulae (I-C) and (I-C'), a nitrogen atom may be located at the following positions.

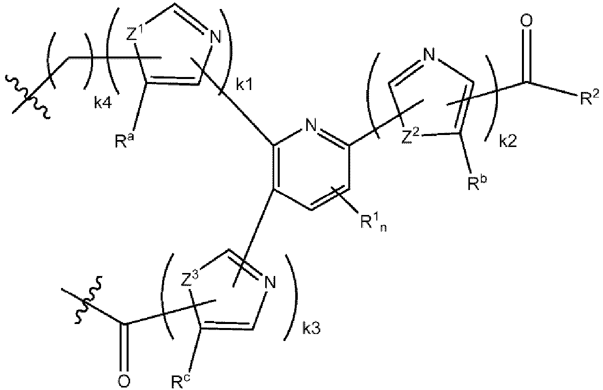

Furthermore, in the above-described structure, the portion after the wavy line is omitted.

### (Compound of Formula (1))

In the production method of the present embodiment, the compound represented by Formula (1) is used. In Formula (1), ring A, Z¹, Z², Z³, R^{a}, R^{b}, R^{c}, k1, k2, k3, k4, n, R¹, R², R³, R⁴, and R⁵ are each defined in the same manner as in Formula (I), and PG is a protective group. In addition, in Formula (1), preferred aspects and the like of the ring A, Z¹, Z², Z³, R^{a}, R^{b}, R^{c}, k1, k2, k3, k4, n, R¹, R², R³, R⁴, and R⁵ are also each the same as those in Formula (I), except for the following points.

In Formula (1), R² is a monovalent group. The monovalent group is not particularly limited, and examples thereof include a hydrogen atom, -OH, -OR^{2a}, -NH₂, -NHR^{2b}, -NR^{2b}₂, and other groups (protective groups) that can be eliminated by a deprotection treatment. R² is preferably -OR^{2a} or other groups that can be eliminated by a deprotection treatment.

Here, definitions, preferred aspects, and the like of R^{2a} and R^{2b} are each the same as those in Formula (I).

R² may be a group in which R^{2a} in -OR^{2a} is substituted with a substituent including a heteroatom, such as a group in which R^{2a} in -OR^{2a} is a 4-{N-[1-(4,4-dimethyl-2,6-dioxocyclohexylidene)-3-methylbutyl]-amino}benzyl group (Dmab group), a benzyl group having a substituent other than a hydrocarbon group, or a trityl group having a substituent other than a hydrocarbon group.

In Formula (1), R³ is a hydrogen atom, a side chain of an amino acid, or a side chain of an amino acid derivative, forms a ring together with R⁴ or R⁵, or forms a double bond together with R⁴, but it is preferable that R³ is the hydrogen atom, the side chain of the amino acid, or the side chain of the amino acid derivative, or forms a ring together with R⁴ or R⁵. The side chain of the amino acid derivative may be a monovalent group derived from a side chain of an amino acid.

In Formula (1), in a case where R³ forms a ring together with R⁴, the ring thus formed is not particularly limited, but may be defined in the same manner as the ring in Formula (I).

In Formula (1), in a case where R³ forms a double bond with R⁴, the group thus formed is not particularly limited, but may be defined in the same manner as the group in Formula (I).

In Formula (1), in a case where R³ forms a ring together with R⁵, the ring thus formed is not particularly limited, but may be defined in the same manner as the ring in Formula (I).

In a case where R³ in Formula (1) is a side chain of an amino acid derivative, it may be a group including selenium, such as a selenocysteine derivative side chain. In this case, R³ may be a monovalent group represented by the following formula:

-CR⁷₂-Se-R⁸ (3).

Here, in Formula (3), Se represents selenium, R⁷'s each independently represent a hydrogen atom or a hydrocarbon group, and R⁸ represents a hydrocarbon group which may have a substituent. In Formula (3), the hydrocarbon groups of R⁷ and R⁸ are each independently preferably a hydrocarbon group having 1 to 10 carbon atoms, and more preferably an alkyl group having 1 to 3 carbon atoms or an aryl group having 6 to 8 carbon atoms. In Formula (3), both of two R⁷'s may be hydrocarbon groups having 1 to 10 carbon atoms, or one may be a hydrogen atom and the other may be a hydrogen atom or a hydrocarbon group having 1 to 10 carbon atoms (more preferably the hydrogen atom, an alkyl group having 1 to 3 carbon atoms, or an aryl group having 6 to 8 carbon atoms).

The substituent in R⁸ is not particularly limited, and examples thereof include a hydroxy group, a carboxy group, an amino group, a carbamoyl group, and the like.

In Formula (1), R⁴ is a hydrogen atom or a hydrocarbon group, or forms a ring or a double bond together with R³, but is preferably the hydrogen atom or the hydrocarbon group, or forms a ring together with R³. The hydrocarbon group of R⁴ in Formula (1) preferably has 1 to 10 carbon atoms, more preferably has 1 to 5 carbon atoms, and still more preferably has 1 to 3 carbon atoms, and may have 1 carbon atom. In addition, the hydrocarbon group is preferably the alkyl group. In Formula (1), in a case where R⁴ does not form a ring together with R³, R⁴ may be the hydrogen atom or a methyl group.

In Formula (1), PG is a protective group. Examples of the protective group represented by PG include a 9-fluorenylmethylcarboxy group (Fmoc group), a t-butylcarbonyl group (Boc group), a benzyloxycarbonyl group (Cbz group), an allyloxycarbonyl group (Alloc group), and a (2-trimethylsilyl)-ethanesulfonyl group (SES group), and the Fmoc group is preferable. In addition, with regard to examples of the protective group represented by PG, selection of the protective group, and relevant information, reference can be made to the following documents: Greene's Protective Groups in Organic Synthesis (ISBN 9781118905074); Protecting Groups in Peptide Synthesis (DOI 10.1007/978-1-0716-0227-0_7); Amino Acids, Peptides and Proteins in Organic Chemistry: Building Blocks, Catalysis and Coupling Chemistry, Volume 3 (ISBN 9783527631803); Amino Acids, Peptides and Proteins in Organic Chemistry: Volume 4: Protection Reactions, Medicinal Chemistry, Combinatorial Synthesis, Volume 4 (ISBN:9783527631827); Solid-Phase Synthesis A Practical Guide (ISBN 9780367398712); Fmoc Solid Phase Peptide Synthesis: A Practical Approach (ISBN 9780199637249); Peptide Synthesis Methods and Protocols (ISBN 9781071602270); Peptide Synthesis and Applications (ISBN 9781627035439).

The ring A, Z¹, Z², Z³, R^{a}, R^{b}, R^{c}, k1, k2, k3, k4, n, R¹, and R⁵ in Formula (1) may be the same as the ring A, Z¹, Z², Z³, k1, k2, k3, k4, n, R¹, and R⁵ in Formula (I), respectively. R², R³, and R⁴ in Formula (1) may be the same as or different from R², R³, and R⁴ in Formula (I), respectively.

The compound represented by Formula (1) is preferably represented by Formula (1-A) or (1-B), and more preferably represented by Formula (1-C).

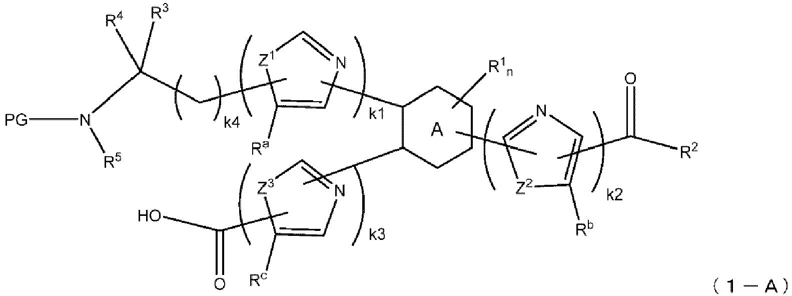

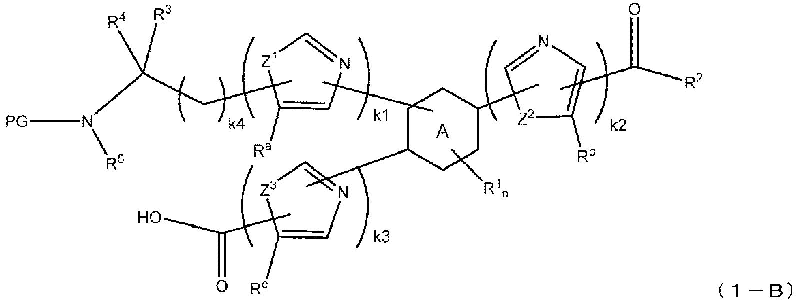

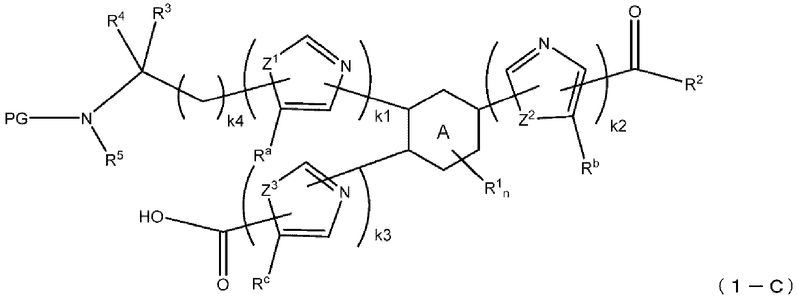

The compound represented by Formula (1) is preferably represented by Formula (1').

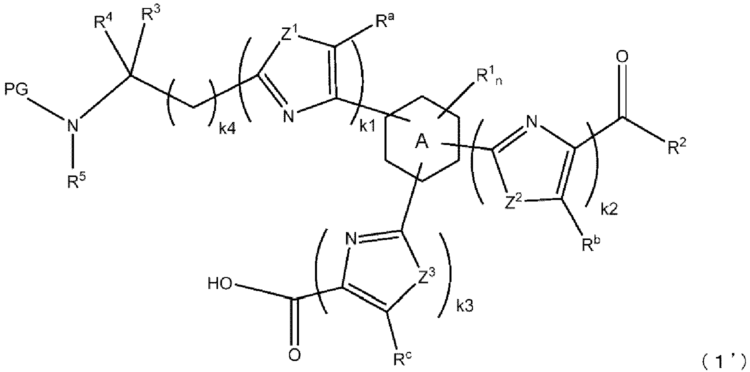

The compounds represented by Formulae (1-A), (1-B), and (1-C) are preferably represented by Formulae (1-A'), (1-B'), and (1-C'), respectively. The compound represented by Formula (1) is more preferably represented by Formula (1-C').

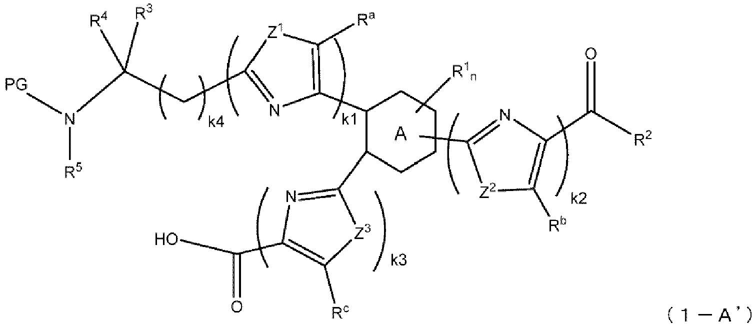

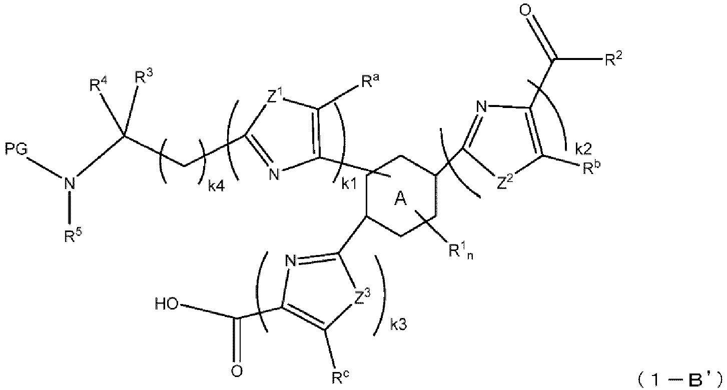

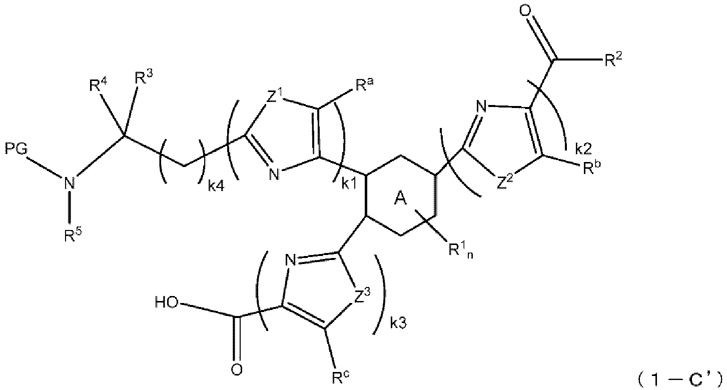

In Formulae (1'), (1-A), (1-B), (1-C), (1-A'), (1-B'), and (1-C'), definitions, preferred aspects, and the like of ring A, Z¹, Z², Z³, R^{a}, R^{b}, R^{c}, k1, k2, k3, k4, n, R¹, R², R³, R⁴, R⁵, and PG are each the same as those in Formula (1).

In Formulae (1-A), (1-B), (1-C), (1-A'), (1-B'), and (1-C'), in a case where the ring A is a pyridine ring,
in Formulae (1-A) and (1-A'), a nitrogen atom may be located at the following position, 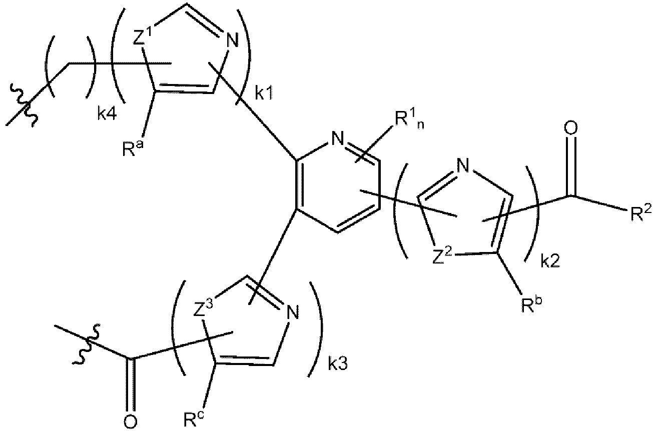 in Formulae (1-B) and (1-B'), a nitrogen atom may be located at the following position, and 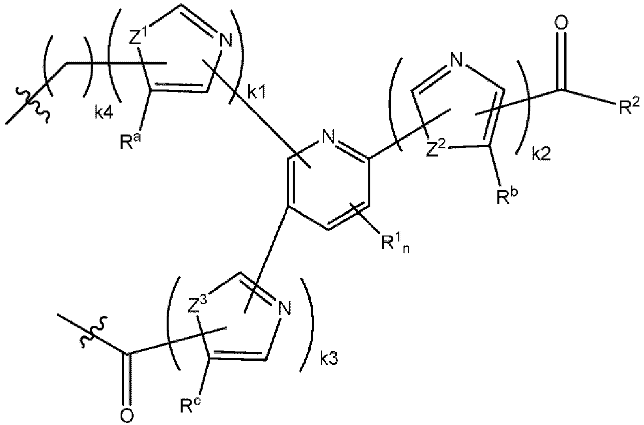 in Formulae (1-C) and (1-C'), a nitrogen atom may be located at the following positions.

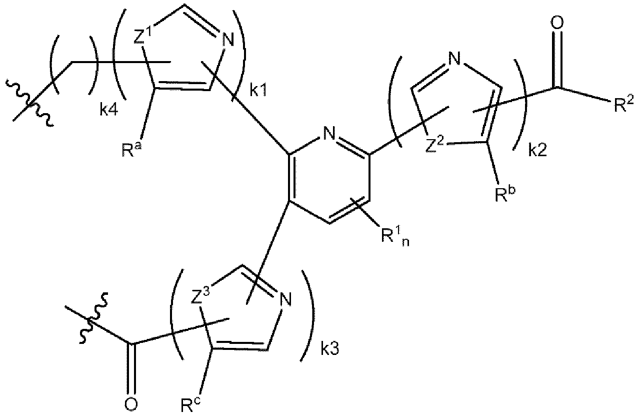

Furthermore, in the above-described structure, the portion after the wavy line is omitted.

### (Solid-Phase Carrier)

The solid-phase carrier that can be used in the production method of the present embodiment is not particularly limited as long as it is used in a solid-phase synthesis method, and examples thereof include inorganic carriers such as glass beads and silica gel; organic carriers consisting of a synthetic polymer such as a crosslinked polyvinyl alcohol, a crosslinked polyacrylate, a crosslinked polyacrylamide, and a crosslinked polystyrene, and/or polysaccharides such as crosslinked cellulose, crosslinked cellulose, crosslinked agarose, and crosslinked dextran; composite carriers such as an organic-organic carrier and an organic-inorganic carrier, which are obtained by a combination thereof; and the like.

A form of the solid-phase carrier can be any of a bead shape (which may be, for example, a magnetic bead, a paramagnetic bead, or a non-magnetic bead), a fibrous shape, a particle shape, a film shape (including a hollow fiber), a gel shape, a pin shape, a plate shape, and the like, and any form can be selected.

In addition, a method for immobilizing the amino acid, the amino acid derivative, and the peptide on the solid-phase carrier may be carried out by a covalent bonding method, a physical adsorption method, an ionic bonding method, an intermolecular interaction method, or the like.

In a case where the solid-phase carrier is a resin, with a classification based on a functional group presented on the carrier surface, examples thereof include a chloromethyl resin, a hydroxymethyl resin, a benzhydrylamine resin, an aminomethyl resin, a 4-benzyloxybenzyl alcohol resin (Wang resin), a 4-methylbenzhydrylamine resin, a PAM resin, a 4-hydroxymethylmethylphenylacetamidomethyl resin, a polyacrylamide resin, a 4-(2',4'-dimethoxyphenyl-hydroxymethyl)phenoxy resin, a 4-(2',4'-dimethoxyphenyl-Fmoc-aminomethyl)phenoxy resin (Rink resin), a 2-chlorotrityl chloride resin, and the like.

In addition, the solid-phase carrier may be a carrier such as a polystyrene resin, a NovaPEG resin, a ChemMatrix resin, a PEG resin, a TentaGel resin, and a SpheriTide resin.

In the production method of the present embodiment, a solid-phase carrier to which an amino acid or amino acid derivative, or a peptide, which has a free amino group, is bonded, or a solid-phase carrier having a free amino group is reacted with the compound represented by Formula (1). Therefore, in the production method of the present embodiment, the solid-phase carrier as it is may be reacted with the compound represented by Formula (1), or one or more amino acids or amino acid derivatives may be bonded to the solid-phase carrier by a solid-phase synthesis method, and then the amino acid or amino acid derivative having a free amino group, or a peptide, bonded to the solid-phase carrier, may be reacted with the compound represented by Formula (1). Therefore, the production method of the present embodiment may include reacting one or more amino acids or amino acid derivatives with the solid-phase carrier. Such a reaction may be carried out by a method used in a general solid-phase synthesis method. With regard to a method for bonding one or more amino acids or amino acid derivatives to a solid-phase carrier, and relevant information, reference can be made to the following documents: Greene's Protective Groups in Organic Synthesis (ISBN 9781118905074); Protecting Groups in Peptide Synthesis (DOI 10.1007/978-1-0716-0227-0_7); Amino Acids, Peptides and Proteins in Organic Chemistry: Building Blocks, Catalysis and Coupling Chemistry, Volume 3 (ISBN 9783527631803); Amino Acids, Peptides and Proteins in Organic Chemistry: Volume 4: Protection Reactions, Medicinal Chemistry, Combinatorial Synthesis, Volume 4 (ISBN:9783527631827); Solid-Phase Synthesis A Practical Guide (ISBN 9780367398712); Fmoc Solid Phase Peptide Synthesis: A Practical Approach (ISBN 9780199637249); Peptide Synthesis Methods and Protocols (ISBN 9781071602270); Peptide Synthesis and Applications (ISBN 9781627035439).

The number m1 of the amino acids or the amino acid derivatives to be bonded to the solid-phase carrier by the solid-phase synthesis method before the reaction with the compound represented by Formula (1) is not particularly limited, and may be, for example, 0 or more and 100 or less, and may be 1 or more, 2 or more, 3 or more, 4 or more, 5 or more, 6 or more, 7 or more, 8 or more, 10 or more, 15 or more, or 20 or more, and may be 90 or less, 80 or less, 70 or less, 60 or less, 50 or less, 40 or less, 30 or less, or 25 or less, within the above-described range. m1 may be in a range obtained by optionally combining the above-described upper limit value and lower limit value. In a case where m1 is 2 or more, a peptide chain consisting of m1 pieces of amino acids and/or amino acid derivatives is bonded to the solid-phase carrier, and the free amino group of the peptide chain reacts with the compound represented by Formula (1).

### (Reaction between Compound of Formula (1) and Solid-Phase Carrier)

The production method of the present embodiment includes reacting the compound of Formula (1) with the solid-phase carrier to obtain a compound represented by Formula (2).

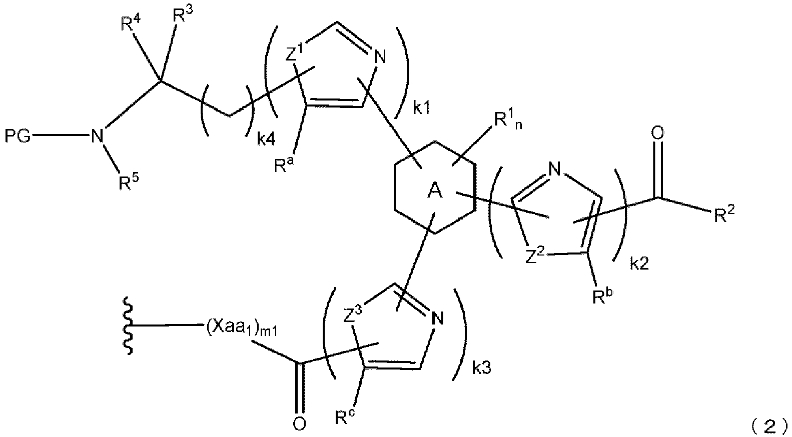

In Formula (2), ring A, Z¹, Z², Z³, R^{a}, R^{b}, R^{c}, k1, k2, k3, k4, n, R¹, R², R³, R⁴, and R⁵ are each defined in the same manner as in Formula (1), and preferred aspects and the like thereof are also the same as those in Formula (1).

The ring A, Z¹, Z², Z³, R^{a}, R^{b}, R^{c}, k1, k2, k3, k4, n, R¹, R², R³, R⁴, and R⁵ in Formula (2) may be the same as the ring A, Z¹, Z², Z³, R^{a}, R^{b}, R^{c}, k1, k2, k3, k4, n, R¹, R², R³, R⁴, and R⁵ in Formula (1), respectively.

In Formula (2), (Xaa₁)ₘ₁ represents an amino acid or amino acid derivative, or a peptide, which is bonded to a solid-phase carrier before the reaction with the compound represented by Formula (1). (Xaa₁)ₘ₁ is a peptide residue including m1 pieces of amino acids and/or amino acid derivatives bonded to the solid-phase carrier in a case where m1 is 2 or more, an amino acid residue or an amino acid derivative residue in a case where m1 is 1, or a single bond in a case where m1 is 0.

In Formula (2), m1 is an integer of 0 or more, and may be, for example, 0 or more and 100 or less, and may be 1 or more, 2 or more, 3 or more, 4 or more, 5 or more, 6 or more, 7 or more, 8 or more, 10 or more, 15 or more, or 20 or more, and may be 90 or less, 80 or less, 70 or less, 60 or less, 50 or less, 40 or less, 30 or less, or 25 or less, within the above-described range. m1 may be in a range obtained by optionally combining the above-described upper limit value and lower limit value.

Furthermore, in Formula (2), a wavy line indicates that (Xaa₁)ₘ₁ is bonded to a solid carrier directly or through a linker in a case where m1 is 1 or more, or a carbonyl is bonded to the solid carrier directly or through a linker in a case where m1 is 0. The linker is not particularly limited as long as it is a linker that is usually used for a solid-phase carrier.

The compound represented by Formula (2) is a compound in which 0 or more amino acids and/or amino acid derivatives are bonded to a carboxy group of the compound represented by Formula (1), and optionally, a solid-phase carrier is bonded to the carboxy group of the compound represented by Formula (1) through a linker. Therefore, in a case where Formula (1) is represented by Formula (1'), (1-A), (1-B), (1-C), (1-A'), (1-B'), or (1-C'), a compound having each of the corresponding three-dimensional configurations in Formula (2) can be obtained.

The reaction between the compound of Formula (1) and the solid-phase carrier is not particularly limited as long as it is a reaction used in a general solid-phase synthesis method. With regard to a method for reacting the compound of Formula (1) with the above-described solid-phase carrier, and relevant information, reference may be made to the following documents: Greene's Protective Groups in Organic Synthesis (ISBN 9781118905074); Protecting Groups in Peptide Synthesis (DOI 10.1007/978-1-0716-0227-0_7); Amino Acids, Peptides and Proteins in Organic Chemistry: Building Blocks, Catalysis and Coupling Chemistry, Volume 3 (ISBN 9783527631803); Amino Acids, Peptides and Proteins in Organic Chemistry: Volume 4: Protection Reactions, Medicinal Chemistry, Combinatorial Synthesis, Volume 4 (ISBN:9783527631827); Solid-Phase Synthesis A Practical Guide (ISBN 9780367398712); Fmoc Solid Phase Peptide Synthesis: A Practical Approach (ISBN 9780199637249); Peptide Synthesis Methods and Protocols (ISBN 9781071602270); and Peptide Synthesis and Applications (ISBN 9781627035439).

### (Amino Acid Condensation Reaction of Compound of Formula (2))

The production method of the present embodiment may further include deprotecting PG of the compound represented by Formula (2) before the compound obtained by deprotecting PG of the compound represented by Formula (2) is cleaved from the solid-phase carrier, and bonding one or more amino acids or amino acid derivatives to the deprotected compound by an amino acid condensation reaction to obtain a compound represented by Formula (4).

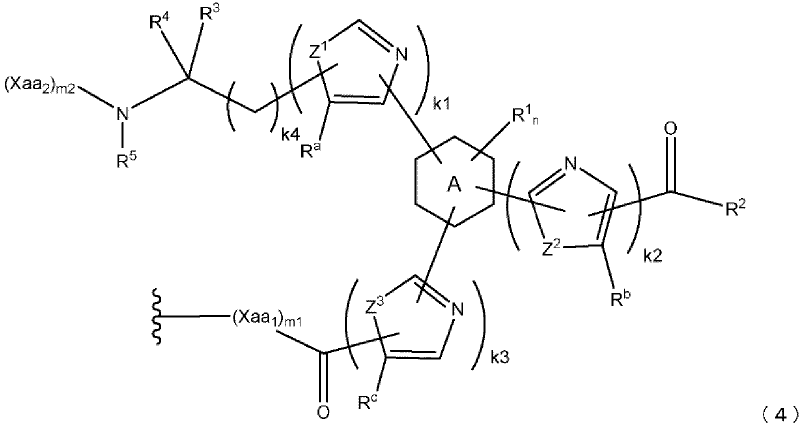

In Formula (4), ring A, Z¹, Z², Z³, R^{a}, R^{b}, R^{c}, k1, k2, k3, k4, n, R¹, R², R³, R⁴, R⁵, (Xaa₁)ₘ₁, and m1 are each defined in the same manner as in Formula (2), and preferred aspects and the like thereof are also the same as those in Formula (2).

The ring A, Z¹, Z², Z³, R^{a}, R^{b}, R^{c}, k1, k2, k3, k4, n, R¹, R², R³, R⁴, R⁵, (Xₐₐ₁)ₘ₁, and m1 in Formula (4) may be the same as the ring A, Z¹, Z², Z³, k1, k2, k3, k4, n, R¹, R², R³, R⁴, R⁵, (Xaa₁)ₘ₁, and m1 in Formula (2), respectively.

In Formula (4), (Xaa₂)ₘ₂ represents an amino acid or amino acid derivative, or a peptide, which is fused with the compound represented by Formula (2). (Xaa₂)ₘ₂ is a peptide residue including m1 pieces of amino acids and/or amino acid derivatives bonded to the solid-phase carrier in a case where m1 is 2 or more, or an amino acid residue or amino acid derivative residue in a case where m1 is 1. Furthermore, the N-terminal of (Xaa₂)ₘ₂ may be a free amino group or an amino group protected by a protective group. Examples of the protective group include the same ones as those for PG in Formula (2).

In Formula (4), m2 is an integer of 1 or more, may be, for example, 1 or more and 100 or less, may be 2 or more, 3 or more, 4 or more, 5 or more, 6 or more, 7 or more, 8 or more, 10 or more, 15 or more, or 20 or more, and may be 90 or less, 80 or less, 70 or less, 60 or less, 50 or less, 40 or less, 30 or less, or 25 or less. m2 may be a range obtained by optionally combining the above-described upper limit value and lower limit value.

It should be noted that in the compound represented by Formula (2), in a case where m1 is 0, m2 is 2 or more, and in a case where m1 is 1, m2 is 1 or more. That is, in Formula (4), the sum of m1 and m2 is 2 or more. The sum of m1 and m2 may be, for example, 2 or more and 100 or less, may be 3 or more, 4 or more, 5 or more, 6 or more, 7 or more, 8 or more, 10 or more, 15 or more, or 20 or more, and may be 90 or less, 80 or less, 70 or less, 60 or less, 50 or less, 40 or less, 30 or less, or 25 or less, in the above-described range. m2 may be a range obtained by optionally combining the above-described upper limit value and lower limit value. m2 may be a difference (m - m1) between m and m1.

The compound represented by Formula (4) is a compound in which one or more amino acids and/or amino acid derivatives are bonded, instead of PG of the compound represented by Formula (2). Therefore, in a case where Formula (1) is represented by Formula (1'), (1-A), (1-B), (1-C), (1-A'), (1-B'), or (1-C'), a compound having each of the corresponding three-dimensional configurations in Formula (4) can be obtained.

The deprotection of PG of the compound represented by Formula (2) may be carried out by an appropriate treatment depending on the type of PG which is a protective group. For example, in a case where PG is a 9-fluorenylmethylcarboxy group (Fmoc group), the deprotection may be performed by a treatment with a base such as pyrrolidine, piperidine, and morpholine, and in a case where PG is a t-butylcarbonyl group (Boc group), the deprotection may be performed by a treatment with a strong acid such as trifluoroacetic acid (TFA) and hydrochloric acid. With regard to a method for performing the deprotection and relevant information, reference may be made to the following documents: Greene's Protective Groups in Organic Synthesis (ISBN 9781118905074); Protecting Groups in Peptide Synthesis (DOI 10.1007/978-1-0716-0227-0_7); Amino Acids, Peptides and Proteins in Organic Chemistry: Building Blocks, Catalysis and Coupling Chemistry, Volume 3 (ISBN 9783527631803); Amino Acids, Peptides and Proteins in Organic Chemistry: Volume 4: Protection Reactions, Medicinal Chemistry, Combinatorial Synthesis, Volume 4 (ISBN:9783527631827); Solid-Phase Synthesis A Practical Guide (ISBN 9780367398712); Fmoc Solid Phase Peptide Synthesis: A Practical Approach (ISBN 9780199637249); Peptide Synthesis Methods and Protocols (ISBN 9781071602270); and Peptide Synthesis and Applications (ISBN 9781627035439).

As a method of reacting one or more amino acids or amino acid derivatives after deprotecting PG of the compound represented by Formula (2), a method usually used in a solid-phase synthesis method may be used. With regard to a method using the amino acid condensation reaction by solid-phase synthesis, and relevant information, reference may be made to the following documents: Greene's Protective Groups in Organic Synthesis (ISBN 9781118905074); Protecting Groups in Peptide Synthesis (DOI 10.1007/978-1-0716-0227-0_7); Amino Acids, Peptides and Proteins in Organic Chemistry: Building Blocks, Catalysis and Coupling Chemistry, Volume 3 (ISBN 9783527631803); Amino Acids, Peptides and Proteins in Organic Chemistry: Volume 4: Protection Reactions, Medicinal Chemistry, Combinatorial Synthesis, Volume 4 (ISBN:9783527631827); Solid-Phase Synthesis A Practical Guide (ISBN 9780367398712); Fmoc Solid Phase Peptide Synthesis: A Practical Approach (ISBN 9780199637249); Peptide Synthesis Methods and Protocols (ISBN 9781071602270); and Peptide Synthesis and Applications (ISBN 9781627035439).

In a case where the amino acid derivative is bonded in the above reaction, the amino acid may be bonded to the solid carrier or the compound represented by Formula (2), and then the bonded amino acid may be converted into the amino acid derivative. However, it is preferable that the amino acid derivative prepared in advance is bonded to the solid carrier or the compound represented by Formula (2). Examples of the compound to which the amino acid derivative is to be bonded include the following compounds. It should be noted that the compound to which the amino acid derivative is to be bonded is not limited to the following compounds, and various compounds can be synthesized and used in the production method according to the present embodiment, with appropriate reference to the following compounds and a method for synthesizing the same.

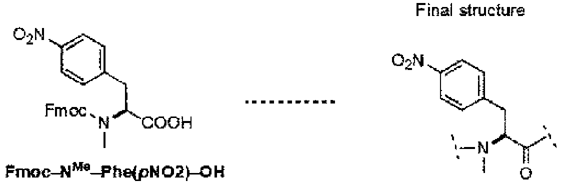

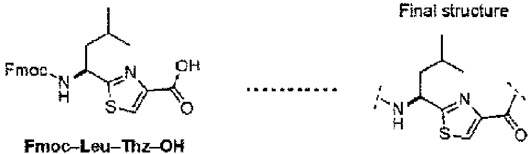

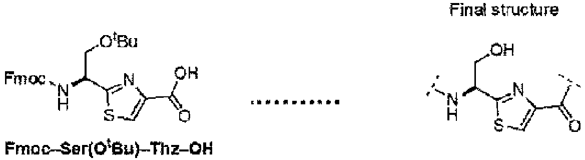

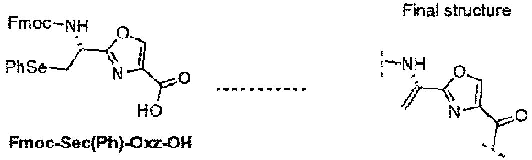

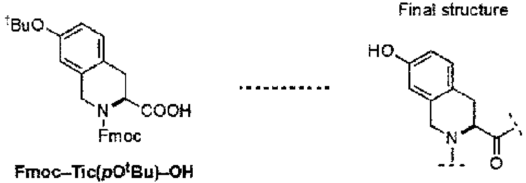

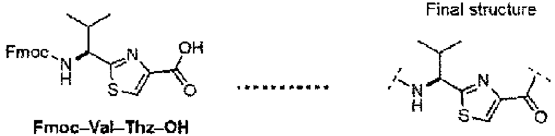

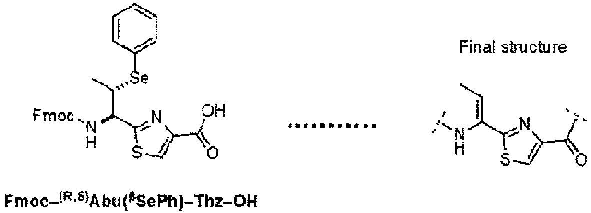

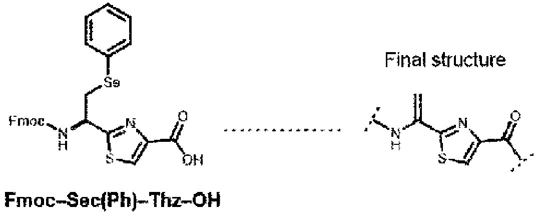

An example of a synthesis route for the compound is shown below.

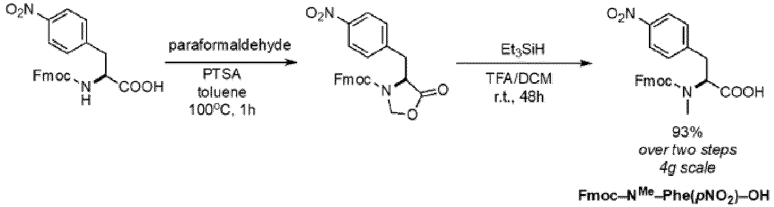

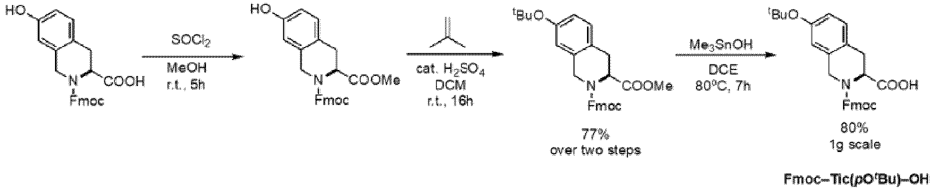

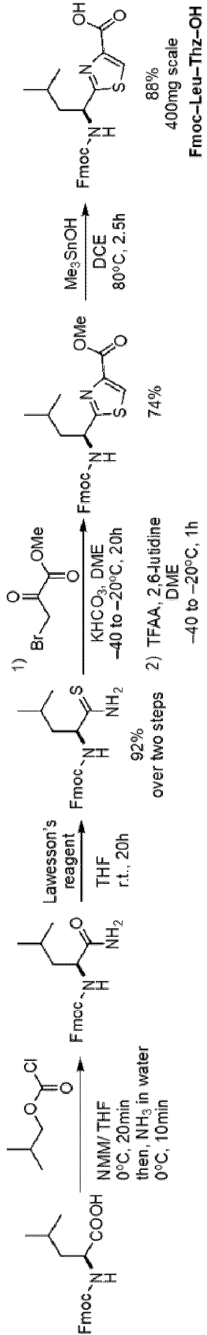

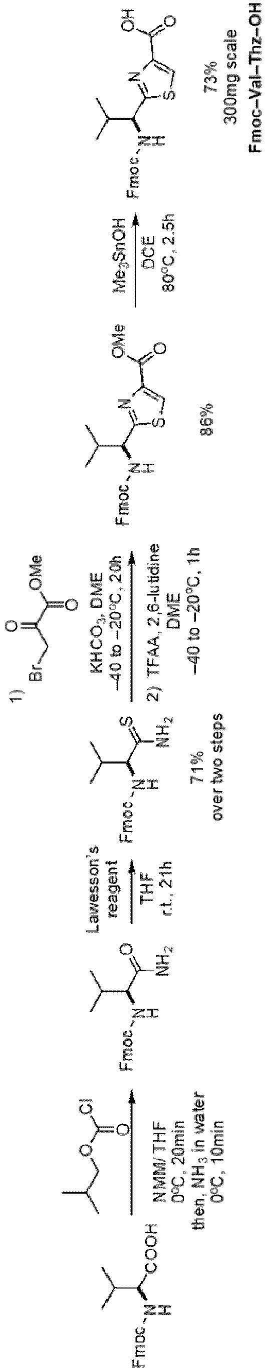

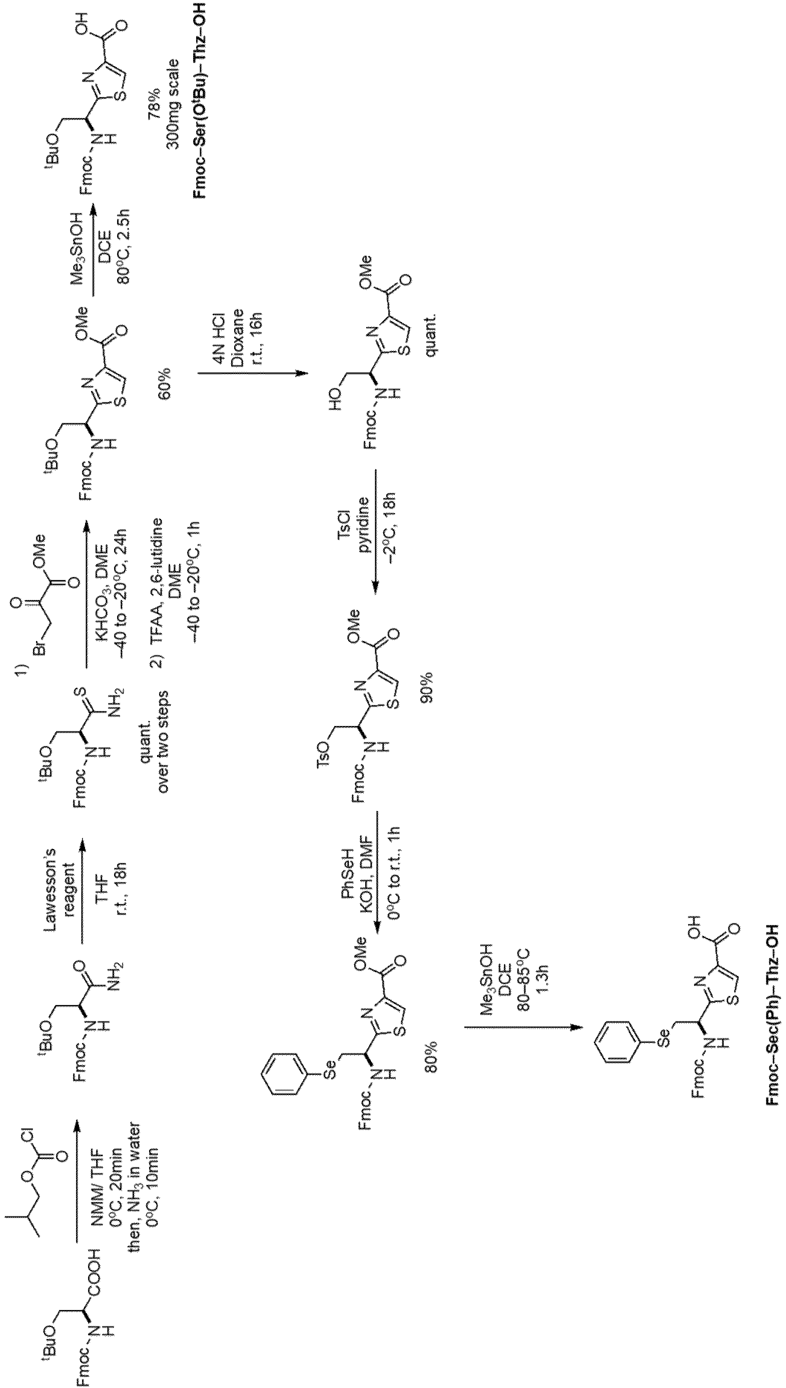

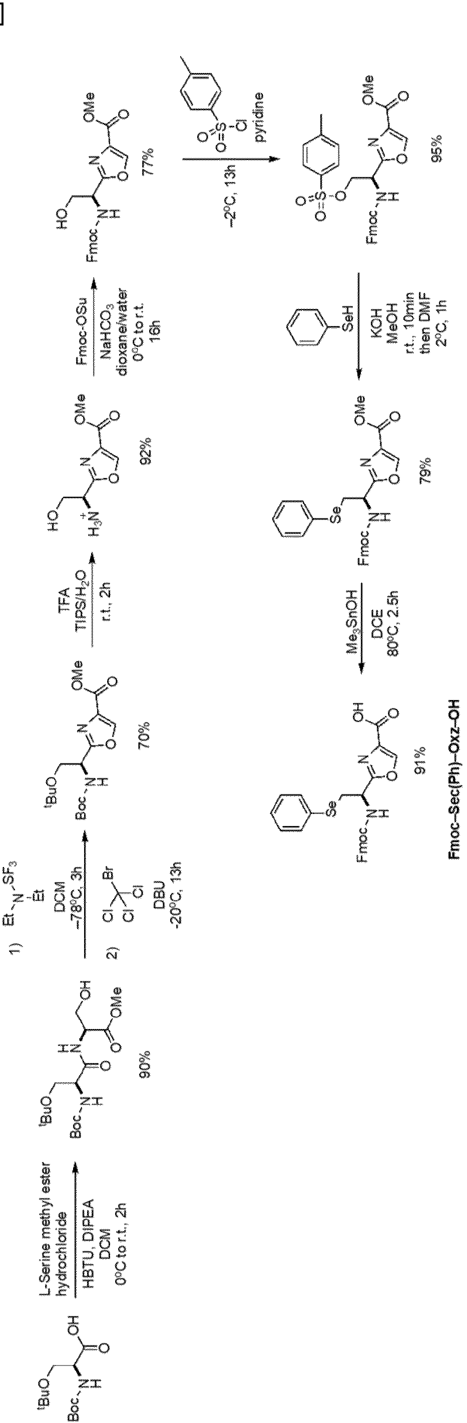

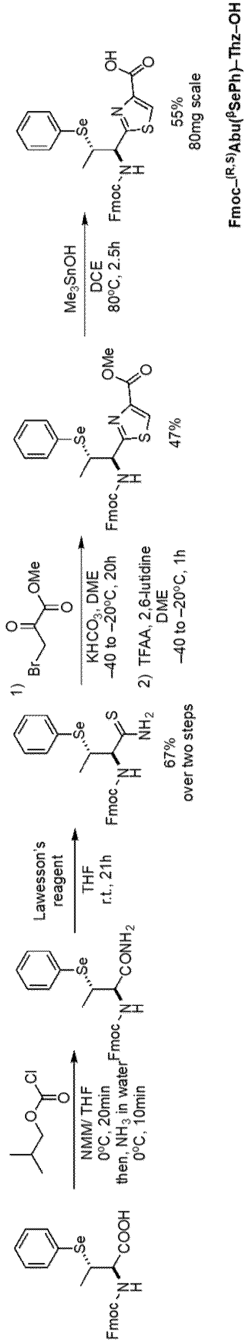

### (Cyclization Reaction)

The production method of the present embodiment includes, in a case of the above-described amino acid condensation reaction has been carried out, deprotecting the compound represented by Formula (4), as necessary, in a case where an N-terminal of (Xaa₂)ₘ₂ is protected by a protective group, followed by cleaving the compound from the solid-phase carrier to perform a cyclization reaction; and includes, in a case where the above-described amino acid condensation reaction has not been carried out, deprotecting PG of the compound represented by Formula (2), followed by cleaving the compound from the solid-phase carrier to perform a cyclization reaction. In this manner, in Formula (4), a carboxy group of (Xaa₁)ₘ₁ and an amino group of (Xaa₂)ₘ₂ are subjected to a condensation reaction, and in Formula (2), a carboxy group of (Xaa₁)ₘ₁ and -NHR⁵ are subjected to a condensation reaction, whereby the compound of Formula (1), which is a peptide consisting of m pieces of amino acids and/or amino acid derivatives and is a cyclic peptide including an aromatic 6-membered ring in the main backbone, can be obtained.

The deprotection of the N-terminal of (Xaa₂)ₘ₂ of the compound represented by Formula (4) and the deprotection of PG of the compound represented by Formula (2) may be carried out by an appropriate treatment depending on the protective group (PG). For example, in a case where the protective group is a 9-fluorenylmethylcarboxy group (Fmoc group), the deprotection may be performed by a treatment with a base such as pyrrolidine, piperidine, and morpholine, and in a case where the protective group is a t-butylcarbonyl group (Boc group), the deprotection may be performed by a treatment with a strong acid such as trifluoroacetic acid (TFA) and hydrochloric acid.

As a method for cleaving the compound represented by Formula (2) or (4) from the solid-phase carrier, an appropriate method may be used depending on the solid-phase carrier used. Examples thereof include a method using trifluoroacetic acid (TFA), acetic acid, 2,2,2-trifluoroethanol, diisobutylaluminum hydride (DIBAL), sodium borohydride, sodium hydroxide, an amine, or the like.

The cyclization reaction of the compound cleaved from the solid-phase carrier is not particularly limited, but may be performed, for example, under conditions in which an amino acid condensation reaction proceeds. With regard to a method used in a cyclization reaction and relevant information, reference may be made to the following documents: Greene's Protective Groups in Organic Synthesis (ISBN 9781118905074); Protecting Groups in Peptide Synthesis (DOI 10.1007/978-1-0716-0227-0_7); Amino Acids, Peptides and Proteins in Organic Chemistry: Building Blocks, Catalysis and Coupling Chemistry, Volume 3 (ISBN 9783527631803); Amino Acids, Peptides and Proteins in Organic Chemistry: Volume 4: Protection Reactions, Medicinal Chemistry, Combinatorial Synthesis, Volume 4 (ISBN:9783527631827); Solid-Phase Synthesis A Practical Guide (ISBN 9780367398712); Fmoc Solid Phase Peptide Synthesis: A Practical Approach (ISBN 9780199637249); Peptide Synthesis Methods and Protocols (ISBN 9781071602270); and Peptide Synthesis and Applications (ISBN 9781627035439). In addition, reference can also be made to the documents described in the following tables.

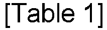

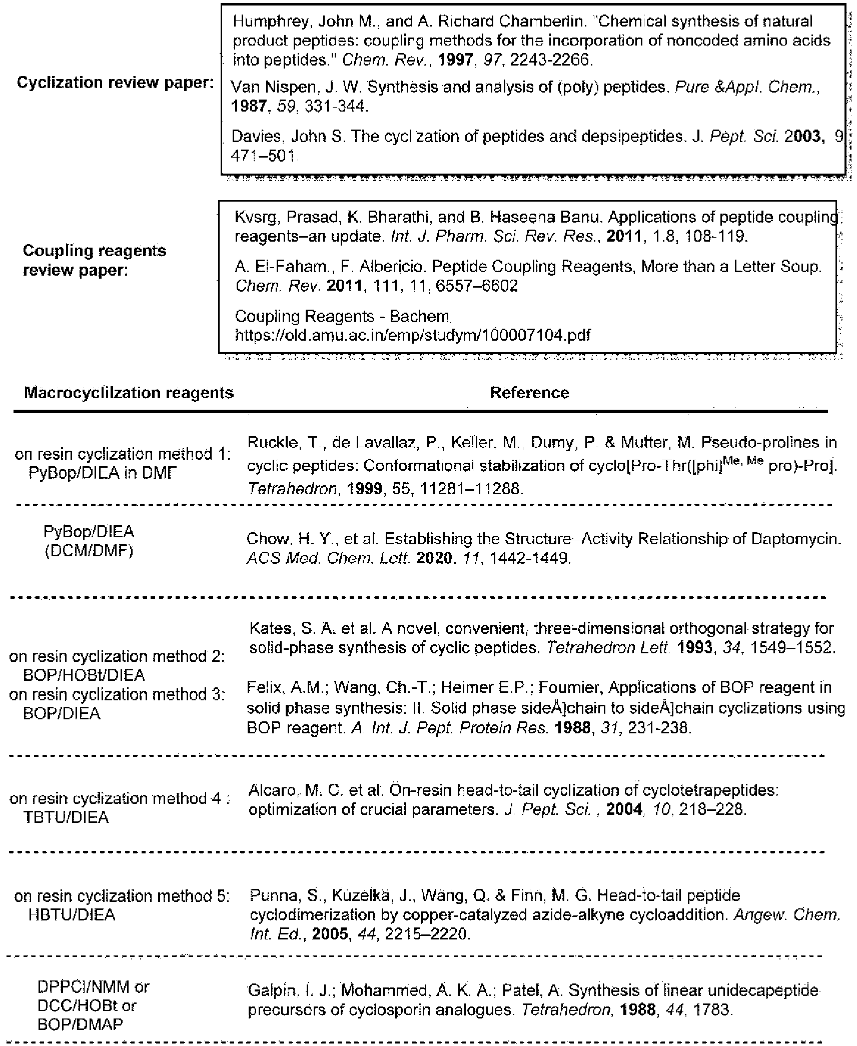

**[Table 2]**

| | |
|---|---|
| Step 1) | Rich, D. H.; Bhatnagar, P.; Mathiaparanam, P. Grant, J. A.; Tam |
| trichlorophenyl/DCC/pyridine | |
| Step 2) | Synthesis of tentoxin and related dehydro cyclic tetrapeptides. J. P. J. Org. Chem., 1978, 43, 296. |
| Boc deprotection then pyridine under 90oC | |
| DPPA/HOBt/DMAP/TEA | Edwards, J. V.; Lax, A. R.; Lillehoj, E. B., Boudreaux, G. New synthesis and biological activity of the cyclic tetrapeptides tentoxin and [Pro 1] tentoxin. J. Int.J. Peptide Protein Res., 1986, 28, 603. |
| DCC/DMAP/DMPA chloroform | Boden, Eugene P., and Gary E. Keck. "Proton-transfer steps in Steglich esterification: A very practical new method for macrolactonization" J. Org. Chem. Res., 1985, 50, 2394-2395. |
| | >Convergent, enaGrieco, P. A., Hon, Y. S., & Perez-Medrano, A. Convergent, enantiospecific total synthesis of the novel cyclodepsipeptide (+)-jasplakinolide (jaspamide). J. Am. Chem. Soc., 1988, 110, 1630-1631. |
| HAPyU | Ehrlich, A.; Rothemund, S.; Brudel, M.; Beyermann, M.; Carpino, L. A.; Bienert, M. Synthesis of cyclic peptides via efficient new coupling reagents. Tetrahedron Lett., 1993, 34, 4781. |
| DPPA/TEA or DIEA | Christy, M. P., et al. "Total synthesis of micrococcin P1 through scalable thiazole forming reactions of cysteine derivatives and nitriles." Org. lett., 2020, 22 ,2365-2370. |
| | Hughes, R. A., et al. Total synthesis of the thiopeptide antibiotic amythiamicin D. J. Am. Chem. Soc. 2006, 127, 15644-15651. |
| FDPP/DIEA (DCM/DMF) | Nicolaou, K. E., Dethe, D. H., Leung, G. Y., Zou, B., & Chen, D. Y. K. Total synthesis of thiopeptide antibiotics GE2270A, GE2270T, and GE2270C1. Chem. Asian J., 2008, 3, 413 - 429 |
| HOAt/HATU/DIEA | Zong, Y., Fang, F., Meyer, K.J et al. Gram-scale total synthesis of teixobactin promoting binding mode study and discovery of more potent antibiotics. Nat. Commun. 2019, 10, 3268. |
| HATU/DIEA/DMF or EDCI/DMAP (DCM/DMF) | Davies J. S., Howe J, Jayatilake J, Riley T. Synthesis and applications of cyclopeptides and depsipeptides. Lett. Pept. Sci., 1997; 4: 441-445. |

### (Deprotection Reaction of Amino Acid Side Chain Functional Group)

The production method of the present embodiment may further include deprotecting a protective group of a side chain functional group of the amino acid and/or amino acid derivative constituting (Xaa)ₘ of the compound obtained by the cyclization reaction. That is, the production method of the present embodiment may include eliminating the protective group of the side chain functional group of the amino acid and/or amino acid derivative constituting (Xaa)ₘ of the compound represented by Formula (I) obtained by the cyclization reaction to obtain the compound represented by Formula (I), from which the protective group has been removed. For such a deprotection reaction, a known appropriate reaction in the related art may be selected depending on the protective group of the side chain functional group of the amino acid or the amino acid derivative. With regard to a method for performing the deprotection and relevant information, reference may be made to the following documents: Greene's Protective Groups in Organic Synthesis (ISBN 9781118905074); Protecting Groups in Peptide Synthesis (DOI 10.1007/978-1-0716-0227-0_7); Amino Acids, Peptides and Proteins in Organic Chemistry: Building Blocks, Catalysis and Coupling Chemistry, Volume 3 (ISBN 9783527631803); Amino Acids, Peptides and Proteins in Organic Chemistry: Volume 4: Protection Reactions, Medicinal Chemistry, Combinatorial Synthesis, Volume 4 (ISBN:9783527631827); Solid-Phase Synthesis A Practical Guide (ISBN 9780367398712); Fmoc Solid Phase Peptide Synthesis: A Practical Approach (ISBN 9780199637249); Peptide Synthesis Methods and Protocols (ISBN 9781071602270); and Peptide Synthesis and Applications (ISBN 9781627035439).

### (Double Bond Forming Reaction)

The production method of the present embodiment may further include oxidizing a monovalent group represented by the following formula:

-CR⁷₂-Se-R⁸ (3),

contained in the compound obtained by the cyclization reaction to form a double bond.

Here, in Formula (3), Se represents selenium, R⁷'s each independently represent a hydrogen atom or a hydrocarbon group, and R⁸ represents a hydrocarbon group which may have a substituent.

In Formula (3), R⁷'s are each independently preferably a hydrogen atom or a hydrocarbon group having 1 to 10 carbon atoms, and more preferably the hydrogen atom, an alkyl group having 1 to 3 carbon atoms, or an aryl group having 6 to 8 carbon atoms. In Formula (3), both of two R⁷'s may be hydrocarbon groups having 1 to 10 carbon atoms, or one may be a hydrogen atom and the other may be a hydrogen atom or a hydrocarbon group having 1 to 10 carbon atoms (more preferably the hydrogen atom, an alkyl group having 1 to 3 carbon atoms, or an aryl group having 6 to 8 carbon atoms).

In Formula (3), the hydrocarbon group of R⁸ is preferably a hydrocarbon group having 1 to 10 carbon atoms, and more preferably an alkyl group having 1 to 3 carbon atoms or an aryl group having 6 to 8 carbon atoms.

The substituent in R⁸ is not particularly limited, and examples thereof include a hydroxy group, a carboxy group, an amino group, a carbamoyl group, and the like.

The production method of the present embodiment can include the double bond forming reaction as described above, thereby forming a group represented by =CR⁷₂. Therefore, for example, in a case where R³ of the compound represented by Formula (I) obtained by the cyclization reaction is a group represented by Formula (3), such as a selenocysteine derivative side chain, and in a case where R⁴ is a hydrogen atom, R³ and R⁴ can be bonded to each other to form a double bond, thereby obtain the compound represented by Formula (I) including a group represented by =CR⁷₂.

The group represented by Formula (3) may be bonded to a carbon atom to which at least one hydrogen atom is bonded, in addition to R³, in the compound represented by Formula (I). For example, the compound represented by Formula (I) obtained by the cyclization reaction may have a side chain of the amino acid derivative in (Xaa)ₘ, and in a case where R² includes an amino acid derivative or a peptide chain, the compound may have the group represented by Formula (3) as the side chain of the amino acid derivative. Therefore, for example, by subjecting the compound represented by Formula (I), which has a group represented by Formula (3) at the side chain of the amino acid derivative in (Xaa)ₘ, to a double bond forming reaction, the compound represented by Formula (I), which includes a group represented by =CR⁷₂, can be obtained as the amino acid derivative in (Xaa)ₘ.

Examples of a method for oxidizing the group represented by Formula (3) to form a double bond include a method using organic peroxides such as tert-butyl hydroperoxide (TBHP), sodium periodate, hydrogen peroxide, benzoyl peroxide, lauroyl peroxide, meta-chloroperbenzoic acid (mCPBA), and peracetic acid, or peroxides such as NaClO, KHSO₅, and Oxone. More specifically, the methods described in Just-Baringo, X. et al., Angew. Chem. Int. Ed. 2013, 52, 7818-7821; Just-Baringo, X. et al., J. Med. Chem. 2014, 57, 4185-4195; and the like may be used.

Such a double bond forming reaction may be carried out immediately after the cyclization reaction or may be carried out after the deprotection reaction of the amino acid side chain functional group.

### (C-Terminal Modification Reaction)

The production method of the present embodiment may further include eliminating R² of the compound represented by Formula (I) obtained by the cyclization reaction, and reacting a carboxy group generated by the elimination with one or more of compounds.

R² to be eliminated is not particularly limited, and examples thereof include -OR^{2a} or other groups that can be eliminated by a deprotection treatment,. Here, a definition, preferred aspects, and the like of R^{2a} are each the same as those in Formula (I).

R² to be eliminated may be a group in which R^{2a} in -OR^{2a} is substituted with a substituent including a heteroatom, such as a group in which R^{2a} in -OR^{2a} is a 4-{N-[1-(4,4-dimethyl-2,6-dioxocyclohexylidene)-3-methylbutyl]-amino}benzyl group (Dmab group), a benzyl group having a substituent other than a hydrocarbon group, or a trityl group having a substituent other than a hydrocarbon group.

Although a method for eliminating R² is not particularly limited, for example, in a case where R² is -OR^{2a}, the method may be a hydrolysis reaction of an ester, and in particular, in a case where R² is -OMe, hydrolysis using Me₃SnOH may be used. With regard to details of the hydrolysis method using Me₃SnOH, reference can be made to K. C. Nicolaou, et al., Angew. Chem. Int. Ed., 2005, 44, 1378-1382.

The compound to be reacted with the carboxy group generated by the elimination of R² is not particularly limited as long as it is a compound which can be reacted with the carboxy group. In addition, the first compound that can react with a carboxy group may be reacted, and the second compound that can react with the functional group contained in the first compound may be further reacted.

The compound that is reacted with the carboxy group generated by the elimination of R² may be, for example, amines, compounds including a chloroalkyl moiety, compounds including a labeling substance, compounds including an ethylene glycol (PEG) moiety, compounds including a hydrocarbon chain (which may have, for example, 1 to 20 carbon atoms or 3 to 10 carbon atoms) moiety, amino acids and amino acid derivatives, and peptide chains. Examples of such R² include NH₂R^{2b}, NHR^{2b}₂, NH₂R^{2c}, and salts thereof. Here, a definition, preferred aspects, and the like of R^{2b} are each the same as those in Formula (I).

A definition, preferred aspects, and the like of R^{2c} are each the same as those in Formula (I).

By configuring the production method of the present embodiment to include the C-terminal modification reaction as described above, to the compound represented by Formula (I), it is possible to impart a labeling substance useful for detection or quantification, impart a chloroalkane tag, which can evaluate cell membrane permeability, impart a polyethylene glycol moiety capable of improving water solubility, or impart a hydrocarbon chain capable of improving blood stability.

Such a C-terminal modification reaction may be carried out immediately after the cyclization reaction, may be carried out after the deprotection reaction of the amino acid side chain functional group, or may be carried out after the double bond forming reaction.

### [Compound of Formula (1) and Production Method for Same]

The present invention also provides the compound represented by Formula (1). The compound represented by Formula (1) is useful, for example, as a starting material of the above-described production method of the present embodiment. In addition, with regard to each of the following steps, appropriate reference may be made to M. Christy et al., Org. Lett. 2020, 22, 2365-2370.

The compound represented by Formula (1) is not particularly limited, and can be produced, for example, by the following scheme. Furthermore, in the following synthesis scheme, ring A, Z¹, Z², Z³, k1, k2, k3, k4, n, R¹, R², R³, R⁴, R⁵, and PG are each defined in the same manner as in Formula (1). In addition, each of the following schemes may be appropriately combined.

### (Scheme 1: Synthesis of Compound of Formula (1) (Intermediate 1))

An intermediate 1, which is the compound of Formula (1), can be synthesized as follows.

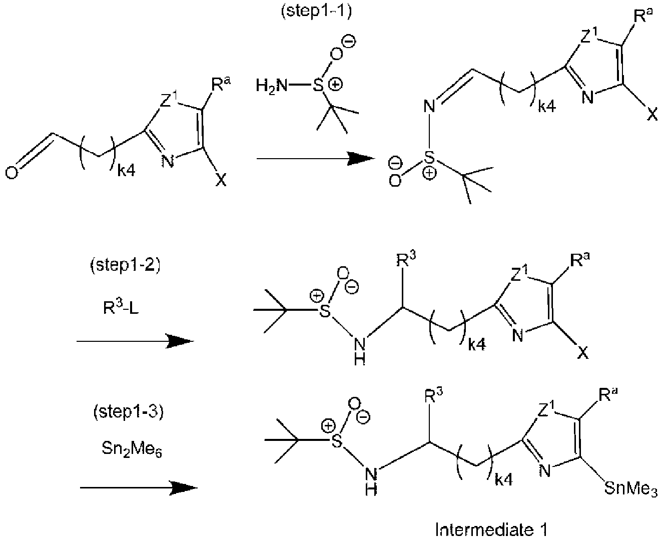

Step 1-1 is a step of reacting tert-butanesulfinamide with a starting material. This step may be performed with dichloromethane (DCM) including Cs₂CO₃. X is an appropriate leaving group such as I, Br, OTf (triflate group), and Cl.

Step 1-2 is a step of reacting the product of Step 1-1 with R³-L (in which L is an appropriate leaving group) corresponding to an amino acid side chain or an amino acid derivative side chain. Examples of R³-L include bis(methylseleno)methane. This step may be performed at a low temperature of -50°C or lower in tetrahydrofuran (THF) including a reducing agent such as ⁿBuLi.

Step 1-3 is a step of reacting the product of Step 1-2 with hexamethylditin. Hexamethylditin can be a hexalkyltin. This step may be performed under a heating condition of 50°C or higher in toluene including a palladium catalyst such as Pd[PPh₃]₄.

### (Scheme 2: Synthesis of Compound Having Pyridine Ring in Formula (I) Using Intermediate 1)

The compound represented by Formula (I) having a pyridine ring can be synthesized as follows by using the intermediate 1 synthesized in Scheme 1.

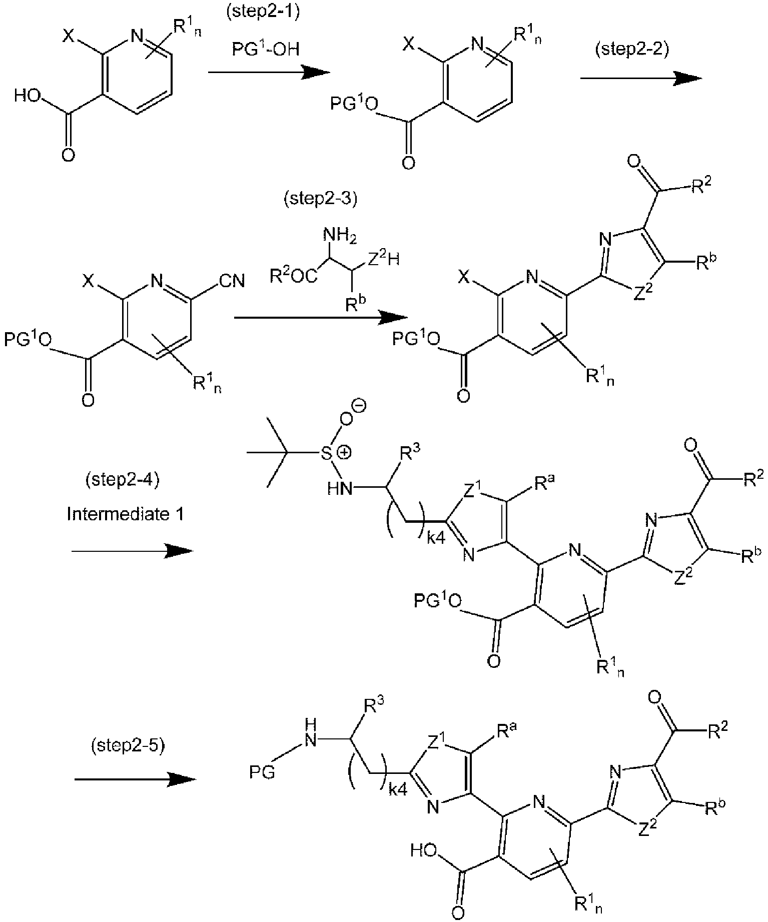

Step 2-1 is a step of protecting a carboxy group of the starting material with a protective group. The protective group represented by PG¹ is not particularly limited as long as it is a group that has been used in the related art as a protective group of a carboxy group, and may be, for example, a tert-butyl group, a benzyl group, a methyl group, an alkyl group, or the like. This step may be a step of reacting PG¹-OH with the product after the tosylation of the carboxy group with paratoluenesulfonyl chloride and a base such as pyridine.

Step 2-2 is a step of cyanating the product of Step 2-1. This step may include, for example, a first reaction with urea-hydrogen peroxide (UHP) and trifluoroacetic acid anhydride (TFAA), and a second reaction with trimethylsilyl cyanide under basic conditions such as triethylamine.

Step 2-3 is a step of forming a thiazole ring or an oxazole ring by reacting the product of Step 2-2 with threonine, cysteine, or serine, or a derivative thereof. This reaction may include a first reaction in which a nitrile is reacted with threonine, cysteine, or serine, or a derivative thereof to form a thiazoline ring or an oxazoline ring, and a second reaction in which the thiazoline ring or the oxazoline ring is dehydrogenated to form a thiazole ring or an oxazole ring. The dehydrogenation of the thiazoline ring or the oxazoline ring may be performed using, for example, bromotrichloromethane and diazabicycloundecene (DBU).

With regard to the reaction conditions and the like in Steps 2-1 to 2-3, reference can be made to Christy, M. P. et al., Org. Lett. 2020, 22, 2365-2370.

Step 2-4 is a step of coupling the product of Step 2-3 with the intermediate 1 obtained in Scheme 1. This step may be a Stille coupling using a palladium catalyst such as Pd[P^{t}Bu₃]₂. In the Stille coupling, CsF and Cul may be added as an additive. The reaction temperature may be a heating condition of 50°C or higher.

Step 2-5 is a step of converting the product of Step 2-4 into the compound represented by Formula (1). This step may include a first reaction of removing the sulfinamide moiety with a strong acid such as trifluoroacetic acid (TFA) and hydrochloric acid, and deprotecting PG¹ which is a protective group of a carboxy group, and a second reaction of protecting an amino group with a protective group PG.

### (Scheme 3: Synthesis of Compound Having Benzene Ring in Formula (I) Using Intermediate 1)

The compound represented by Formula (I) having a benzene ring can be synthesized as in the following schemes 3-1, 3-2, and 3-3 using the intermediate 1 synthesized in Scheme 1.

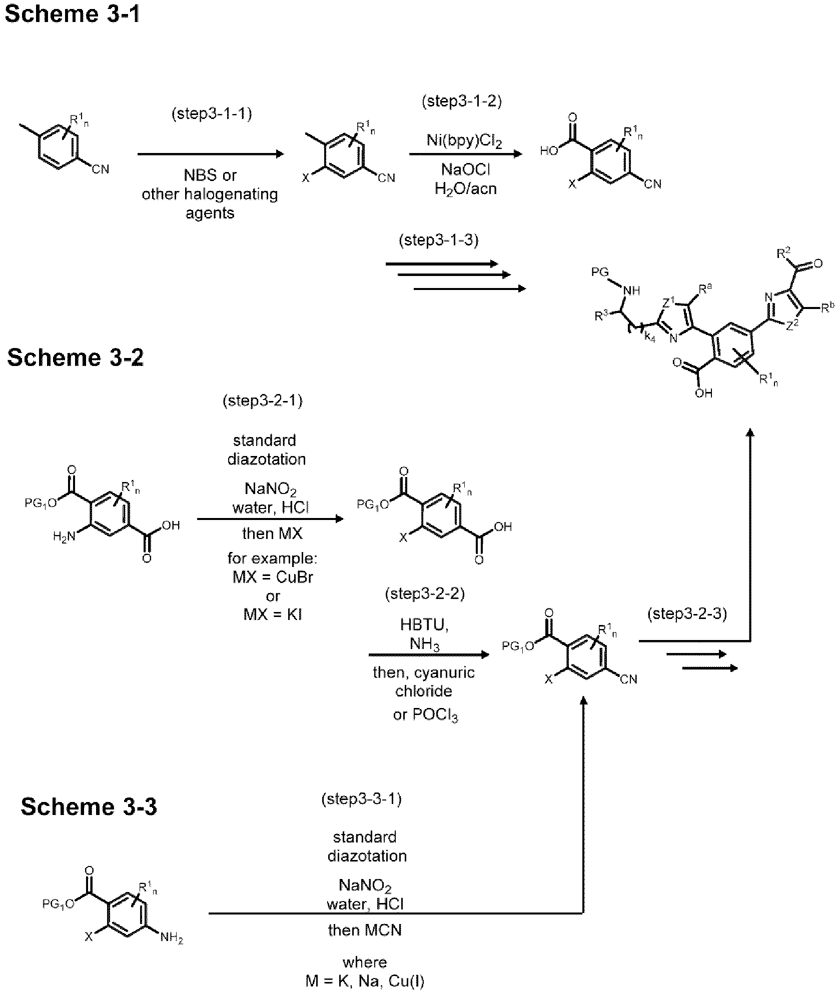

Step 3-1-1 is a step of halogenating the starting material. Reference can be made to, for example, loannis N. Houpis et al., Synlett 2007, 14, 2179-2184. In this step, a halogenating agent such as N-bromosuccinimide may be used.

Step 3-1-2 is a step of oxidizing the methyl group of the product of Step 3-1-1 to convert the methyl group into a carboxy group. This step may be a step of performing oxidation with an oxidizing agent such as NaOCl in the presence of Ni(bpy)Cl₂.

Step 3-1-3 is a step of performing Steps 2-1, and 2-3 to 2-5 in order on the product of Step 3-1-2 to convert the product into the compound represented by Formula (1).

Step 3-2-1 is a step of converting an amino group of the starting material into a halogen group. In this step, a general reaction such as a Zandmeyer reaction may be used.

Step 3-2-2 is a step of converting the free carboxy group of the product of Step 3-1-1 into a cyano group. In this step, a method of performing a reaction with 2-(1H-benzotriazol-1-yl)-1,1,3,3-tetramethyluronium hexafluorophosphate (HBTU), and then performing a reaction with cyanuric acid chloride or phosphoryl chloride may be used.

Step 3-2-3 is a step of sequentially performing Step 2-3 to Step 2-5 to convert the product of Step 3-2-2 into the compound represented by Formula (1).

Step 3-3-1 is a step of converting the amino group of the starting material into a cyano group. In this step, a general reaction such as a Zandmeyer reaction may be used.

### (Scheme 4: Synthesis of Compound of Formula (1))

The compound of Formula (1) can be synthesized as in the following Schemes 4-1 and 4-2 instead of Scheme 1.

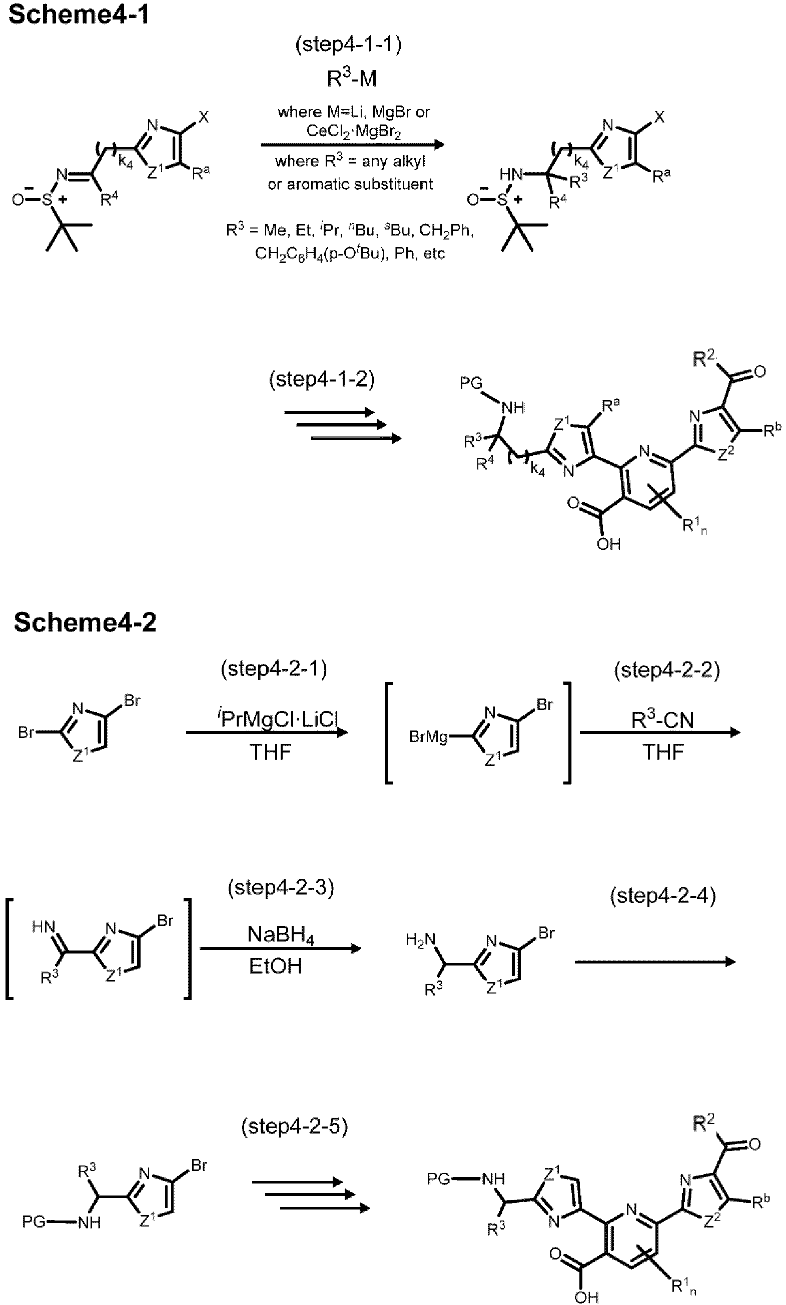

Step 4-1-1 is a step of bonding R³, which is a hydrocarbon group, to carbon of the C=N bond of the starting material. In this step, a known reagent in the related art, which can generate a C-C bond on carbon of a C=N bond, such as an organic lithium reagent, a Grignard reagent, and an organic cerium reagent, may be used. As such a reagent, an organic zinc reagent ((R⁴)₂Zn) and an organic boron reagent (R⁴-B(OH)₂) may also be used. This step may be performed at a reaction temperature from -78°C to room temperature using a solvent such as THF, diethyl ether, and benzene.

In this step, reference can be made to MaryAnn T. Robak et al., Chem. Rev., 2010, 110, 6, 3600-3740.

Step 4-1-2 is each step of Scheme 2. Step 4-1-2 may be each step of Scheme 3.

Step 4-2-1 is a step of preparing a Grignard reagent using the starting material. In this step, a so-called turbo Grignard reagent such as ⁱPrMgCl·LiCl may be used, and a method of reacting a starting material with Mg may be used.

Step 4-2-2 is a step of reacting the product of Step 4-2-1 with R³-CN. Here, R³ is not particularly limited as long as it is a non-acidic group (for example, having a pKa of 30 or more).

In this step, reference can be made to Oscar Delgado et al., Org. Chem., 2006, 71, 12, 4599-4608.

In addition, in Step 4-2-2, an aldimine or a ketimine having the following structure may be used instead of R³-CN. In this case, Step 4-2-3 can be omitted.

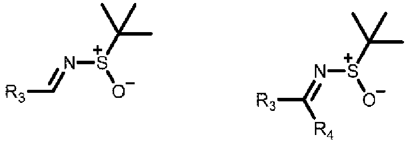

Step 4-2-3 is a step of reducing the C=N bond of the product of Step 4-2-2. In this step, an appropriate reducing agent such as NaBH₄ may be used.

Step 4-2-4 is a step of protecting the amino group of the product of Step 4-2-3. As the protective group, although various known protective groups in the related art can be used, for example, a t-butylcarbonyl group (Boc group) may be used.

Step 4-2-5 is the same as or similar to Steps 1-3 and 2-4. In Step 4-2-5, each step of Scheme 3 may be used.

### (Scheme 5: Synthesis of Compound of Formula (1) (Intermediate 3: Intermediate in Which R³ and R⁵ Form Ring))

In Formula (1), the intermediate 3 in which R³ and R⁵ form a ring can be synthesized as in the following Schemes 5-1 and 5-2.

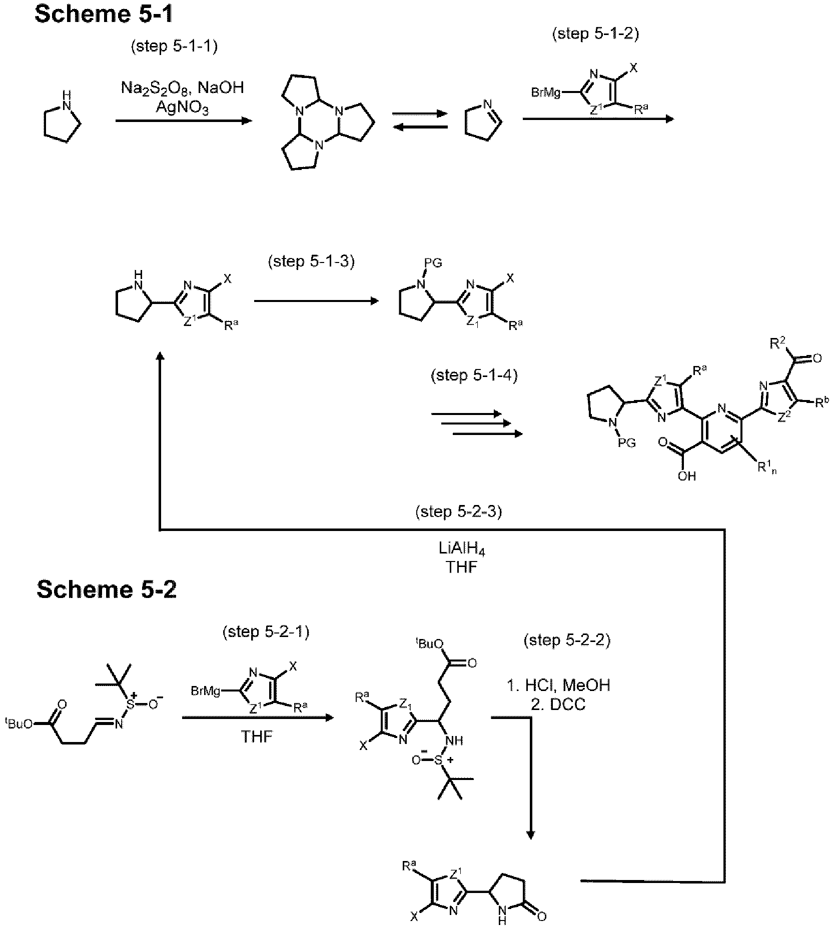

Step 5-1-1 is a step of trimerizing the starting material.

In this step, reference can be made to Chem. Eur. J., 2016, 22, 3009-3018.

Step 5-1-2 is a step of reacting the product of Step 5-1-1 with a Grignard reagent as obtained in Step 4-2-1.

Step 5-1-3 is a step of protecting the amino group of the product of Step 5-1-2. As the protective group, although various known protective groups in the related art can be used, for example, a t-butylcarbonyl group (Boc group) may be used.

Step 5-1-4 is the same as or similar to Steps 1-3 and 2-4. In step 5-1-4, each step of Scheme 3 may be used.

Step 5-2-1 is a step of reacting the starting material with the Grignard reagent as obtained in Step 4-2-1.

Such a starting material can be synthesized by the method described in J. Org. Chem., 2017, 13, 2428-2441.

Step 5-2-2 is a step of removing the sulfinamide moiety of the product of Step 5-2-1 with a strong acid, and deprotecting the protective group of the carboxy group to perform intramolecular cyclization.

Step 5-2-3 is a step of reducing the carbonyl group of the product of Step 5-2-2 under appropriate reducing conditions.

### (Scheme 6: Synthesis of Compound of Formula (I) Having Plurality of Azole Rings)

The compound represented by Formula (I) having a plurality of azole rings can be synthesized as in the following Schemes 6-1, 6-2, and 6-3.

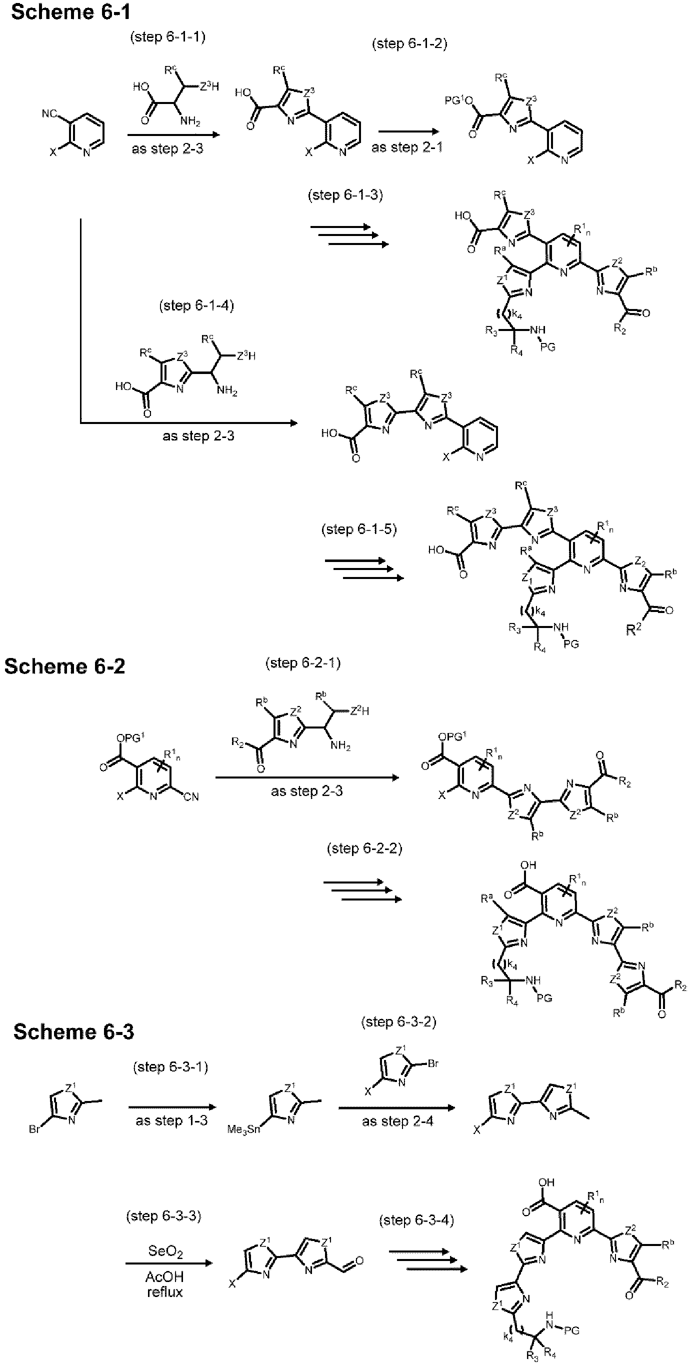

Steps 6-1-1 and 6-1-4 can be carried out in the same manner as in Step 2-3.

Step 6-1-2 can be carried out in the same manner as in Step 2-1.

In Step 6-1-3, the same or similar steps to each step of Scheme 2 may be used.

Step 6-2-1 can be carried out in the same manner as in Step 2-3.

In Step 6-2-2, the same or similar steps as the steps of Scheme 2 may be used.

Step 6-3-1 can be carried out in the same manner as in Step 1-3.

Step 6-3-2 can be carried out in the same manner as in Step 2-4.

Step 6-3-3 is a step of converting a methyl group of the product of Step 6-3-2 into a formyl group. In this step, a method using selenium oxide and acetic acid may be used.

In this step, reference can be made to J. Am. Chem. Soc., 2011, 133, 15, 5900-5904.

In Step 6-3-4, the steps that are the same as or similar to the steps of Scheme 1 and Scheme 2 may be used.

### [Production Method for Library Including Compound of Formula (I)]

The present invention can also provide a production method for a library including 2 or more kinds of compounds represented by Formula (I). That is, in the above-described compound production method of the present embodiment, a library including 2 or more kinds of compounds represented by Formula (I) can be produced by randomizing the compound represented by Formula (1), the amino acid, and the amino acid derivative to be reacted.

For the production of the library, a production method for a peptide library by solid-phase synthesis known in the related art, such as a pin method in which a plurality of pins whose a distal end part is aminated by chemical modification are used as a solid-phase carrier; a split-and-mix method in which a plurality of beads are divided into a plurality of sets, different amino acids or amino acid derivatives are reacted in each set, the plurality of beads are mixed, and the plurality of beads are divided into a plurality of sets again, and different amino acids or amino acid derivatives are reacted in each set again, these steps being repeated; or the like may be used. In the compound production method of the present embodiment, since the aromatic 6-membered ring is introduced into the peptide by using Formula (1), a method applied in a general solid-phase peptide synthesis method can be adopted.

Therefore, according to the compound production method of the present embodiment, it is also possible to produce a one-bead-one-compound (OBOC) library in which each of different compounds is bonded to each solid-phase carrier.

### [Screening Method]

The present invention can also provide a method of screening a compound that is bonded to or interacts with a desired target from the compounds represented by Formula (I).

According to the compound production method of the present embodiment, it is possible to simply and efficiently biosynthesize various types of thiopeptides and pyritides, and artificial derivatives thereof. In this manner, it is possible to easily carry out the production and the functional evaluation of a candidate compound identified using the library construction/search method described in Pamphlet of International Publication No. 2020/067550.

. Further, it is possible to freely synthesize derivatives of these drug candidate compounds.

For example, a compound library constructed by Pamphlet of International Publication No. 2020/067550.
and a desired target are brought into contact with each other to specify a compound candidate having a high possibility of being bonded to or interacting with the target, and a large amount of a specified compound is produced by the compound production method of the present embodiment, whereby the target binding ability, the target inhibition ability, the serum stability, and the like can be measured using the specified compound, and a compound that is bonded to or interacts with the target can be screened with high efficiency.

### [Compound that can act as TNIK Kinase Inhibitor]

The present invention can also provide a compound that can act as a TNIK kinase inhibitor. The TNIK belongs to a Ste20 family of a Ser/Thr protein kinase, and the kinase activity is known to be associated with tumorigenesis formation in Wnt-driven colon cancer and lung squamous cell carcinoma. Since the therapeutic effect of the TNIK inhibitor has been confirmed in several reports, the selective TNIK inhibitor is promising as an anticancer agent candidate.

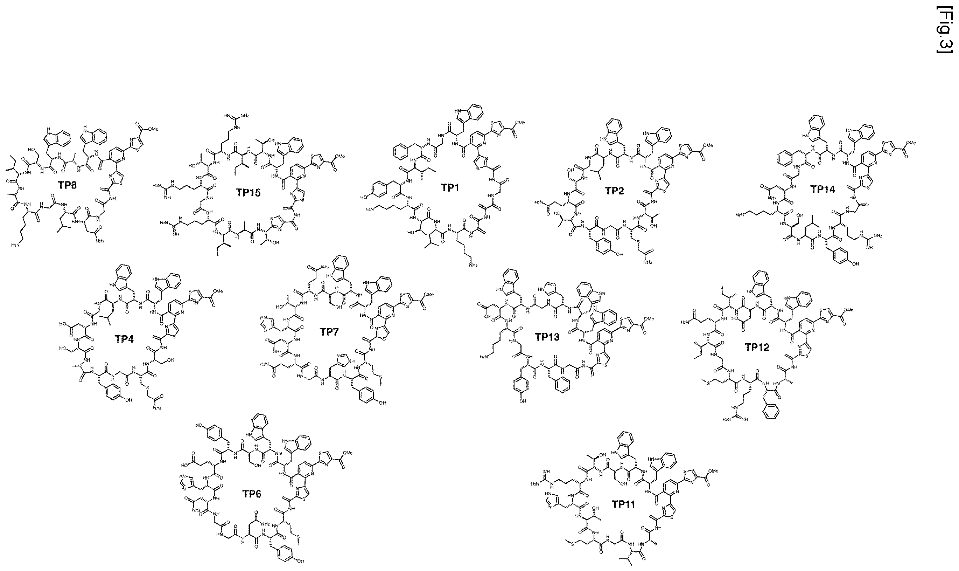

The compound that can act as a TNIK kinase inhibitor in the present embodiment has any of the structures described in Figure 3.

### [Compound that can act as IRAK4 Kinase Inhibitor]

The present invention can also provide a compound that can act as a IRAK4 kinase inhibitor. IRAK4 is an intracellular Ser/Thr protein kinase involved in the downstream inflammatory signal transduction of Toll-like receptors (TLRs) and receptors of an interleukin-1 (IL-1) family. From several studies, it has been shown that IRAK4 kinase-inactive mice are resistant to Alzheimer's disease, arthritis, atherosclerosis, and MOG-induced encephalomyelitis. IRAK4 is recognized as a promising drug target for chronic inflammatory diseases.

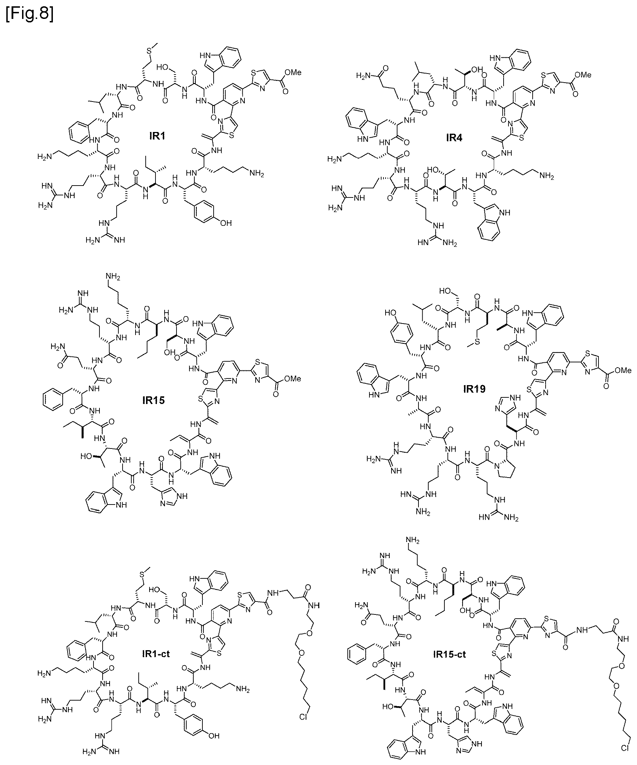

In the present embodiment, the compound that can act as an IRAK4 kinase inhibitor has any of the structures described in Figure 8.

### [Compound that can act as Artificial Ligand for TLR10 Receptor]

The present invention can also provide a compound that can act as an artificial ligand for a TLR10 receptor. TLR10 is a unique member of the TLR family that has an anti-inflammatory function rather than an inflammatory inducing function.

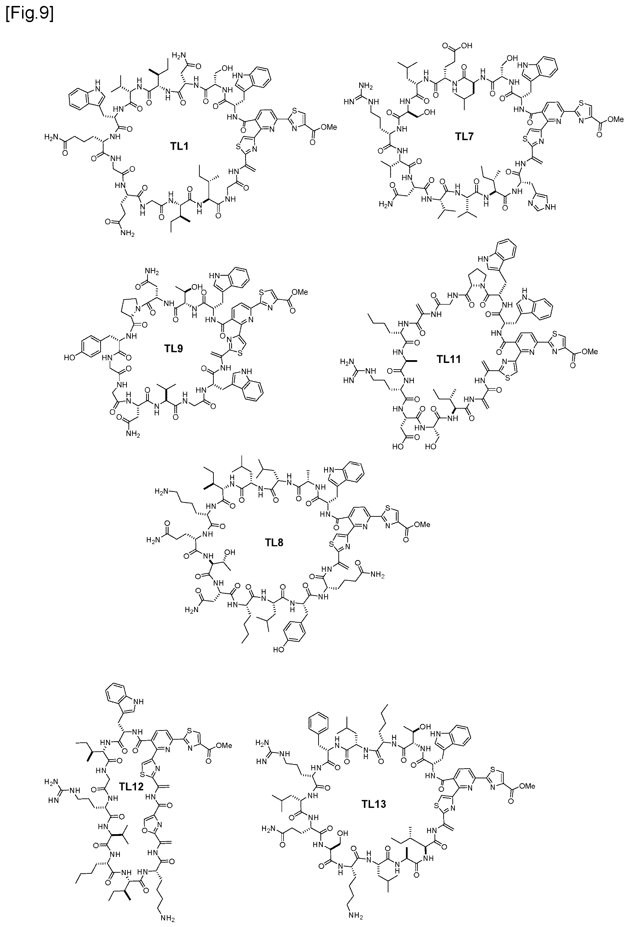

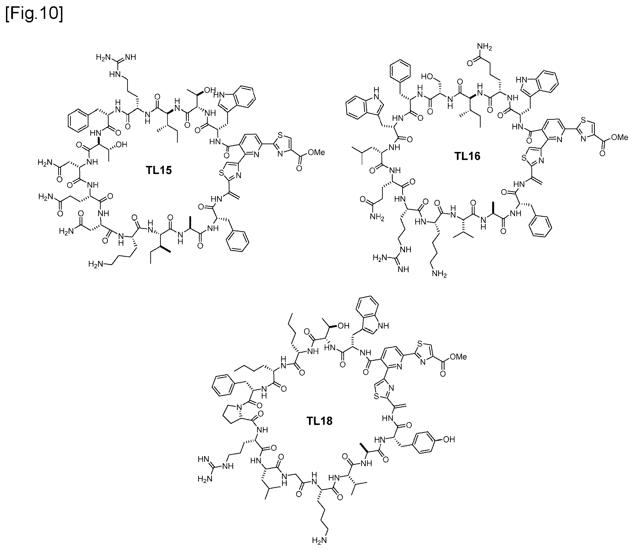

In the present embodiment, the compound that can act as an artificial ligand for a TLR10 receptor has any of the structures described in Figures 9 and 10.

### [Pharmaceutical Composition and the Like]

The present invention also provides a pharmaceutical composition including a compound produced by the compound production method of the present embodiment.

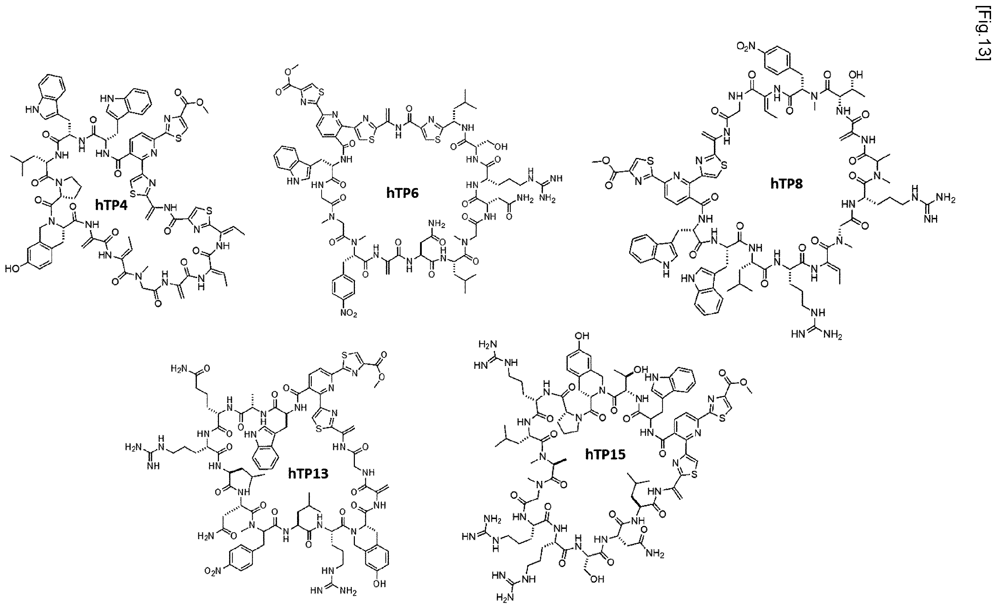

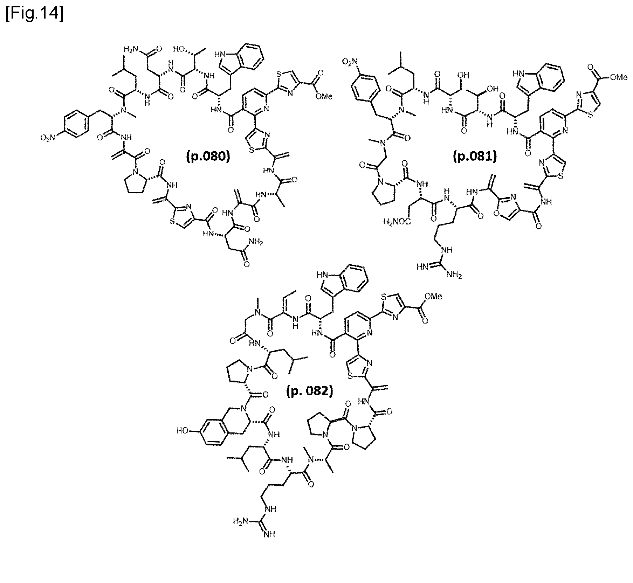

The pharmaceutical composition of the present embodiment includes a compound having any of the structures described in Figures 3, 5, 8 to 10, and 12 to 14.

The pharmaceutical composition of the present embodiment includes, in one embodiment, a compound having any of the structures described in Figures 3 and 12 to 13. In this embodiment, such a pharmaceutical composition may be used for the treatment of diseases involved in the TNIK kinase, including Wnt-driven colon cancer and lung squamous cell carcinoma.

Therefore, the present invention further provides:
a method for treating a disease, including administering an effective amount of a compound having any of the structures described in Figures 3 and 12 to 13 to a patient having such diseases;
a compound having any of the structures described in Figures 3 and 12 to 13 for use in the treatment or prevention of such diseases;
a use of a compound having any of the structures described in Figures 3 and 12 to 13 for the production of a pharmaceutical composition for use in the treatment or prevention of such diseases; and
a prophylactic or therapeutic agent for such diseases, including a compound having any of the structures described in Figures 3 and 12 to 13.

The pharmaceutical composition of the present embodiment includes, in one embodiment, a compound having any of the structures described in Figure 8. In this embodiment, such a pharmaceutical composition may be used for the treatment of diseases in which an IRAK4 kinase is involved, including Alzheimer's disease, arthritis, atherosclerosis, and MOG-induced encephalomyelitis.

Therefore, the present invention further provides:
a method for treating a disease, including administering an effective amount of a compound having any of the structures described in Figure 8 to a patient having such diseases;
a compound having any of the structures described in Figure 8 for use in the treatment or prevention of such diseases;
a use of a compound having any of the structures described in Figure 8 for producing a pharmaceutical composition for use in the treatment or prevention of such diseases; and
a prophylactic or therapeutic agent for such diseases, including a compound having any of the structures described in Figure 8.

The pharmaceutical composition of the present embodiment includes, in one embodiment, a compound having any of the structures described in Figures 9 and 10. In this embodiment, such a pharmaceutical composition may be used for the treatment of a disease in which a TLR10 receptor is involved.

Therefore, the present invention further provides:
a method for treating a disease, including administering an effective amount of a compound having any of the structures described in Figures 9 and 10 to a patient having such diseases;
a compound having any of the structures described in Figures 9 and 10 for use in the treatment or prevention of such diseases;
a use of a compound having any of the structures described in Figures 9 and 10 for the production of a pharmaceutical composition for use in the treatment or prevention of such diseases; and
a prophylactic or therapeutic agent for such diseases, including a compound having any of the structures described in Figures 9 and 10.

The administration form of the pharmaceutical composition of the present embodiment is not particularly limited, and it may be oral administration or parenteral administration. Examples of the parenteral administration include injection administration such as intramuscular injection, intravenous injection, and subcutaneous injection, transdermal administration, transmucosal administration (transnasal, transoral, transocular, transpulmonary, vaginal, or transrectal), and the like.

The active ingredient of the pharmaceutical composition may be used as it is, or the pharmaceutical composition may be formulated by adding a carrier, an excipient, and/or an additive and the like, which are pharmaceutically acceptable. Examples of the formulation form include a liquid agent (for example, an injection agent), a dispersing agent, a suspension agent, a tablet, a pill, a powder agent, a suppository, a powder, a granule agent, a granule, a capsule agent, a syrup, a troche, an inhalant, an ointment, an eye drop, a nasal drop, an ear drop, a patch, and the like.

The formulation can be carried out by a conventional method by using, for example, an excipient, a binder, a disintegrating agent, a lubricant, a solvent, a dissolution assisting agent, a coloring agent, a taste masking and flavoring agent, a stabilizer, an emulsifying agent, an absorption promoting agent, a surfactant, a pH adjusting agent, a preservative, an antioxidant, and the like as appropriate.

Examples of the component to be used for formulation include, but are not limited to, purified water, saline solution, a phosphate buffer solution, dextrose, glycerol, an organic solvent that is pharmaceutically acceptable, such as ethanol, animal and vegetable oils, lactose, mannitol, glucose, sorbitol, crystalline cellulose, hydroxypropyl cellulose, starch, corn starch, anhydrous silicic acid, aluminum magnesium silicate, collagen, polyvinyl alcohol, polyvinyl pyrrolidone, a carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohol, stearyl alcohol, stearic acid, human serum albumin, and the like.

As the absorption promoting agent that ameliorates absorption, the following substances may be used: surfactants such as polyoxyethylene lauryl ethers, sodium lauryl sulfate, and saponin; bile acid salts such as glycocholic acid, deoxycholic acid, and taurocholic acid; chelating agents such as EDTA and salicylic acid; fatty acids such as caproic acid, capric acid, lauric acid, oleic acid, linoleic acid, and mixed micelles; an enamine derivative, an N-acyl collagen peptide, and an N-acyl amino acid, cyclodextrins, chitosans, a nitric oxide donor, and the like.

The pill or the tablet may be coated with a sugar coating, a gastric-soluble substance, or an enteric substance.

The injection agent may include distilled water for injection, physiological saline, propylene glycol, polyethylene glycol, a vegetable oil, alcohols, and the like. Further, a wetting agent, an emulsifying agent, a dispersing agent, a stabilizer, a solvent, a dissolution assisting agent, a preservative, or the like may be added.

The pharmaceutical composition of the present embodiment may be administered in combination with other medicines or therapies useful for the diseases.

In a case where the pharmaceutical composition of the present embodiment is administered to a mammal (for example, a human, a monkey, or the like), particularly to a human, the administration dose thereof differs depending on symptoms, the age, sex, and body weight of the patient, the difference in sensitivity, the method of administration, the interval of administration, the kind of active ingredient, and the kind of formulation, and is not particularly limited. However, for example, 30 µg to 1,000 mg, 100 µg to 500 mg, or 100 µg to 100 mg can be administered once or dividedly several times. In a case of injection administration, 1 µg/kg to 3,000 µg/kg or 3 µg/kg to 1,000 µg/kg may be administered once or dividedly several times depending on the body weight of the patient.

In the above-described aspects described in the present specification, all aspects including the aspects described as the aspects described in the present specification, the preferred aspects in the present specification, and the like can be adopted as aspects in any combination thereof.

### Examples

Hereinafter, the present invention will be described in detail using Examples and Comparative Examples. The present invention is not limited by the following Examples.

### [Example 1: Synthesis of Compound of Formula (1)]

The compound represented by Formula (1) was synthesized by a scheme shown in Figure 1(a). The synthesis of the compound represented by "1" in Figure 1(a) started from the preparation of a 2-bromopyridine fragment 3. As shown in Figure 1(a), the synthesis route started with the esterification of 2-bromonicotinic acid and the subsequent synthesis of 2-cyanopyridine 5. Next, a nitrile-cysteine condensation was performed in an aqueous buffer solution having a pH of 7, and then the obtained thiazoline was dehydrogenated with bromotrichloromethane and diazabicycloundecene to obtain a thiazole-containing fragment 3. This five-stage sequence is operationally simple and could be implemented on a large scale.

In the above synthesis, reference was made to Christy, M. P.; Johnson, T.; McNerlin, C. D.; Woodard, J.; Nelson, A. T.; Lim, B.; Hamilton, T. L.; Freiberg, K. M.; and Siegel, D. Total synthesis of micrococcin P1 through scalable thiazole forming reactions of cysteine derivatives and nitriles. Org. Lett. 2020, 22, 2365-2370.

Next, Selenostannane 4, which is the second Stille fragment, was synthesized. N-sulfinylimine 8 can be easily produced from commercially available 2-formyl-4-bromothiazole and (R)-(+)-tert-butylsulfinamide. The addition of 8 to methylselenomethyl lithium generated by selenium-lithium exchange from bis(methylseleno)methane and n-butyllithium gave a 2:1 mixture of the diastereomer product 9 at a 40% combined yield (Figure 1(b), entry 1). It is known that the alkyl selenoacetal performs selenium-lithium exchange more slowly and efficiently than a generally used Ar-substituted analogue. Therefore, the lithiation time was extended, the amount of the reagent driving the addition reaction was increased, and the reaction yield under the optimum conditions was improved to 67% (Figure 1(b), entries 2 to 4). The addition of a known additive such as HMPA or boron trifluoride to the reaction mixture was counterproductive in further attempts to improve the stereoselectivity of this process (Figure 1(b), entries 5 and 6). Although a C1'-C2' double bond is generated by selenooxidative elimination in a subsequent stage, it is considered that the inconvenience of undefined stereochemistry at the C1' carbon is not large, and thus, a mixture of stereoisomers 9 is selected to be used for downstream synthesis. The preparation of the second Stille fragment 4 was finally completed by Pd-catalyzed stannylation of the compound 9 with hexamethylditin, which proceeded with a good yield.

For the above synthesis, reference was made to Christy, M. P.; Johnson, T.; McNerlin, C. D.; Woodard, J.; Nelson, A. T.; Lim, B.; Hamilton, T. L.; Freiberg, K. M.; Siegel, D. Total synthesis of micrococcin P1 through scalable thiazole forming reactions of cysteine derivatives and nitriles. Org. Lett. 2020, 22, 2365-2370.; Gross, S.; Nguyen, F.; Bierschenk, M.; Sohmen, D.; Menzel, T.; Antes, I.; Wilson, D. N.; Bach, T. Amythiamicin D and related thiopeptides as inhibitors of the bacterial elongation factor EF-Tu: modification of the amino acid at carbon atom C2 of ring C dramatically influences activity. ChemMedChem 2013, 8, 1954-1962.; Ammer, C.; Bach, T. Total syntheses of the thiopeptides amythiamicin C and D. Eur. J. Chem. 2010, 16, 14083-14093.; Delgado, O.; Heckmann, G.; Muller, H. M.; and Bach, T. Synthesis and configurational assignment of the amino alcohol in the eastern fragment of the GE2270 antibiotics by regio-and stereoselective addition of 2-metalated 4-bromothiazoles to α-chiral electrophiles. J. Org. Chem. 2006, 71, 4599-4608.

Next, the efficiency of the Stille coupling between the compound 3 and the compound 4 was evaluated. Under the standard conditions in which copper (I) iodide and cesium fluoride were used as additives, the yield of the tricyclic product 10 was moderate (Figure 1(c), entries 1 to 3), which was considered to be due to the decomposition of the product. Further experiments revealed that the coupling proceeded considerably quickly and the compound 10 could be isolated with a yield of 87% after being reacted at 80°C for 1 hour (Figure 1(c), entry 4). Finally, in order to obtain the compound 1 (the compound represented by Formula (1)) that is appropriately protected, the coupling product was treated with a mixture of trifluoroacetic acid and hydrochloric acid to deprotect the tert-butyl ester, and the sulfinamide auxiliary was removed. Next, the liberated primary amine was reacted with Fmoc-succinimide to obtain the compound 1 for direct use in a solid-phase peptide synthesis method (SPPS). As a whole, the synthesis route for the compound 1 included eleven reactions (longest linear sequence: eight stages), proceeded from 2-bromonicotinic acid with an overall yield of 21%, and could be carried out on a multigram scale.

### [Example 2: Synthesis of Compound of Formula (I)]

Next, a compound represented by Formula (I) was synthesized from the compound represented by Formula (1) synthesized in Example 1. In order to prepare a side chain-protected linear peptide as a C-terminal carboxylate suitable for macrocyclization, Fmoc-SPPS was carried out on a 2-chlorotrityl chloride resin, and a mixture of acetic acid and trifluoroethanol in DCM was used to release the peptide from the solid-phase carrier without deprotecting the side chain functional group (Figure 2(a)). (Chow, H. Y.; Po, K. H. L.; Jin, K.; Qiao, G.; Sun, Z.; Ma, W.; Ye, X.; Zhou, N.; Chen, S.; Li, X. Establishing the Structure-Activity Relationship of Daptomycin. ACS Med. Chem. Lett. 2020, 11, 1442-1449).

The compound 1 could be efficiently coupled using only PyBOP as an activating agent in SPPS at 1.2 equivalents (with respect to the resin loading), whereas linear thiopeptide precursors could be manufactured using a standard protocol of SPPS. In order to optimize the macrocyclization conditions, various macrocyclization bonds were screened along with several generally used reagents and peptide backbones. DPPA and FDPPA gave little desired product (Christy, M. P.; Johnson, T.; McNerlin, C. D.; Woodard, J.; Nelson, A. T.; Lim, B.; Hamilton, T. L.; Freiberg, K. M.; Siegel, D. Total synthesis of micrococcin P1 through scalable thiazole forming reactions of cysteine derivatives and nitriles. Org. Lett. 2020, 22, 2365-2370.; Hughes, R. A.; Thompson, S. P.; Alcaraz, L.; Moody, C. J. Total synthesis of the thiopeptide antibiotic amythiamicin D. J. Am. Chem. Soc. 2005, 127, 15644-15651.; Nicolaou, K. e. C.; Dethe, D. H.; Leung, G. Y.; Zou, B.; and Chen, D. Y. K. Total synthesis of thiopeptide antibiotics GE2270A, GE2270T, and GE2270C1. Chem.: Asian J. 2008, 3, 413-429), whereas PyBOP, PyAOP, and HBTU proved moderately effective, and the combination of HATU, HOAt, Oxymapure, and DIPEA as bases was consistently excellent (Jin, K.; Sam, I. H.; Po, K. H. L.; Lin, D. a.; Ghazvini Zadeh, E. H.; Chen, S.; Yuan, Y.; and Li, X. Total synthesis of teixobactin. Nat. Commun. 2016, 7, 1-6).

For TP15, macrocyclization could be performed between Ile10 and Ala11 under the conditions (Figure 2(b)). In order to convert the obtained TP15-Sec(Me)14 into TP15, oxidative elimination of the Se-alkyl selenocysteine derivative was used. For this purpose, TP15-Sec(Me)14 completely deprotected with 200 mM of t-butyl hydroperoxide in a water/acetonitrile mixture at a pH of 8 was treated to obtain TP15 (Figure 2(b)). Finally, the thiopeptide was uniformly purified by reverse phase HPLC and evaluated by NMR, UPLC, and MS.

A total of 11 types of target thiopeptides were synthesized using the above synthesis protocol (Figure. 2(c)). All of these were obtained in a sufficient amount for further biochemical evaluation. The overall synthetic yield calculated based on the original resin loading varied between 5% and 27%, and in most cases, the macrocyclization, deprotection, and oxidation steps could be carried out without the purification of the peptide intermediates. The four sequences (TP6, TP7, TP11, and TP12) include a Met residue, and the two peptides (TP3 and TP4) have htG amino acids (γ^{S,L} homoGln, that is, a product of Cys alkylation with iodoacetamide). The both include a sulfide moiety, and are thus sensitive to oxidation. In these cases, it was found that it is necessary to control the oxidation conditions in order to alleviate the generation of sulfoxide. By decreasing the concentration of ^{t}BuOOH to 50 mM and monitoring the progress of the reaction, the Sec residue could be selectively and oxidatively removed while minimizing overoxidation. The structures of all 11 types of peptides are shown in Figure 3.

### [Example 3: C-Terminal Modification Reaction of Compound of Formula (I)]

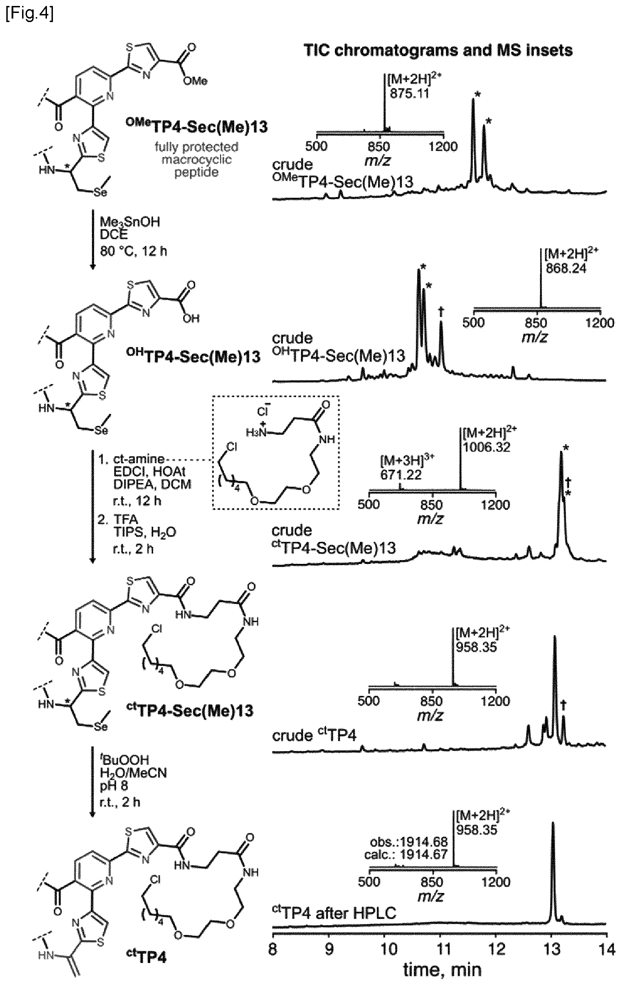

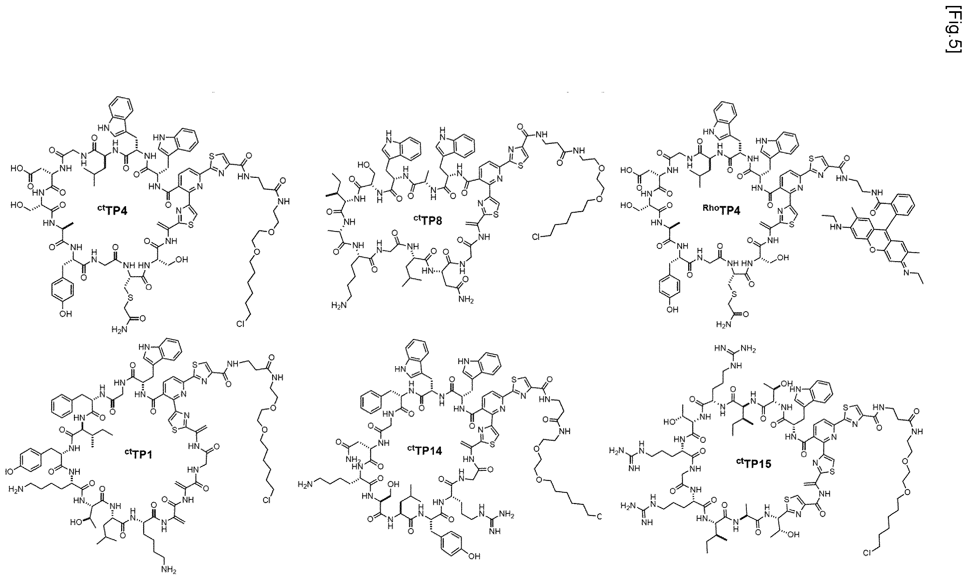

Finally, a functionalization strategy for the synthesized thiopeptide was established (Figure 4). Biophysical and biological assays often require tagged or labeled compounds (fluorophore labelling for confocal microscopy, azide handles for bioconjugation, and the like). Since the amino acid in the macrocyclic compound that is easily modified is not known in advance, the modification of the thiopeptide tail region was carried out. It was found that selective hydrolysis of methyl esters in a tail portion with trimethyltin hydroxide allows for rapid access to various functionalized thiopeptides (Nicolaou, K.; Estrada, A. A.; Zak, M.; Lee, S. H.; Safina, B. S. A mild and selective method for the hydrolysis of esters with trimethyltin hydroxide. Angew. Chem. 2005, 117, 1402-1406). For example, in a case where a fully protected macrocyclic peptide ^{OMe}TP4-Sec(Me)13 was heated for 12 hours in the presence of Me₃SnOH, carboxylate was selectively liberated at the tail part. The only by-product that could be evaluated was the one that had undergone early deselenization of the peptide (Figure 4). The obtained carboxylic acid ^{OH}TP4-Sec(Me)13 can be coupled with various amines such as a chloroalkane tag or a rhodamine 6G-amine, and the rest of the synthesis was carried out according to the established technique. Following these protocols, 6 types of functionalized thiopeptides (^{ct}TP1, ^{ct}TP4, ^{ct}TP8, ^{ct}TP14, ^{ct}TP15; and ^{Rho}TP4) were synthesized for use in a biological assay. These 6 types of peptides are shown in Figure 5.

### [Example 4: Evaluation of Activity of Thiopeptide Derivative Discovered in Screening Targeting TNIK]

Surface plasmon resonance (SPR) was used to evaluate the binding affinity of the thiopeptide synthesized in Example 2 to TNIK. It was found that 9 types of compounds out of the 11 types of compounds shown in Figure 3 show a single-digit or double-digit nanomolar dissociation constant (K_{D}; Figure 6(b)) and are strongly bound to the target protein. The most potent compound TP15 had a K_{D} of 1.2 nM. TP15 was the most potent inhibitor in the ADP-Glo-based kinase inhibition assay and the IC₅₀ value was 0.014 µM (Figure 6(b)). A total of 10 types of thiopeptides inhibited TNIK, and most of the thiopeptides had an IC₅₀ in single-digit µM range, but TP1 (IC₅₀ = 0.13 µM) and TP8 (IC₅₀ = 0.6 µM) were outstanding as a sub-µM inhibitor. According to the results of SPR, the compounds (TP1, TP8, TP14, and TP15; the rest have not been tested) also bonded to TNIK in the presence of the saturated concentration of ATP (1 mM; 15-fold higher than K_{M}), suggesting that the thiopeptide targets an interface other than the ATP binding site. Consistent with the data, the kinetic analysis of the TNIK inhibition by TP15 indicated that this compound acts as a substrate competitive inhibitor of an enzyme with an inhibition constant (Kᵢ) of 3 nM (Figure 6(c)).

Next, a kinase selectivity profiling experiment was carried out using a panel of various human kinases. TP1 and TP15 exhibited good selectivity for a target enzyme. At a concentration of 1 µM, TP15 had a selectivity score [S(0.1)] of 0.03, and exhibited selectivity (Figure 6(d)) (Bosc, N.; Meyer, C.; Bonnet, P. The Use of Novel Selectivity Metrics in Kinase Research. BMC Bioinformatics 2017, 18, 17). TP15 inhibited not only TNIK but also MST1 and LOK (two other Ste20 family kinases). TP1 was more selective [S(0.1) = 0.01]. In brief, these data indicate that the compounds shown in Figure 3 can be potent and selective inhibitors of the target kinases.

Next, the metabolic stability of the five most active thiopeptides (TP1, TP4, TP8, TP14, and TP15) was examined. The compounds were incubated with a human serum at 37°C, and the amount of the residual test substance at various points in time was quantified by LC/MS with respect to an internal standard substance. TP4 and TP8 exhibited good stability, and half-lives (τ_{1/2}) thereof were 88 hours and 14 hours, respectively (Figure 6(b)). Next, it was examined whether or not the half-lives of the compounds were affected by the reactivity of the Dha residue. The Dha residue may be conjugated with various serum thiols. For this purpose, TP1, 14, and 15 were incubated while increasing the concentration of glutathione (GSH), and the reaction results were analyzed by LC/MS. As a result, it was found that Dha11 and Dha12 in TP1 react with GSH, but the 2-(1-aminovinyl)-thiazole moiety present in all peptides (Dha13/14) does not react. TP14 and TP15 were stable (residual amount > 95%) in the presence of 10 mM GSH after 24 hours, and thus, the short half-life of these in the serum was mainly due to proteolysis. In fact, some decomposition products of TP14 and TP15 were observed by LC/MS.

Next, it was confirmed whether or not the obtained compound could also access an intracellular target protein (TNIK is localized in the cytoplasm and the cell nucleus) in a human cell model. First, a chloroalkane penetration assay (CAPA) was performed on 4 types (TP1-ct, TP8-ct, TP14-ct, and TP15-ct) out of the 6 types of chloroalkane-labeled thiopeptide derivatives shown in Figure 5 in order to measure an uptake degree into the cytoplasm (Peraro, L.; Deprey, K. L.; Moser, M. K.; Zou, Z.; Ball, H. L.; Levine, B.; and Kritzer, J. A. Cell Penetration Profiling Using the Chloroalkane Penetration Assay. J. Am. Chem. Soc. 2018, 140, 11360-11369). It was found that 3 types (TP1-ct, TP14-ct, and TP15-ct) out of the 4 types of the tested compounds were taken up into HEK293H cells at a concentration of a single-digit µM. This is comparable to Tat which is a well-known cell-penetrating peptide (Figure 7(a)).

Based on these results, the cell inhibition of TNIK by the most active thiopeptide, TP15, was examined. Evaluation by an immunoblotting method revealed that In a case where HCT116 colon cancer cells were treated with 20 µM of TP15 for 24 hours, the level of TNIK pSer764, a product of TNIK autophosphorylation, was decreased, which was the same as in the case of the small molecule inhibitor NCB0846 (Figure 7(b)) (Shitashige, M.; Satow, R.; Jigami, T.; Aoki, K.; Honda, K.; Shibata, T.; Ono, M.; Hirohashi, S.; Yamada, T. Traf2- and Nck-Interacting Kinase Is Essential for Wnt Signaling and Colorectal Cancer Growth. Cancer Res. 2010, 70, 5024-5033.; Masuda, M.; Uno, Y.; Ohbayashi, N.; Ohata, H.; Mimata, A.; Kukimoto-Niino, M.; Moriyama, H.; Kashimoto, S.; Inoue, T.; Goto, N.; Okamoto, K.; Shirouzu, M.; Sawa, M.; and Yamada, T. TNIK Inhibition Abrogates Colorectal Cancer Stemness. Nat. Commun. 2016, 7, 12586).

At the same time, NCB0846, but not TP15, down-regulated the overall expression of TNIK in both the protein and mRNA levels (measured by RT-qPCR; Figure 7(b)), affecting only TNIK pSer764. Consistent with the TNIK inhibition by NCB0846, it was also found that the RT-qPCR experiment revealed that in a case where HCT116 cells are treated with TP15, the transcription of AXIN2 and MYC mRNA, which are two classical targets of the Wnt signaling pathway, was suppressed in a concentration-dependent manner (Figure 7(c)). The protein levels of c-Myc and Axin2 were also decreased after incubation with 20 µM TP15 for 24 hours (Figure 7(b)). These results suggest that the newly discovered thiopeptide is promising for targeting intracellular proteins.

The efficient cytoplasmic translocation of TP1, TP14, and TP15 at a concentration comparable to that of Tat suggests that the newly discovered lactazole-like thiopeptide can be used to target intracellular proteins. At present, the exact mechanism of the cell invasion of the thiopeptide (both the natural product and the synthesized compound) is unknown. It is considered that the structural rigidity and the hydrophobicity imparted to the structure by the heterocyclic core are presumably involved in this process.

### [Example 5: Evaluation of Activity of Thiopeptide Derivative Discovered in Screening Targeting IRAK4 or TLR10]

The compounds shown in Figures 8 to 10 were further synthesized in the same manner as in Examples 1 and 2. As an example, the synthesis route for TL12 shown in Figure 9 is shown in Figure. 11(a).

Next, the binding affinity of each of the synthesized thiopeptides (14 types excluding IR1-ct and IR15-ct in Figure 8) to the target IRAK4 and TLR10 was examined using surface plasmon resonance (SPR). It was found that 10 types out of the 14 types of synthetic compounds exhibit significant binding to each target, IR1 exhibits the strongest binding affinity (K_{D} = 1.3 nM) to IRAK4, and TL7 exhibits the strongest binding affinity (K_{D} = 306 nM) to TLR10 (Figures 11(b) and 11(c)). Next, using an ADP-Glo-based kinase inhibition assay, the IC₅₀ values of 4 types of thiopeptides for IRAK4 excluding IR1-ct and IR15-ct were evaluated. In the IC₅₀ value for IRAK4, 3 types out of the 4 types inhibited IRAK4 in a single-digit µM region.

In addition, the metabolic stability of all of the 14 types of candidate thiopeptides was examined. The compound was incubated with the human serum at 37°C, and the amount of the residual thiopeptide at various points in time was quantified by LC/MS with respect to the internal standard substance. The half-life of the thiopeptide for TLR10 was generally as long as 38.2 hours, and the thiopeptide was stable. The short half-life of the IRAK4-targeted thiopeptide was mostly due to the proteolysis of a plurality of arginines present in the inserted sequence.

Finally, the cell activity of the thiopeptide against IRAK4, which is an intracellular target protein localized in the cytoplasm and the cell nucleus, was examined. In order to confirm that the synthesized thiopeptide can access the intracellular target, a chloroalkane penetration assay (CAPA) for profiling the degree of cytoplasmic penetration related to concentration in the presence of the serum was carried out using two chloroalkane-labeled thiopeptides (IR1-ct and IR15-ct) illustrated in Figure 8. It was found that IR15 is taken up into HEK293T cells 2-fold more efficiently than Tat (Figure. 11(d)). Further, both the compounds exhibited significant inhibition of an NFkB reporter gene activity in the THP1-XBlue stable cell line (7.8 and 7.0 µM, respectively, Figure 11(b)). These results suggest that the obtained thiopeptides are promising for targeting intracellular proteins, similar to the natural thiopeptides.

### [Example 6: Synthesis of Compound of Formula (I) Including Non-Proteinogenic Amino Acid]

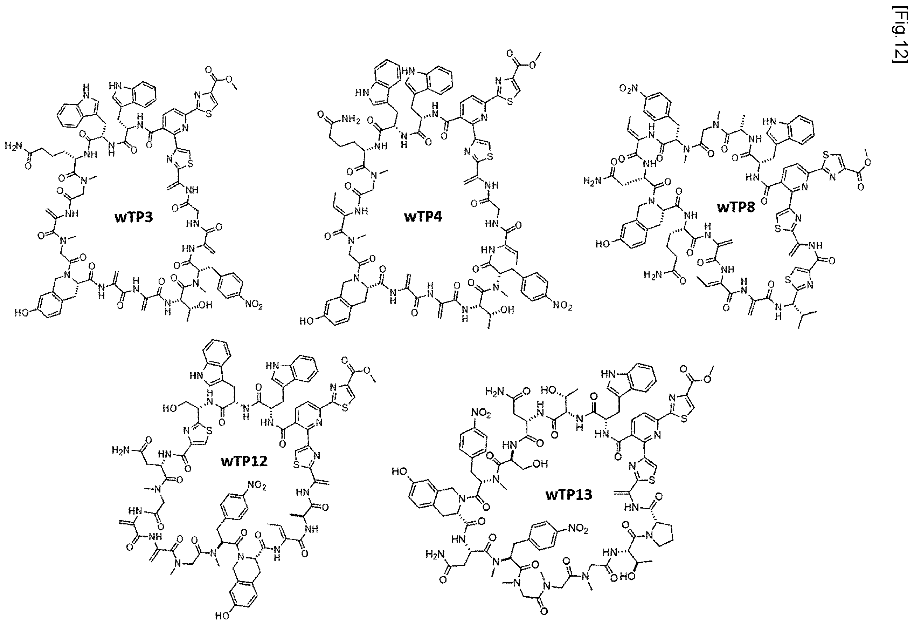

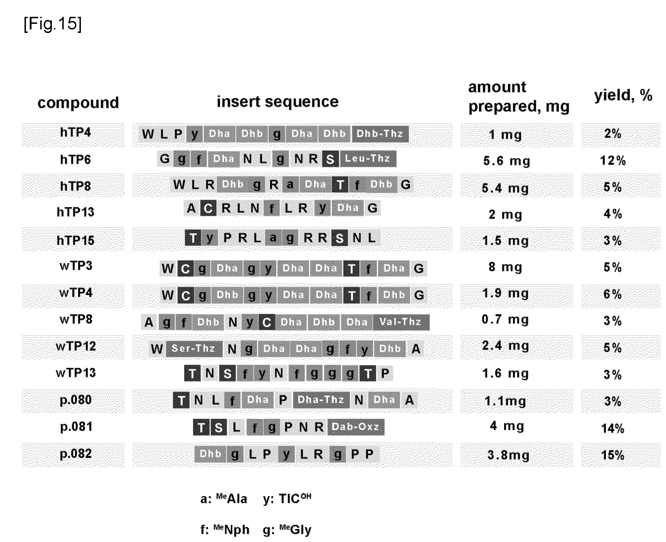

A compound represented by Formula (I) including a non-proteinogenic amino acid was synthesized in accordance with the synthesis method described in Examples 1 and 2. Structural formulae of the synthesized compounds are shown in Figures 12 to 14. The synthesis results of the compounds of Figures 12 to 14 are shown in Figure 15.

All of the compounds described in Figures 12 to 14 were synthesized using the compound represented by Formula (1) synthesized in Example 1 as a starting material. Specifically, in the same manner as in Example 1, first, Fmoc-SPPS was carried out on a 2-chlorotrityl chloride resin, and then a peptide was liberated from a solid-phase carrier without deprotecting the side chain functional group, using a mixture of acetic acid and trifluoroethanol in DCM, to synthesize the following precursors.

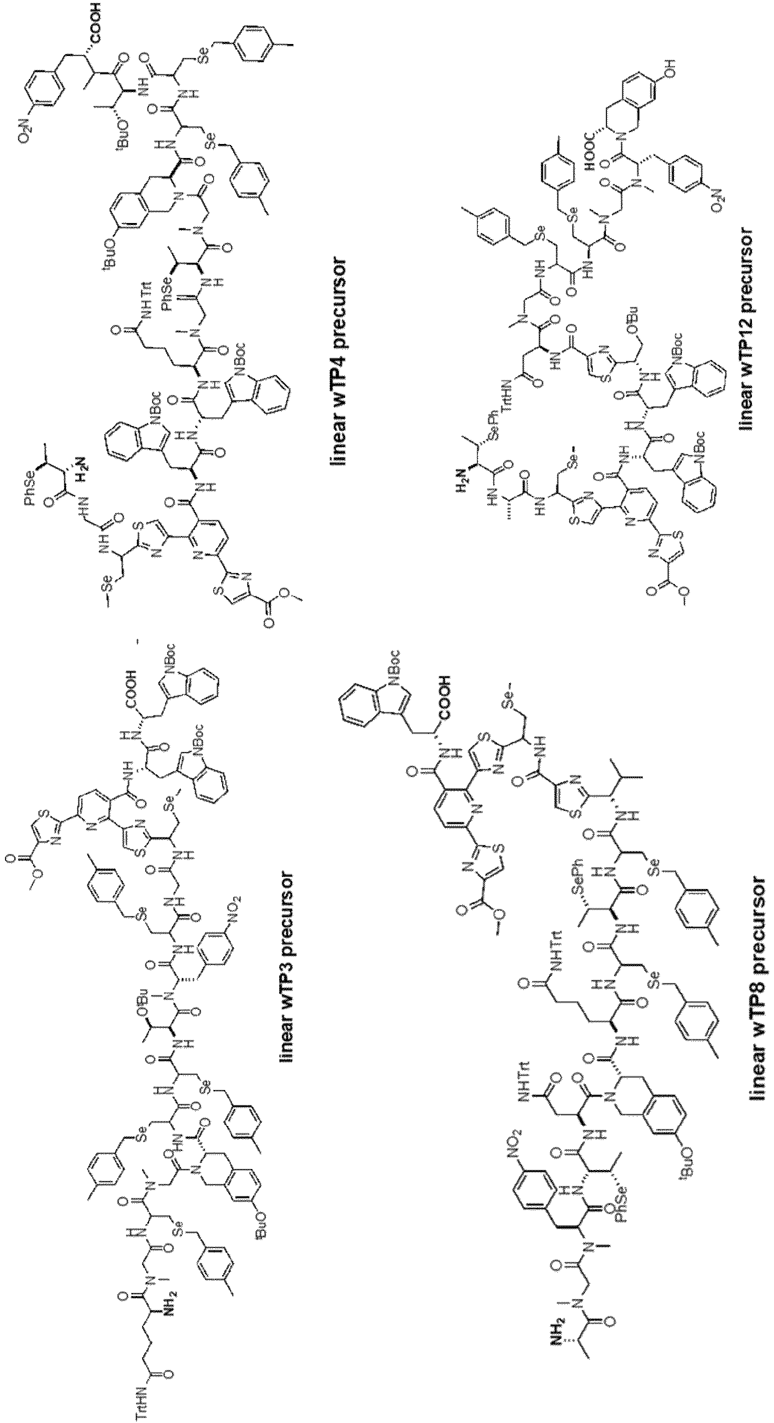

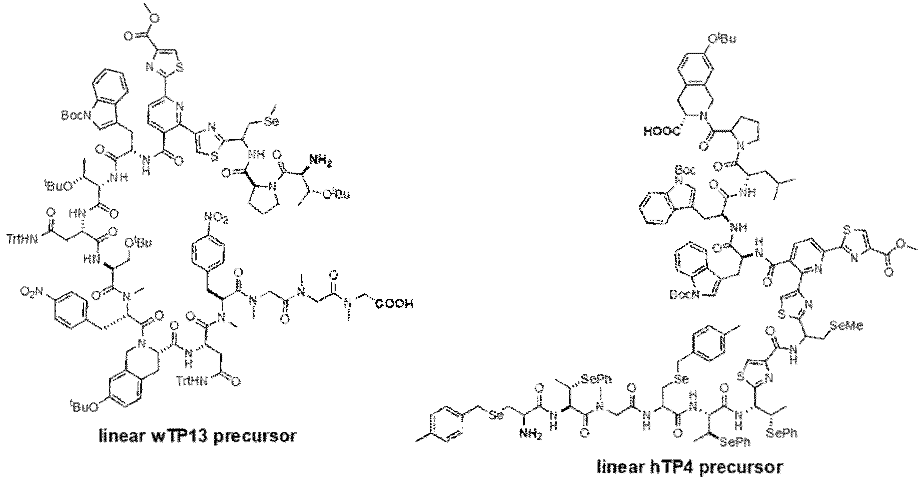

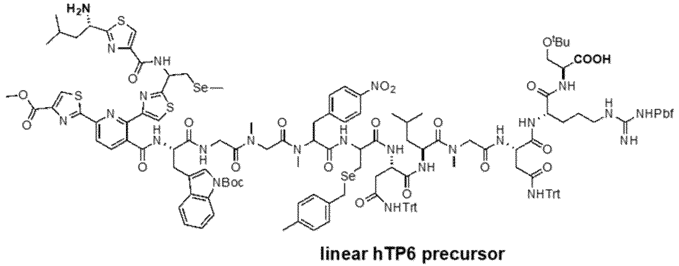

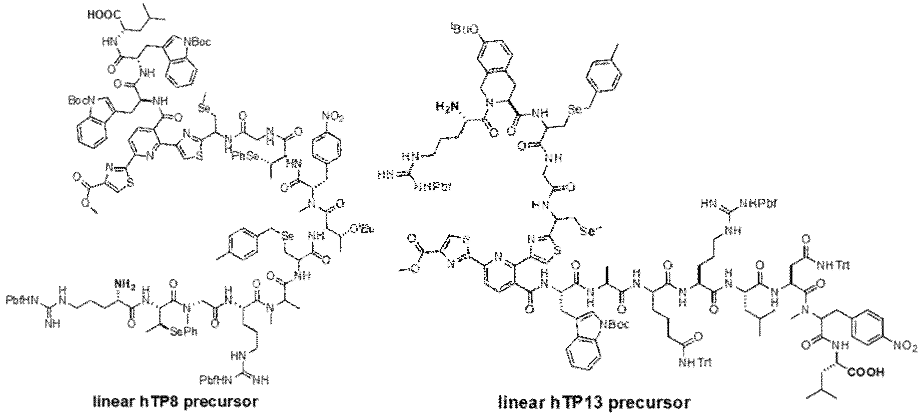

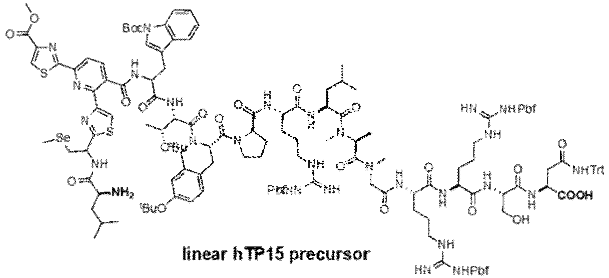

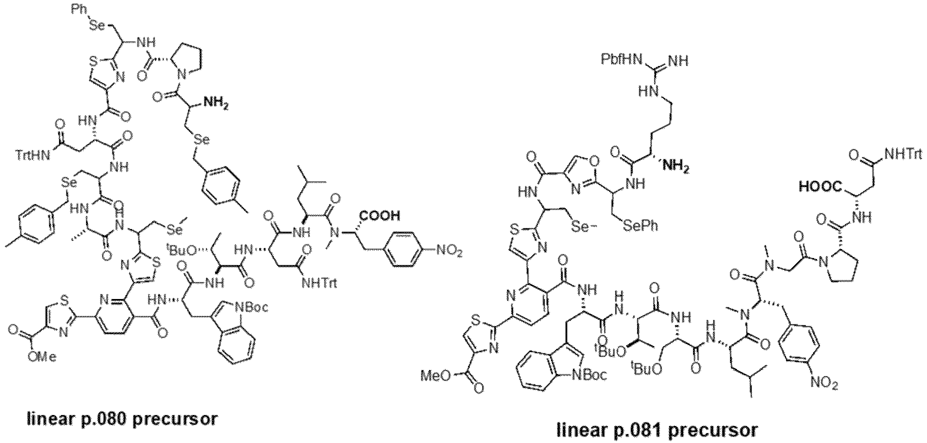

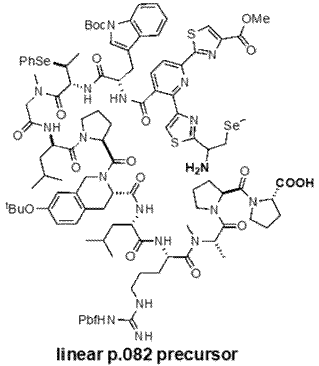

The obtained precursor was macrocyclized under the same conditions as in Example 1. Furthermore, the selenium-containing group was treated with an oxidizing agent in a water/acetonitrile mixture, and thus, converted into a carbon-carbon double bond. For wTP3 and wTP12, NaIO₄ was used as an oxidizing agent, and for the other compounds, hydrogen peroxide was used as the oxidizing agent.

## Claims

1. A production method for a compound represented by Formula (I), 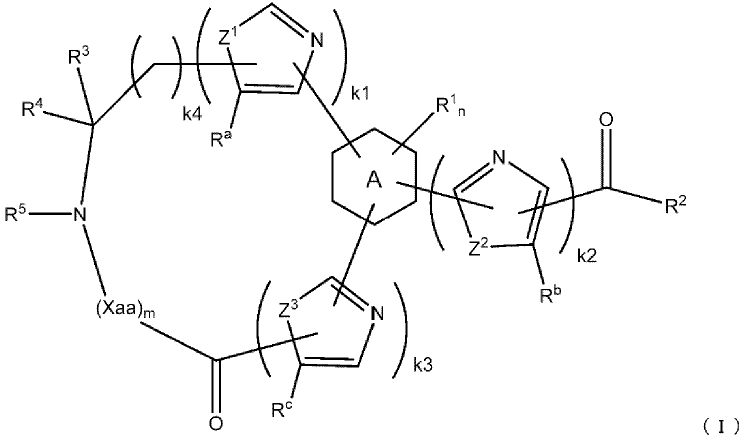 (in Formula (I),
ring A is an aromatic 6-membered ring,
Z¹, Z², and Z³ are each independently an oxygen atom or a sulfur atom,
R^{a}, R^{b}, and R^{c} are each independently a hydrogen atom or a hydrocarbon group,
k1, k2, and k3 are each independently an integer of 0 or more and 2 or less,
k4 is an integer of 0 or more and 2 or less,
n is an integer of 0 or more and 2 or less,
R¹ and R² are each independently a monovalent group,
R³ is a hydrogen atom, a side chain of an amino acid, or a side chain of an amino acid derivative, forms a ring together with R⁴ or R⁵, or forms a double bond together with R⁴,
R⁴ is a hydrogen atom or a hydrocarbon group, or forms a ring or a double bond together with R³,
R⁵ is a hydrogen atom or a hydrocarbon group, or forms a ring together with R³,
(Xaa)ₘ is a peptide including m pieces of amino acids and/or amino acid derivatives, and
m is an integer of 2 or more),
the method comprising:
reacting an amino acid or amino acid derivative, or a peptide, which is bonded to a solid-phase carrier and has a free amino group, or a solid-phase carrier having a free amino group with a compound represented by the following formula: 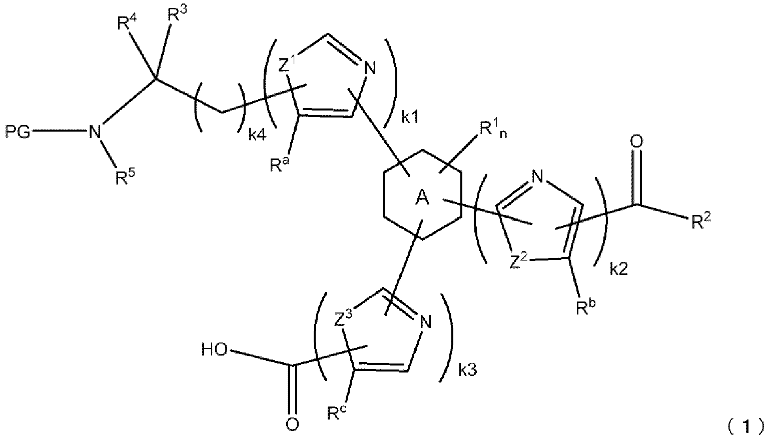 (in Formula (1),
ring A, Z¹, Z², Z³, R^{a}, R^{b}, R^{c}, k1, k2, k3, k4, n, R¹, R², R³, R⁴, and R⁵ are each defined in the same manner as in Formula (I), and
PG is a protective group)
to obtain a compound represented by the following formula: 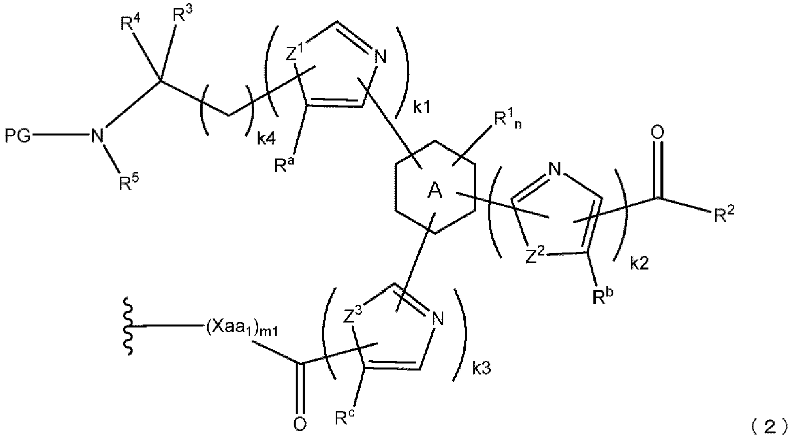 (in Formula (2),
ring A, Z¹, Z², Z³, R^{a}, R^{b}, R^{c}, k1, k2, k3, k4, n, R¹, R², R³, R⁴, and R⁵ are each defined in the same manner as in Formula (1),
(Xaa₁)ₘ₁ is a peptide residue including m1 pieces of amino acids and/or amino acid derivatives bonded to the solid-phase carrier in a case where m1 is 2 or more, an amino acid residue or an amino acid derivative residue in a case where m1 is 1, or a single bond in a case where m1 is 0,
m1 is an integer of 0 or more, and
a wavy line represents that (Xaa₁)ₘ₁ in a case where m1 is 1 or more or a carbonyl in a case where m1 is 0 is bonded to the solid-phase carrier),
wherein a compound obtained by deprotecting PG of the compound represented by Formula (2) is optionally subjected to one or more amino acid condensation reactions and then cleaved from the solid-phase carrier to perform a cyclization reaction.

2. The method according to claim 1,
wherein the compound represented by Formula (1) is represented by Formula (1-A), 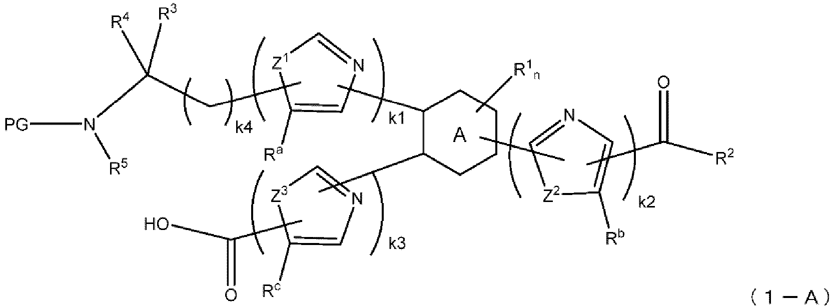 (in Formula (1-A),
ring A, Z¹, Z², Z³, R^{a}, R^{b}, R^{c}, k1, k2, k3, k4, n, R¹, R², R³, R⁴, R⁵, and PG are each defined in the same manner as in Formula (1)).

3. The method according to claim 1,
wherein the compound represented by Formula (1) is represented by Formula (1-B), 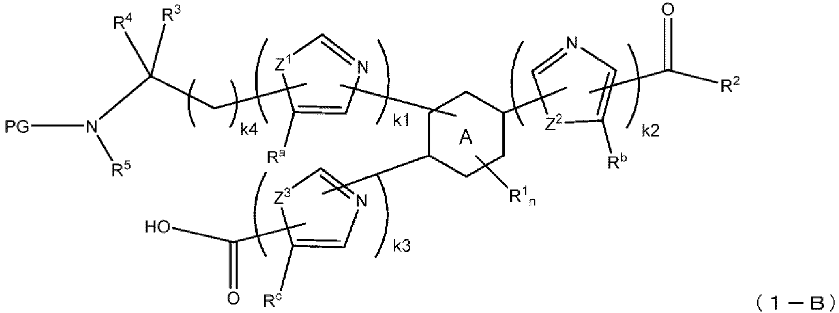 (in Formula (1-B),
ring A, Z¹, Z², Z³, R^{a}, R^{b}, R^{c}, k1, k2, k3, k4, n, R¹, R², R³, R⁴, R⁵, and PG are each defined in the same manner as in Formula (1)).

4. The method according to claim 1,
wherein the compound represented by Formula (1) is represented by Formula (1-C), 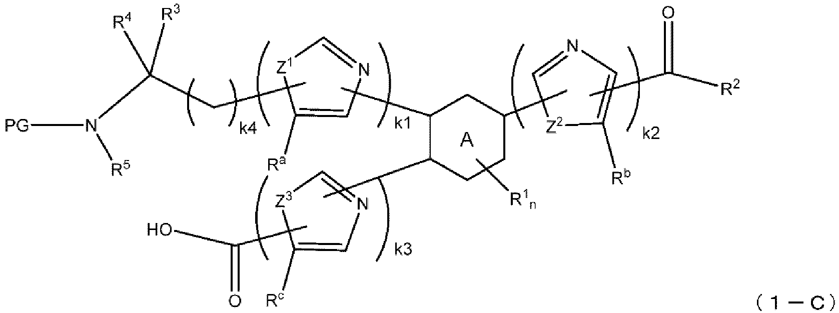 (in Formula (1-C),
ring A, Z¹, Z², Z³, R^{a}, R^{b}, R^{c}, k1, k2, k3, k4, n, R¹, R², R³, R⁴, R⁵, and PG are each defined in the same manner as in Formula (1))

5. The method according to any one of claims 1 to 4,
wherein a side chain functional group of an amino acid and/or amino acid derivative constituting (Xaa)ₘ of the compound obtained by the cyclization reaction is protected by a protective group, and
the method further includes deprotecting the protective group of the side chain functional group.

6. The method according to any one of claims 1 to 4,
wherein the compound obtained by the cyclization reaction has a monovalent group represented by the following formula:
-CR⁷₂-Se-R⁸ (3)
(in Formula (3), Se represents selenium, R⁷'s each independently represent a hydrogen atom or a hydrocarbon group, and R⁸ represents a hydrocarbon group which optionally has a substituent), and
the method further includes oxidizing the group represented by Formula (3) after the cyclization reaction to form a double bond.

7. The method according to any one of claims 1 to 4, the method further comprising:
eliminating R² after the cyclization reaction, and reacting the carboxy group generated by the elimination with one or more of compounds.
